# EUROPEAN PATENT SPECIFICATION

(11) **EP 3 658 901 B1**
(45) Date of publication and mention of the grant of the patent: **03.01.2024**
(21) Application number: 18838851.6
(22) Date of filing: 27.07.2018
(51) Int. Cl.: G01N 21/75, G01N 33/48, G01N 1/30

(54) **A METHOD FOR FIXATION AND RETENTION OF EXTRACELLULAR VESICLES, FIXED EXTRACELLULAR VESICLES AND A METHOD OF DIAGNOSING A DISEASE.**
EIN VERFAHREN ZUR FIXIERUNG UND AUFBEWAHRUNG VON EXTRAZELLULÄREN VESIKELN, FIXIERTE EXTRAZELLULÄRE VESIKEL UND EIN VERFAHREN ZUR DIAGNOSE EINER KRANKHEIT.
UNE MÉTHODE DE FIXATION ET DE RÉTENTION DES VÉSICULES EXTRACELLULAIRES, DES VÉSICULES EXTRACELLULAIRES FIXÉES ET UNE MÉTHODE DE DIAGNOSTIC D'UNE MALADIE.

(30) Priority: 27.07.2017 US 201762537541 P; 05.03.2018 US 201862638554 P
(43) Date of publication of application: 03.06.2020
(73) Proprietor: Cornell University, Ithaca, New York 14850 (US)
(72) Inventor: PENA, John, T. G., Brooklyn NY 11216 (US)
(74) Representative: J A Kemp LLP
(86) International application number: PCT/US2018/044102
(87) International publication number: WO 2019/023584

(56) References cited:
- WO-A1-2013/106852
- WO-A2-2013/016712
- US-A1- 2007 009 497
- US-A1- 2008 070 324
- US-A1- 2010 233 706
- US-A1- 2013 302 856
- US-A1- 2016 245 809
- US-A1- 2016 245 809
- VAN PELT-VERKUIL et al.: "The Use of a Carbodiimide-Containing Fixative for the Immunohistochemical Demonstration of Coagulation Factor VIII in Rat Vascular Tissue", Histochemistry, vol. 71, no. 2, June 1981 (1981-06), pages 187-194, XP055571071,
- RAGUSA et al.: "miRNA profiling in vitreous humor, vitreal exosomes and serum from uveal melanoma patients: Pathological and diagnostic implications", Cancer Biology and Therapy, vol. 16, no. 9, 2 September 2015 (2015-09-02), pages 1387-1396, XP055571077,
- BELLINGHAM et al.: "Exosomes: vehicles for the transfer of toxic proteins associated with neurodegenerative diseases?", Frontiers in Physiology, vol. 2, no. 124, 2012, pages 1-12, XP055571081,

## Description

### FIELD OF THE INVENTION

The present invention relates to a method of fixation of extracellular vesicles using a non-reversible cross-linking agent and, optionally, an aldehyde-containing fixative. This method can be utilized for the imaging of extracellular vesicles as well as for diagnosis or monitoring of disease.

### BACKGROUND OF THE INVENTION

Cancer is a major cause of death (Torre et al., "A. Global Cancer Incidence and Mortality Rates and Trends--An Update," Cancer Epidemiol Biomarkers Prev. 25: 16-27 (2016)) and early diagnosis of cancer and its proper characterization is essential for reducing mortality (McPhail et al., Stage at Diagnosis and Early Mortality from Cancer in England," Br J Cancer S108-115 (2015)). The most common new cases of cancers in men are prostate (161,000 persons), lung (116,900 persons), and colorectal (71,000 persons) cancers. *Id.* For women, the most common new cases of cancers are breast (252,000 persons), lung (105,510 persons), and colorectal (64,000 persons). In 2017, estimated new cases of eye tumors are 3,130 and estimated deaths are 330. *Id.* While intraocular tumors are substantially less prevalent than other cancers, studying ocular tissues and fluids is an opportunity to use the eye as a model system for developing techniques for imaging the structural mediators of metastasis. Once the techniques are established, it is possible to apply the methods to the more prevalent cancers.

Early diagnosis is essential for reducing mortality, and the holy grail of cancer diagnostics is a non-invasive screening test. For most tumors, a tissue biopsy is challenging due to invasive surgical procedures that expose the patient to pain, increased cost, and risk of complication. Moreover, tissue-based tumor profiles are prone to sampling bias, provide only a snapshot of tumor heterogeneity, and cannot be obtained repeatedly. A solution to this technical limitation is to monitor cancer by monitoring predictive biomarkers in the blood or other biological fluids and carrying out liquid biopsies. The development of non-invasive methods to detect and monitor tumors continues to be a major challenge in cancer research and oncology. The goal of this project is to develop a platform technology to non-invasively detect biomarkers secreted from the cancers using a liquid biopsy (blood, or other biological fluids) for early detection, monitoring, or prognostication of a variety of cancers.

A potential source of biomarkers for cancers are extracellular vesicles (EVs), which are natural transport nano-vesicles implicated in inter-cellular communication via transfer of biomolecules such as proteins, lipids, and nucleic acids from one cell to another (Gyorgy et al., "Membrane Vesicles, Current State-of-the-Art: Emerging Role of Extracellular Vesicles," Cell Mol. Life Sci. 68:2667-2688 (2011); Trams et al., "Exfoliation of Membrane Ecto-Enzymes in the Form of Micro-Vesicles," Biochim. Biophys. Acta. 645:63-70 (1981); Dvorak et al., "Tumor Shedding and Coagulation," Science 212:923-924, (1981)). Many cell types secrete exomeres (~35 nm) (Zhang et al., "Identification of Distinct Nanoparticles and Subsets of Extracellular Vesicles by Asymmetric Flow Field-Flow Fractionation," Nat. Cell Biol. 20:332-343 (2018)), exosomes (40-100 nm), larger micro-vesicles (100-10,000 nm), or apoptotic bodies (1-5 µm) (Hristov et al., "Apoptotic Bodies From Endothelial Cells Enhance the Number and Initiate the Differentiation of Human Endothelial Progenitor Cells In Vitro," Blood 104:2761-2766 (2004)) into fluids like blood, cerebrospinal fluid, and urine (Raposo et al., "Extracellular Vesicles: Exosomes, Microvesicles, and Friends," J. Cell Biol. 200:373-383 (2013); Zha et al., "Extracellular vesicles: An Overview of Biogenesis, Function, and Role in Breast Cancer," Tumour Biol 39:1010428317691182 (2017)).

EVs are being implicated in the pathophysiology of several cancers (Gatti et al., "Microvesicles Derived From Human Adult Mesenchymal Stem Cells Protect Against Ischaemia-Reperfusion-Induced Acute and Chronic Kidney Injury," Nephrol Dial Transplant 26:1474-1483 (2011); Zomer et al., "In Vivo Imaging Reveals Extracellular Vesicle-Mediated Phenocopying of Metastatic Behavior," Cell 161:1046-1057 (2015)) including, breast (Luga et al., "Exosomes Mediate Stromal Mobilization of Autocrine Wnt-PCP Signaling in Breast Cancer Cell Migration," Cell 151:1542-1556 (2012); Cho et al., "Exosomes From Breast Cancer Cells Can Convert Adipose Tissue-Derived Mesenchymal Stem Cells Into Myofibroblast-Like Cells," Int J Oncol 40:130-138 (2012); Lee et al., "Exosomes Derived From Mesenchymal Stem Cells Suppress Angiogenesis by Down-Regulating VEGF Expression in Breast Cancer Cells," PLoS One 8:e84256 (2013)), prostate (Nilsson et al., "Prostate Cancer-Derived Urine Exosomes: A Novel Approach to Biomarkers for Prostate Cancer," Br J Cancer 100:1603-1607 (2009)), lung (Wysoczynski et al., "Lung Cancer Secreted Microvesicles: Underappreciated Modulators of Microenvironment in Expanding Tumors," Int J Cancer 125:1595-1603 (2009); Janowska-Wieczorek et al, "Microvesicles Derived From Activated Platelets Induce Metastasis and Angiogenesis in Lung Cancer," Int J Cancer 113:752-760 (2005)), and colorectal (Ji et al., "Proteome Profiling of Exosomes Derived From Human Primary and Metastatic Colorectal Cancer Cells Reveal Differential Expression of Key Metastatic Factors and Signal Transduction Components," Proteomics 13:1672-1686 (2013); Silva et al., "Expression and Cellular Localization of MicroRNA-29b and RAX, an Activator of the RNA-Dependent Protein Kinase (PKR), in the Retina of Streptozotocin-Induced Diabetic Rats,"Mol Vis 17:2228-2240 (2011)), as well as neurodegenerative disorders (Bellingham et al., "Exosomes: Vehicles for the Transfer of Toxic Proteins Associated With Neurodegenerative Diseases?" Front Physiol. 3:124 (2012)).

EVs facilitate the spread of cancer cells, and are involved in the different steps of the metastatic process including; 1) facilitating the movement of cells, 2) promoting the tumor micro-environment, and 3) establishing the pre-metastatic alcove at distant tissues (Tkach etal. "Communication by Extracellular Vesicles: Where We Are and Where We Need to Go," Cell 164:1226-1232 (2016)). Furthermore, EVs are being studied as biomarkers in precancerous (Luga et al., "Exosomes Mediate Stromal Mobilization of Autocrine Wnt-PCP Signaling in Breast Cancer Cell Migration," Cell 151:1542-1556 (2012)) and cancer tissues (Nilsson et al., "Prostate Cancer-Derived Urine Exosomes: A Novel Approach to Biomarkers for Prostate Cancer," Br J Cancer 100:1603-1607 (2009); Rabinowits et al., "Exosomal MicroRNA:A Diagnostic Marker for Lung Cancer," Clin Lung Cancer 10:42-46 (2009)).

In addition to establishing the need for improved EV imaging in fluids, there is a need to image EVs in tissues *in situ.* Recent advances in EV imaging in tissues of living animals include using fusion proteins (Lai et al., "Visualization and Tracking of Tumour Extracellular Vesicle Delivery and RNA Translation Using Multiplexed Reporters," Nat. Commun. 6:7029 (2015)), CRE recombinase with reporter proteins (Ridder et al., "Extracellular Vesicle-Mediated Transfer of Functional RNA in the Tumor Microenvironment," Oncoimmunology 4:e1008371 (2015)), or multiphoton microscopy (Zomer et al., "In Vivo Imaging Reveals Extracellular Vesicle-Mediated Phenocopying of Metastatic Behavior," Cell 161: 1046-1057 (2015)). Yet, a maj or technical challenge in understanding EV biology is the inability to image EVs in tissues and biological fluids *in situ* (Tkach etal. "Communication by Extracellular Vesicles: Where We Are and Where We Need to Go," Cell 164:1226-1232 (2016)). Identifying and solving for technical pitfalls that hinder EV imaging may help elucidate the structure and function of EVs in normal and diseased states.

The most common method to study EV ultrastructure in fluids is transmission electron microscopy (TEM) combined with negative staining. However, it has been found that this technique leads to inconsistent, and often negative results. Moreover, when examining known quantities of EVs applied to a solution, a substantial discrepancy between the amount of EVs applied and the few EVs that were ultimately imaged was observed. For example, over a million EVs were added to the surface of an electron microscopy grid for glutaraldehyde fixation, negative staining, and TEM imaging. However, at the final step, a sparse number of EVs (0 to 50), if any at all were observed. Surprisingly, the results were inconsistent between EV batches; in some cases, technical replicates would vary. Therefore, a methodological gap exists and hinders efficient, consistent and representative EV imaging in solutions. To realize the full potential of imaging EVs in biological fluids, or liquid biopsy, as a medical diagnostic, the existing TEM method requires further development. Therefore, the inventors evaluated each step of the EV TEM imaging protocol and attempted to identify the points at which EVs may be lost.

The present invention is directed to overcoming these and other deficiencies in the art.

### SUMMARY OF THE INVENTION

The present invention relates to a method of fixing extracellular vesicles. The fixation method entails providing a sample containing extracellular vesicles, and contacting the sample with a non-reversible cross-linking agent under conditions effective to fix the extracellular vesicles.

The method of the present invention can optionally further include contacting the sample with an aldehyde-containing fixative before, after, or at the same time as the contacting of the sample with a non-reversible cross-linking agent to fix the extracellular vesicles.

In one embodiment of the present invention the sample being treated with the present invention for the fixation of extracellular vesicle, is a biological fluid or tissue.

In another aspect of the invention the non-reversible cross-linking agent crosslinking agent used to fix the extracellular vesicles is 1-ethyl-3-(3-dimethylaminopropyl)-carbodiimide.

In another aspect the present invention relates to fixed extracellular vesicles obtained by the method of the invention.

Extracellular vesicles (EVs) are secretory nano-sized particles with many physiological functions and a broad range of pathological associations. EVs are made by cells and often secreted into biological fluids and influence the gene expression of distant cell targets. A major technical limitation to understanding the role of EVs in normal and diseased fluid specimens has been the difficulty in reproducibly visualizing EV ultrastructure in tissues and fluids. Here, it is demonstrated that conventional TEM protocols results in inefficient binding of EVs to the electron microscopy grid surface. Moreover, EVs are lost post glutaraldehyde fixation and with wash steps. To more efficiently attach EVs on the surface of the grid, the EVs can be crosslinked using a non-reversible cross-linking agent, 1-ethyl-3-(3-dimethylaminopropyl) carbodiimide (EDC), which retains EVs and enables robust TEM imaging. Moreover, it is demonstrated that this method can be used to image EVs in variety of biological fluids, including blood (plasma), cerebrospinal fluid, nipple aspirate fluid, aqueous humor, and vitreous humor. Finally, it is shown that this method allows for the observation of morphological differences in EVs isolated from healthy controls (blood plasma) and various cancer samples.

Another technical limitation to understanding the role of EVs in normal and diseased specimens has been the inability to visualize the spatial localization of EVs in tissue microenvironments. Here, bovine and human ocular tissue, the vitreous humor, is used as a model system to study EV imaging. Mammalian tissues crosslinked with conventional formaldehyde solutions result in significant EV loss, with subsequent reduced or negative EV signals; however, EV escape can be prevented by additional fixation with 1-ethyl-3-(3-dimethylaminopropyl) carbodiimide (EDC) that permanently holds these nano-sized particles and allows for visualization of EVs in normal and cancer tissues *in situ.*

While methods to image EVs in fluids are inefficient, a similar technical gap in imaging EVs in healthy and disease tissues was found. To study the spatial localization of EVs in tissues, the vitreous body of the eye was used as a model system. The vitreous, located between the lens and the retina, is an optically clear, paucicellular tissue and little-known biological function (Le Goff et al., "Adult Vitreous Structure and Postnatal Changes," Eye (Lond) 22:1214-1222 (2008)). Vitreous EV-associated microRNAs have been described (Ragusa et al. "miRNA Profiling in Vitreous Humor, Vitreal Exosomes and Serum from Uveal Melanoma Patients: Pathological and Diagnostic Implications," Cancer Biol. Ther. 16:1387-1396 (2015)); however, normal vitreous EVs have not yet been imaged nor characterized. It was hypothesized that normal vitreous possesses EVs, yet the repeated attempts to visualize the nanoparticles using multiphoton, confocal or wide-field microscopy failed. Therefore, efforts were focused on optimizing tissue fixation. Conventional fixation methods use 10% formalin to create protein-protein crosslinks. Tissue processing steps generally occur at or above room temperature; however, elevated temperatures are known to revert formalin protein-protein and RNA-protein crosslinks (Shi et al., "Antigen Retrieval In Formalin-Fixed, Paraffin-Embedded Tissues: An Enhancement Method for Immunohistochemical Staining Based On Microwave Oven Heating of Tissue Sections," JHistochem. Cytochem. 39:741-748 (1991); Ikeda et al., "Extraction and Analysis of Diagnostically Useful Proteins from Formalin-Fixed, Paraffin-Embedded Tissue Sections," J. Histochem. Cytochem. 46:397-403 (1998); Pena, et al. "miRNA In Situ Hybridization in Formaldehyde and EDC-Fixed Tissues," Nat. Methods 6:139-141 (2009)). It was hypothesized that EVs are lost from formalin-fixed tissues during processing and imaging steps. Here, it is shown that standard formalin fixation results in loss of EVs from specimens, whereas fixation of proteins with 1-ethyl-3-(3-dimethylaminopropyl) carbodiimide (EDC) retains EVs and allows for EV imaging *in situ.*

The technical challenge with imaging EVs in tissues is that conventional formalin fixation-based methods allows for reversal of crosslinking, and result in escape of EVs from tissues, thus a negative signal. However, EDC-formalin fixation significantly improves retention of EVs in tissues and allows for robust EV imaging *in situ.* This method illuminated a previously unidentified network of functional EVs in normal vitreous humor, a tissue long considered to have few biological functions. Moreover, the vitreous is a potential model to study EVs and ECM. Finally, this fixation technique may be broadly applied for diagnostic purposes for diseases mediated by EVs such as cancer.

Another technical limitation to imaging EVs in tissues and fluids is the inability to determine the spatial localization of EV associated structures. For example, studies have shown that tumor EVs modify tumor cells motility and increase the invasiveness ability (Sung et al., "Directional Cell Movement Through Tissues Is Controlled by Exosome Secretion," Nat Commun 6:7164 (2015)). To study this, the vitreous humor was used as a model system to study the spatial localization of EVs. The vitreous body (vitreous) of the eye is located between the lens and the retina, and is mostly acellular tissue. The vitreous is largely composed of water and extracellular gel matrix of predominantly Type II collagen fibrils in association with hyaluronic acid. The vitreous was used as a model system to solve the dilemma of imaging EVs in biological fluids.

### BRIEF DESCRIPTION OF THE DRAWINGS

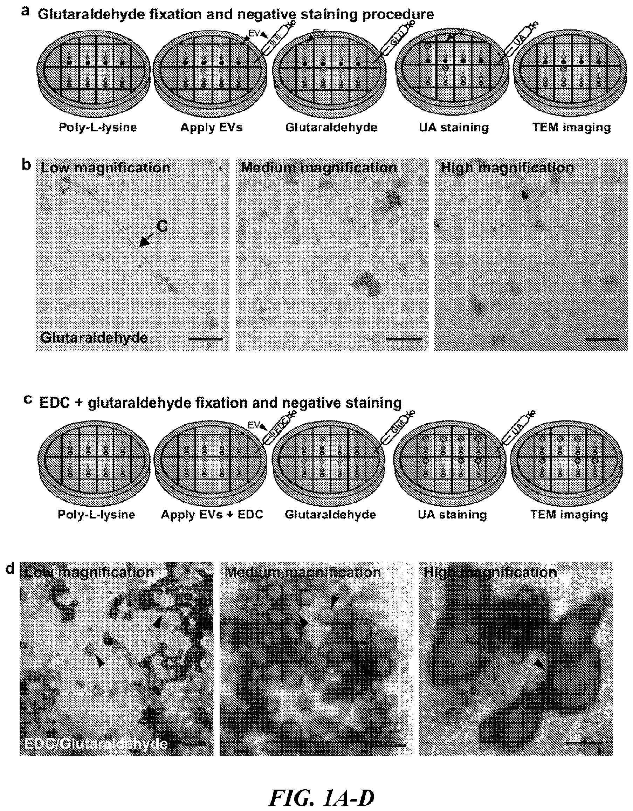

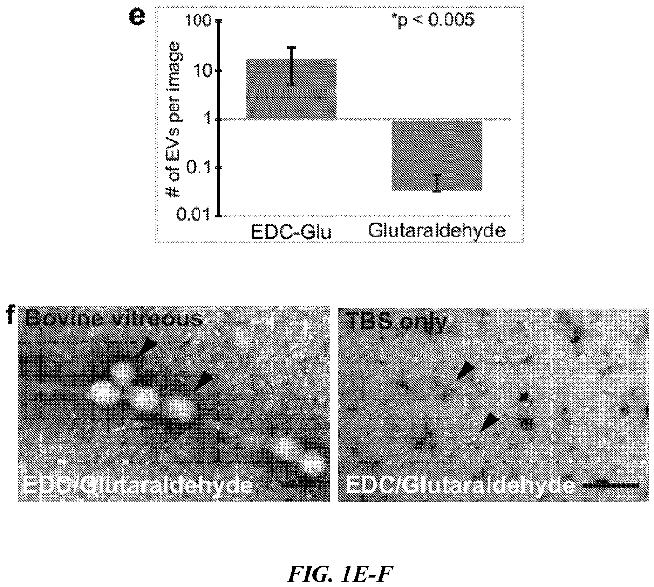

Figures 1A-F are graphical illustrations and transmission electron microscopy (TEM) photomicrographs illustrating that EVs prepared for transmission electron microscopy with glutaraldehyde fixation show few EVs. However, substantially more EVs remain attached to the surface of the electron microscopy grid when using a non-reversible carbodiimide crosslinker. Figure 1A is a schematic diagram showing the steps necessary for glutaraldehyde-based imaging of EVs in liquids using transmission electron microscopy (TEM). (i) A copper grid (disc) coated with formvar (black circle, negative charge) and poly-L-lysine (circle, positive charge) was used for the staining procedure. (ii) A solution of bovine vitreous EVs (small circles, no charge) was applied over the surface of the copper grid coated with formvar and poly-L-lysine. (iii) Excess EV solution was aspirated and the grid was subsequently fixed with conventional glutaraldehyde-fixation (Glut) solution. The Glut solution was removed; the grid washed with water, and (iv) samples were then stained with several applications of uranyl acetate (UA, black circle outline) and lead citrate prior to (v) TEM imaging. Figure 1B are representative photomicrographs of isolated bovine vitreous EVs, fixed to the copper grid with Glut and subsequent UA and lead citrate solution, show no negatively stained EVs at low (left), medium (middle) nor high (right) magnification. Figure 1C is a schematic diagram depicting the protocol for EDC-based fixation of EVs to a copper grid in which EDC is applied to the EVs prior to glutaraldehyde fixation under otherwise identical conditions. Figure 1D are representative TEM photomicrographs of isolated bovine vitreous EVs after EDC-glutaraldehyde-fixation, negative staining and TEM imaging reveal substantially more EVs visualized at low power (left), medium (middle) and high (right) magnification. Figure 1E is a graphical representation of the mean and ± standard deviation that shows significantly more EVs counted per image from EDC fixed specimens (350-fold), when compared to Glut fixed grids (n = 3, counted on average seven images per biological replicate, *p <0.05). Figure 1F Representative TEM photomicrographs of bovine vitreous EVs after EDC-glutaraldehyde-fixation show negative stain surrounding the border of the EV, in contrast to the background signal observed by imaging tris-buffered saline (TBS; mean circular signal size for TBS was < 20 nm), demonstrating the difference between true negative staining versus false positive stain. Scale bars are (B) 600 nm left panel, 125 nm middle panel, and 100 nm right panel; (D) 3 µm left panel, and 2 µm middle panel; and (F) 100 nm left and right panels.

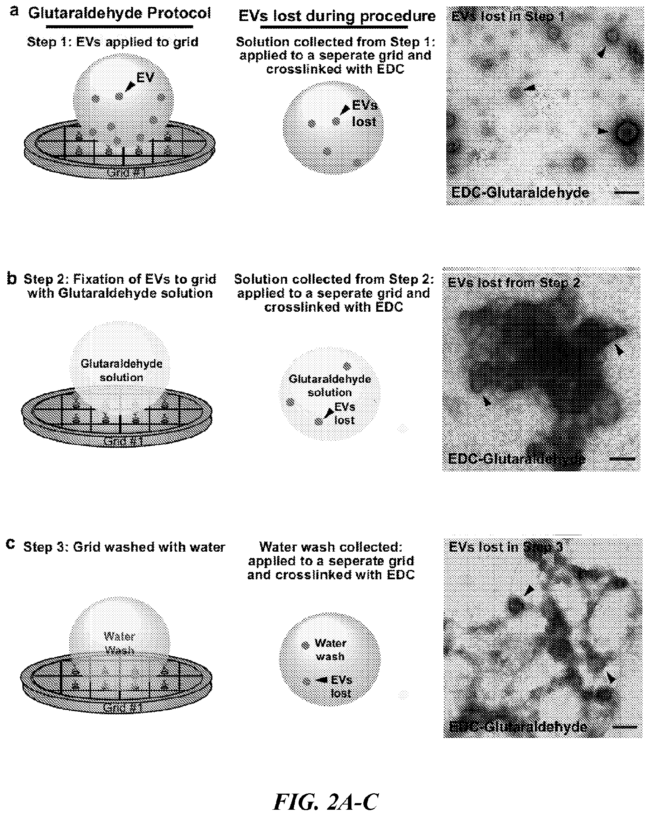

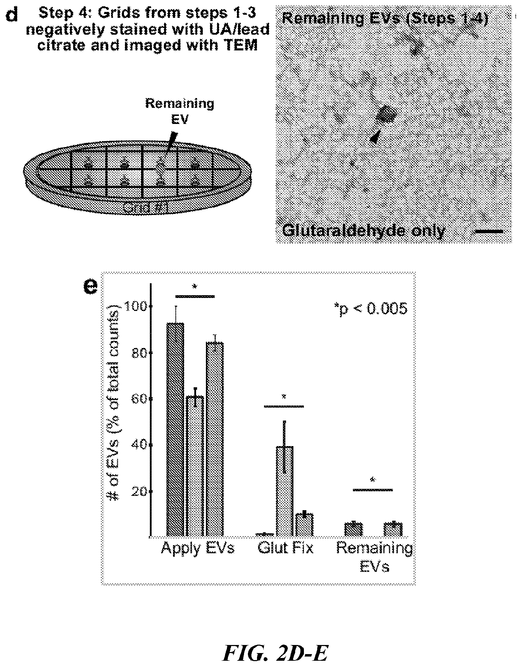

Figures 2A-E are schematic diagrams which show sequential steps for glutaraldehyde-fixation protocols designed to crosslink EVs to an electron microscopy grid. EV loss was assumed to occur at the surface of the grid into the aspirated fluid; and EV escape was monitored and quantified on a separate grid using the more robust non-reversible EDC fixation protocol, negative staining and TEM imaging. Figure 2A is a schematic diagram depicting a solution containing isolated (ultra-centrifuged) aqueous humor derived EVs (grey bubble, solution; small circles, EVs) that was applied to a TEM grid coated with formvar and poly-L-lysine (left). After incubation, the fluid was aspirated from the grid and collected for analysis (middle). The aspirated fluid was applied to a separate grid; subjected to non-reversible EDC fixation, then negatively stained and imaged by TEM. A representative TEM photomicrograph shows a substantial number of EVs (arrowheads) that were lost to the aspirated fluid in step 1 (right). Therefore, the EVs in solution failed to adhere to the electron microscopy grid surface. Figure 2B is a schematic diagram showing the application of glutaraldehyde fixation solution (bubble) to the surface of the grid from step 1 (left). After incubation, the glutaraldehyde fluid is removed and collected for examination (middle). A representative TEM photomicrograph shows a substantial amount of EVs (arrowhead) having leaked into glutaraldehyde-fixation solution from step 2 (right). Figure 2C is a schematic diagram showing the water wash applied to the grid from step 2 (left) and, after incubation, the water wash solution collected (middle) for examination. A representative TEM photomicrograph of solution collected from step 3 and applied to a separate grid, fixed with EDC solution and negatively stained, shows clusters of EVs lost in the wash (right). Figure 2D is a schematic diagram displaying a few EVs (small circle) that remain cross-linked on the grid using the glutaraldehyde fixation protocol (steps 1-4, left). Representative TEM photomicrograph shows a single bovine aqueous humor EV that remains on the copper grid after conventional glutaraldehyde fixation and negative staining (arrowhead). Figure 2E is a graph representing the mean ± standard deviation of the number of EVs (percent of total EVs counted) that were lost to fluid in steps 1 and 2, with few EVs remaining on the grid (*p < 0.05). Scale bars are (A) 500 nm right panel; (B) 100 nm right panel;(C) 400 nm right panel; and (D) 400 nm.

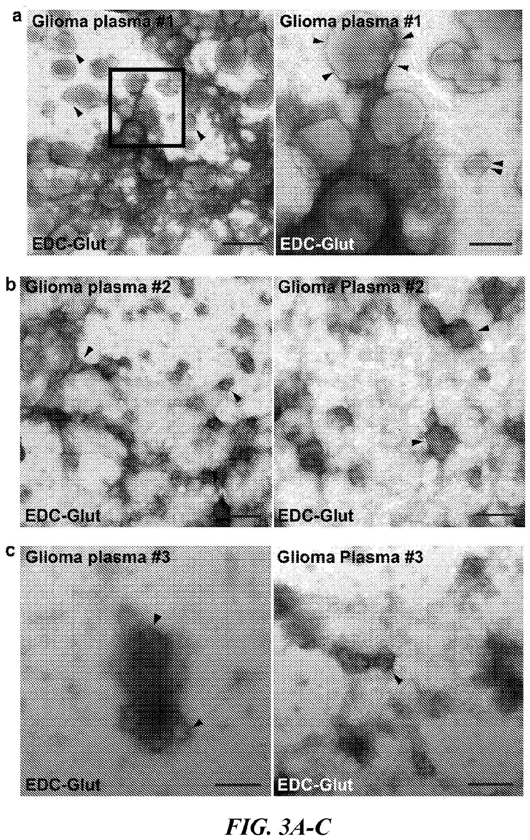

Figures 3A-C are photographs of EVs from patients with glioma. Figure 3A has representative photographs of EVs isolated (ultra-centrifugation purified) from blood (plasma) from patients with glioma after EDC cross-linking and negative staining. TEM images show a numerous EVs of various sizes at low magnification (right). Arrowheads denote EVs with signal (black) surrounding the perimeter of the EV and lower signal (white or grey) in the center. At higher magnification (inset, marked with box), a large diameter EV is noted (encircled between four arrowheads) with negative staining surrounding the perimeter of the EV (right). A smaller EV is highlighted (arrowhead). Figure 3B are representative TEM photographs of second human glioma EVs in plasma from a patient with glioma show relatively smaller EVs (right and left, arrowheads). Figure 3C are representative TEM photographs of a third patient sample of plasma shows glioma derived EVs with the buildup of signal observed surrounding the border of the EV (left and right, arrowhead). Scale bars are (A) 500 nm left panel and 150 nm right panel; (B) 500 nm left panel and 200 nm right panel; and (C) 200 nm left and right panels.

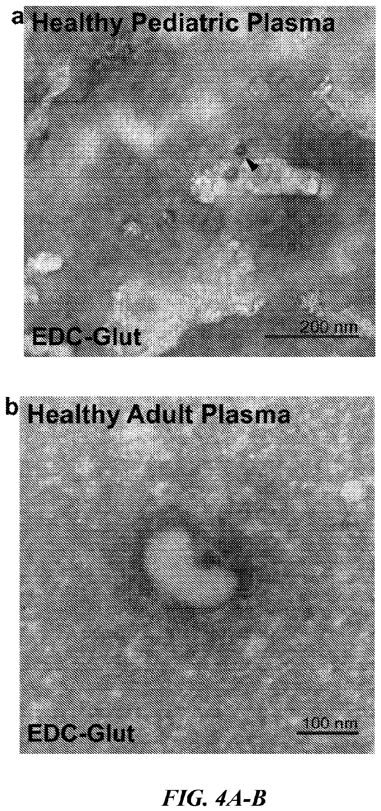

Figures 4A-B are isolated extracellular vesicles visualized in plasma from normal healthy adult and pediatric patients after fixation with EDC and imaged with transmission electron microscopy. Photographs show negatively stained isolated EVs from plasma donated by healthy adult and pediatric patients and subsequently fixed with 1-ethyl-3-(3-dimethylaminopropyl) carbodiimide (EDC) solution. The images show that few EVs are present. Scale bars are 200 nm (A) and 100nm (B).

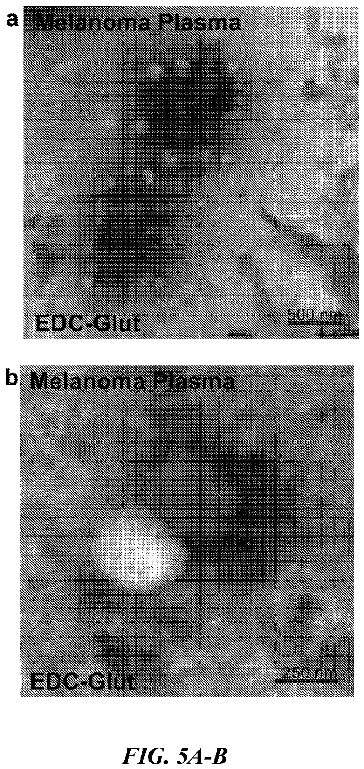

Figures 5A-B are isolated extracellular vesicles visualized in plasma from patients with melanoma after fixation with EDC and imaged with transmission electron microscopy. Figures 5A and B are isolated EVs from plasma in an adult with melanoma and subsequently fixed with 1-ethyl-3-(3-dimethylaminopropyl) carbodiimide (EDC) solution showing numerous negatively stained EVs imaged by TEM. Scale bars are 500 nm (A) and 200 nm (B).

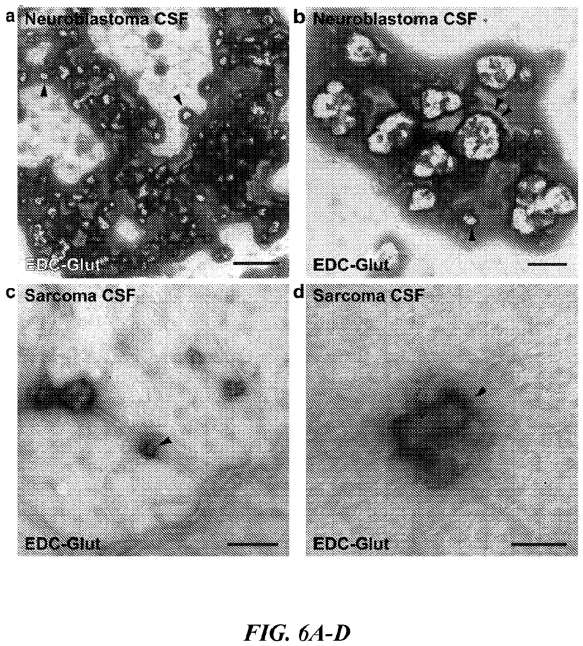

Figures 6A-D are TEM images of EVs from patients with differing types of cancer. Figure 6A is a typical photograph of EVs isolated from cerebral spinal fluid (CSF) from patients with neuroblastoma after EDC cross-linking and negative staining. TEM images show a dense cluster of EVs at low magnification (right). Arrowheads denote EVs with signal (black) surrounding the perimeter of the EV and lower signal (white or grey) in the center. Figure 6B shows a typical example from Figure 6A at higher magnification. A large diameter EV is noted (double arrowheads) with negative staining surrounding the perimeter of the EV (right). Figure 6C is a representative TEM photograph of isolated EVs in CSF from a patient with sarcoma, fixed with EDC-glutaraldehyde, negatively stained, and imaged with TEM. The pictures show EVs small in diameter (left, arrowhead) when visualized at low magnification. Figure 6D shows a typical example of a similar sample from Figure 6C at higher magnification, the buildup of signal is observed surrounding the border of the sarcoma derived EVs (left, arrowhead). Scale bars are (A) 2 µm; (B) 500 nm; (C) 250 nm; left panel and (D) 50 nm.

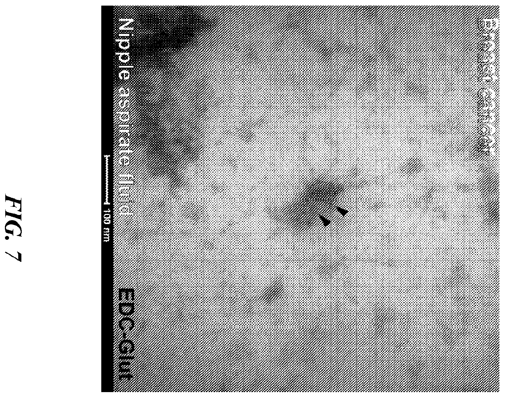

Figure 7 is an image of extracellular vesicles visualized in nipple aspirate fluid obtained from patients with a diagnosis of breast cancer after fixation with EDC and imaged with transmission electron microscopy. Diluted nipple aspirate fluid containing EVs from an adult with breast cancer and subsequently fixed with 1-ethyl-3-(3-dimethylaminopropyl) carbodiimide solution show negatively a stained EV imaged by TEM. Scale bar is 100 nm.

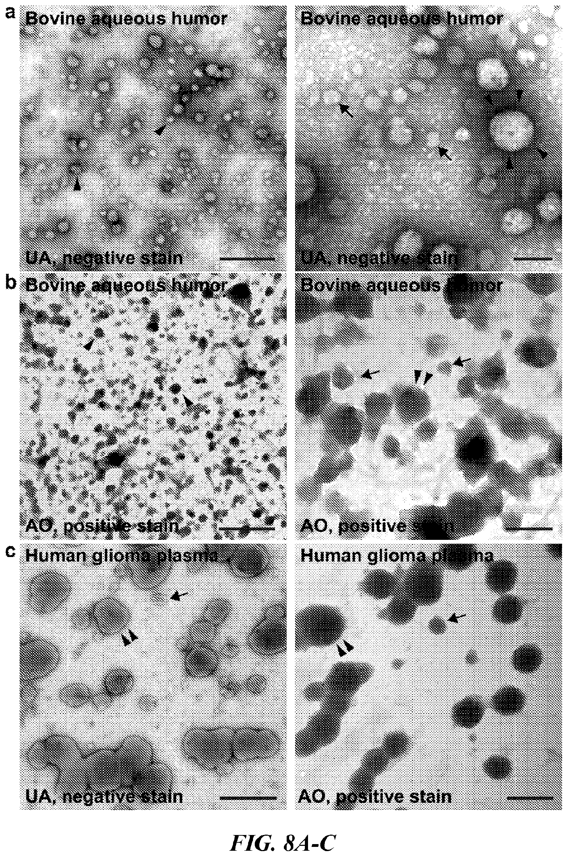

Figures 8A-C identify EVs with a nucleic acid dye that allows for positive staining. Figure 8A contains representative photographs of isolated EVs from bovine aqueous humor fixed with EDC-glutaraldehyde and subsequently labeled by UA and lead citrate; showing numerous negatively stained EVs (arrowhead) at low power (left) and high power (right). A definitive example of a negatively stained EV is shown as the accumulation of signal (black) around the perimeter of a round object and minimal signal (white) within the EV (Figure 8A, right). Four arrowheads encircling the EV mark the vesicle. Examples of ambiguous negatively stained objects are shown (arrow), with an indistinct signal surrounding the perimeter, and lower signal (white) in the center; potentially a false-positive EV. Figure 8B is representative TEM images of isolated EVs from bovine aqueous humor after EDC-glutaraldehyde-fixation and incubation with an electron dense, nucleic acid selective dye, acridine orange (AO). Images show several "positively-stained" EVs with a substantial amount of signal (black) within the EV (Figure 8B, arrowhead) with a clear background. Hundreds of EVs are shown at low power (left). The AO dye marks large (double arrowheads) and small exosomes (arrow) with minimal background (right). Figure 8C, left is a representative TEM photomicrograph of negatively stained EVs isolated from plasma from a patient with a glioma, fixed with EDC-glutaraldehyde, and stained with UA and lead citrate. The image shows the negative stain surrounding the perimeter of a large glioma EV (left, double arrow) and a smaller exosome (left, arrow). Figure 8C right is a representative TEM photomicrograph showing glioma EVs fixed with EDC-glutaraldehyde, and labeled with AO dye, which demonstrate positive staining within the glioma EV. A larger EV is shown (double arrow), as well as an exosome (arrow). In comparing negative (UA and lead citrate) and positive (AO) staining, EVs stained with UA and lead citrate (Figure 8C, left) are similar in size and shape as EVs stained with AO (Figure 8C, right). Scale bars are (A-B) 1 µm left panel and 200 nm right panel; and (C) 500 nm left and right panels.

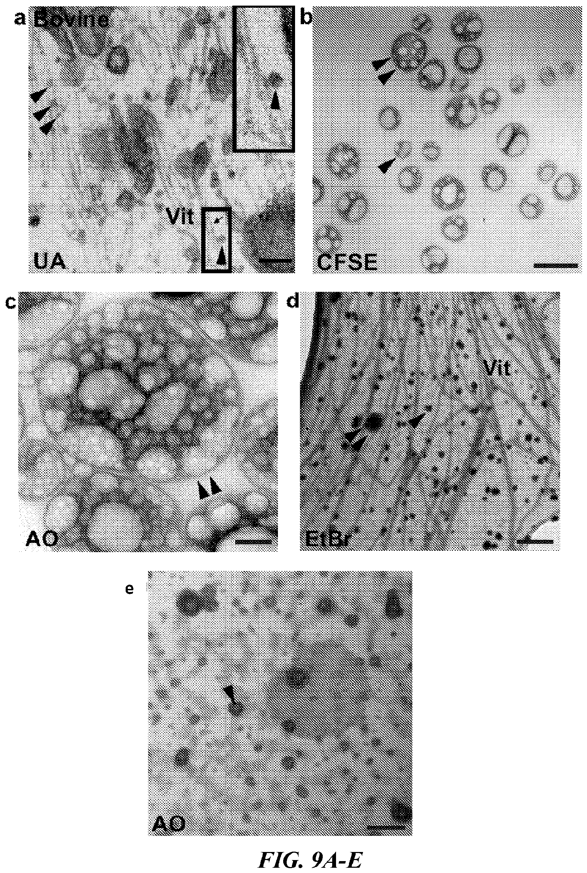

Figures 9A-E demonstrate that bovine and human vitreous humor contains extracellular vesicles. Figure 9A is a representative transmission electron microscopy photomicrograph of bovine vitreous tissue sections stained with uranyl acetate (UA) and lead citrate show a substantial number of EVs that are pleomorphic in size (arrowhead). The inset (upper right corner) is an enlargement of the area-enclosed box in the lower right corner and shows an EV (arrowhead). Figure 9B is a representative TEM photomicrograph of EVs isolated from bovine vitreous and stained with the electron dense protein stain, CSFE, depict EV morphology and large EVs (double arrowhead). Figure 9C is a representative TEM photomicrograph of EVs isolated from bovine vitreous and stained with the electron dense nucleic acid stain acridine orange (AO) showing numerous EVs that are pleomorphic in size (smaller EV marked with arrowhead, larger EV with double arrowhead) and that bear a positive nucleic acid signal. Figure 9D is whole mounted bovine vitreous stained with ethidium bromide (EtBr), an electron dense nucleic acid stain, showing multiple EVs (arrowheads). Figure 9E is a representative TEM photomicrograph of EVs isolated from post-mortem human vitreous and stained with AO show EVs (arrowhead) with positive nucleic acid signal. Scale bars are (A) 100 nm, (B, D-E) 200 nm, and (C) 50 nm.

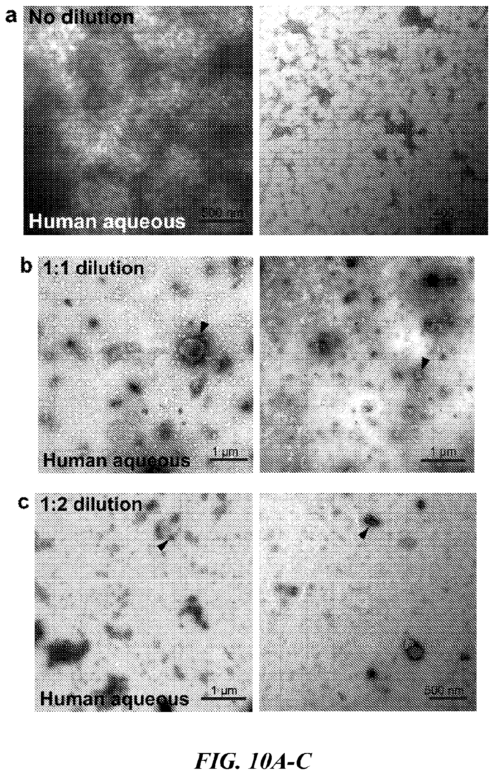

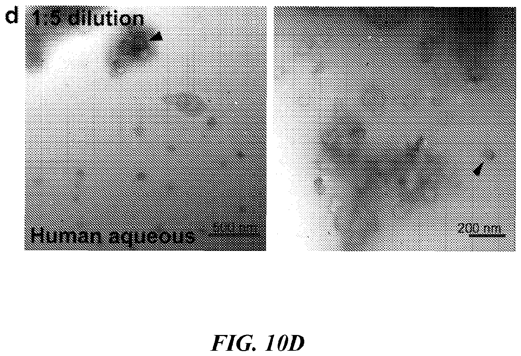

Figures 10A-D are photographs of EVs directly imaged (i.e. the EVs were not isolated with ultracentrifugation) from healthy patients' aqueous humor and show that dilution of the biological fluid is necessary for reducing the non-specific background staining and improve the signal given by EVs. Figure 10A is a photograph depicting a biological fluid, human aqueous humor, that was undiluted, applied to a TEM grid coated with formvar and poly-L-lysine, fixed with EDC-glutaraldehyde, negatively stained with uranyl acetate, and imaged with TEM. The left and right photograph show substantially high background (diffuse black staining) and no evidence of easily identifiable EVs (left panel); and possible EVs in the right panel. Figure 10B are photographs (left and right) depicting the aqueous humor diluted 1:1 with buffered saline and show reduced background (black staining), and build up of negative stain, consistent with the morphology of an EV (arrowhead). Figure 10C (left and right panels) are photographs depicting the aqueous humor diluted 1:2 with buffered saline and showing a reduced background and EVs (arrowhead). Figure 10D (left and right panels) are photographs depicting the aqueous humor diluted 1:5 with buffered saline and showing a reduced background and EVs (arrowhead). Scale bars are (Figure 10A) 1 µm left panel and 500 nm right panel; (Figure 10B) 1 µm left panel and 500 nm right panel; (Figure 10C) 1 µm left and right panel; and (Figure 10D) 500 nm left panel and 200 nm right panel.

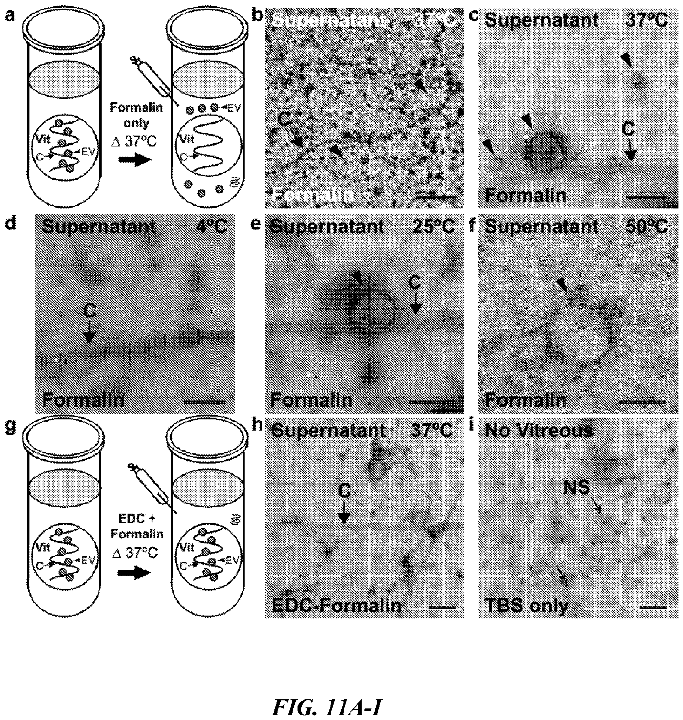

Figures 11A-I demonstrate that extracellular vesicles escape from formalin-fixed bovine vitreous tissues and are retained with 1-ethyl-3-(3-dimethylaminopropyl) carbodiimide - formalin fixation. Figure 11A is a schematic diagram showing formalin-fixed vitreous (Vit) tissue immersed in wash buffer (supernatant) and heated to 37°C results in escape of EVs (EV, arrowhead) and vitreous collagen (Figure 11C, closed arrow) into the supernatant. Figures 11B-C are representative TEM photomicrographs of supernatant collected from formalin-fixed bovine vitreous tissue after incubation at 37°C and uranyl acetate (UA) and lead citrate staining show evidence of collagen strands (Figure 11C, closed arrow) and numerous EVs (arrowhead) that were present in wash buffer imaged at low (Figure 11B) and high power (Figure 11C). Figure 11D are representative TEM photomicrographs of supernatant collected from bovine-fixed vitreous tissue kept at 4°C and stained with heavy metals reveal few collagen strands (Figure 11C, closed arrow), but few EVs. Figures 11E-F are images of supernatant collected from formalin-fixed vitreous tissue after incubation at or above 25°C showing collagen strands (Figure 11C, closed arrow) and EVs (arrowhead). Figure 11G is a schematic diagram showing EDC-formalin-fixed vitreous tissue (Vit) immersed in wash buffer and heated to 37°C resulted in retention of EVs (arrowhead) in the tissue, with no loss of EVs and minimal loss of vitreous collagen strands (Figure 11C, closed arrow) into the supernatant. Figure 11H are representative TEM photomicrographs of supernatant from EDC-formalin-fixed vitreous tissue after incubation at 37°C and heavy metal staining show few collagen strands (Figure 11C, closed arrow), but no EVs in the supernatant. Figure 11I is a representative image of a specificity control, tris buffered Saline (TBS) alone, showing no collagen fibers nor EVs in the supernatant, but it does show non-specific punctate staining of electron dense foci (NS, open arrow) measuring less than 20 nm. Scale bars are (B) 4 µm, (C, D) 200 nm, (E) 75 nm, (F) 40 nm, and (G-H) 200 nm.

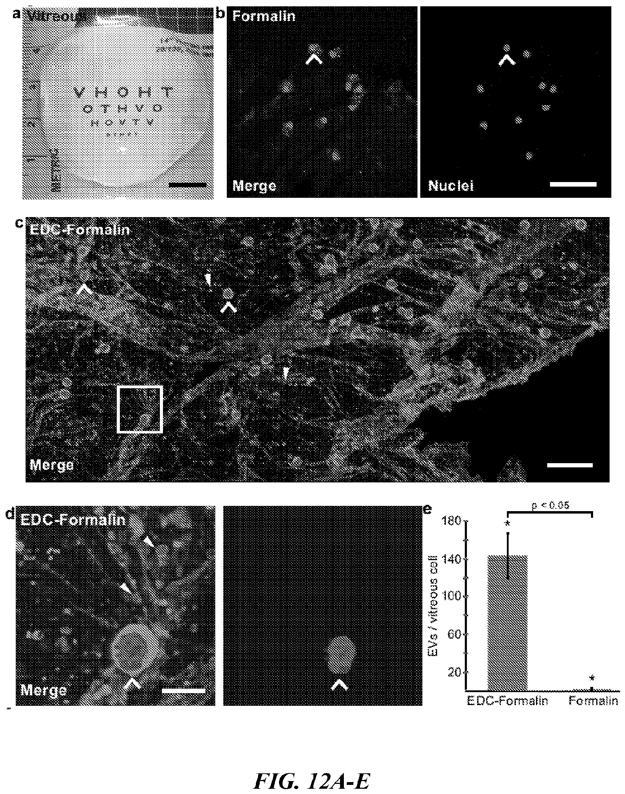

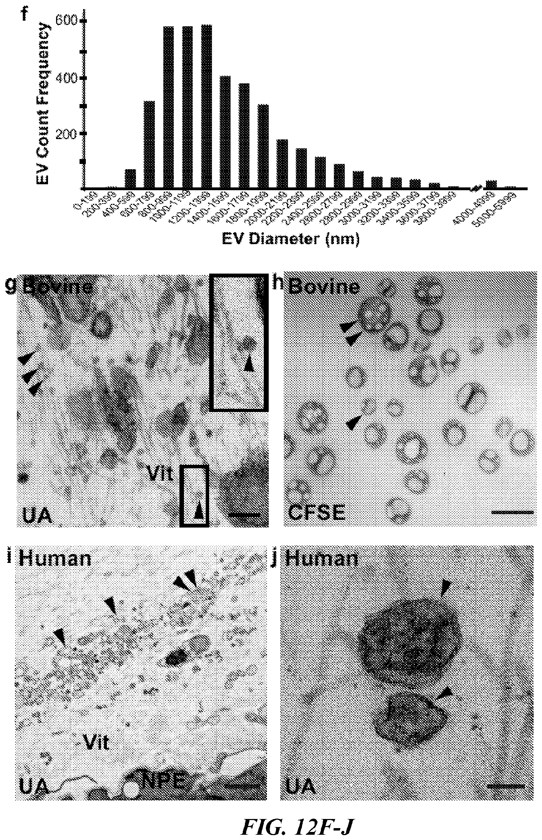

Figures 12A-J shows that 1-ethyl-3-(3-dimethylaminopropyl) carbodiimide (EDC)-formalin fixation of bovine vitreous retains extracellular vesicles, when compared to formalin fixation alone. Figure 12A is a gross image of bovine vitreous placed on a vision testing card demonstrates the highly transparent, gel-like structure. Figure 12B is a representative multifocal microscopy (MPM) photomicrographs of whole mount bovine vitreous specimens fixed with formalin alone and stained with CFSE to mark protein and Hoechst to mark nuclei. CFSE signal is observed surrounding the nuclei **(left panel,** open arrow), but not in the extracellular space **(right panel,** open arrow). Nuclei staining show no extracellular signal **(left panel,** open arrow). Figure 12C are representative MPM photomicrographs of EDC-formalin-fixed vitreous stained with CFSE and Hoechst. Overlay of image shows positive signal consistent with cell bodies (open arrowhead) and foci of extracellular protein signal (closed arrowheads) consistent in size and shape with EVs. Figure 12D is the inset of Figure 12C (white box), shows multiple round intracellular foci **(left panel,** open arrowhead) surrounding the area of nuclear stains **(right panel,** open arrowhead). Numerous focal extracellular protein signals are also observed **(left panel**, closed arrowheads), consistent in size and shape with EVs, and no extracellular DNA is observed. Figure 12E is a graph representing the mean ± standard deviation of the number of EVs per vitreous cell showing that EDC-formalin-fixed vitreous exhibit significantly more EVs than formalin-fixed vitreous *p< 0.05. Figure 12F is a graphical representation of the frequency distribution of bovine vitreous EV diameter as measured by MPM. Figure 12G is representative TEM photomicrographs of bovine vitreous tissue sections stained with uranyl acetate (UA) and lead citrate show a substantial number of EVs that are heterogeneous population size (arrowhead). The inset (upper right corner) is an enlargement of the area-enclosed box in the lower right corner and shows an EV (arrowhead). Figure 12H is a representative TEM photomicrograph of EVs isolated from bovine vitreous and stained with the electron dense protein stain, CSFE, depict EV morphology, with both smaller (arrowhead) and larger EVs (double arrowhead). Figures 12I-J are representative TEM photomicrographs of postmortem human eye sections stained with UA and lead citrate show a substantial number of EVs in the extracellular matrix near the vitreous base (Vit), adjacent to the non-pigmented epithelium (NPE) of the ciliary body (smaller EVs marked with arrowhead, larger EVs with double arrowhead). Scale bars are (A) 1 cm, (B) 40 µm, (C) 50 µm and (D) 10 µm, (G) 100 nm, (H) 200 nm, (I) 2 µm, (J) and 100 nm.

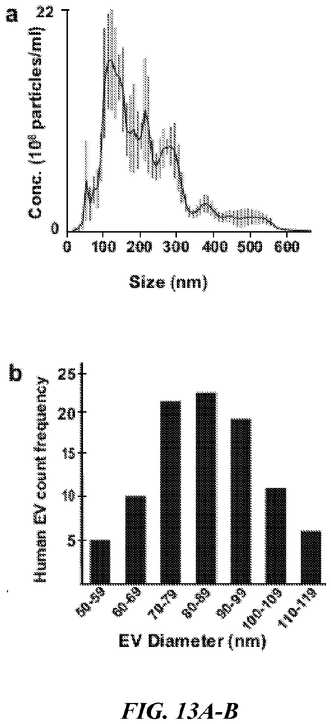

Figures 13A-B show that bovine and human vitreous humor contains a heterogeneous population of extracellular vesicles. Figure 13A is a graphical representation of the mean (line) ± standard error (bars) of the concentration of EVs according to EV diameter, based on nanoparticle tracking analysis of EVs isolated from bovine vitreous. Figure 13B is a graphical representation of frequency distribution of post-mortem human vitreous EV diameter measured by TEM imaging.

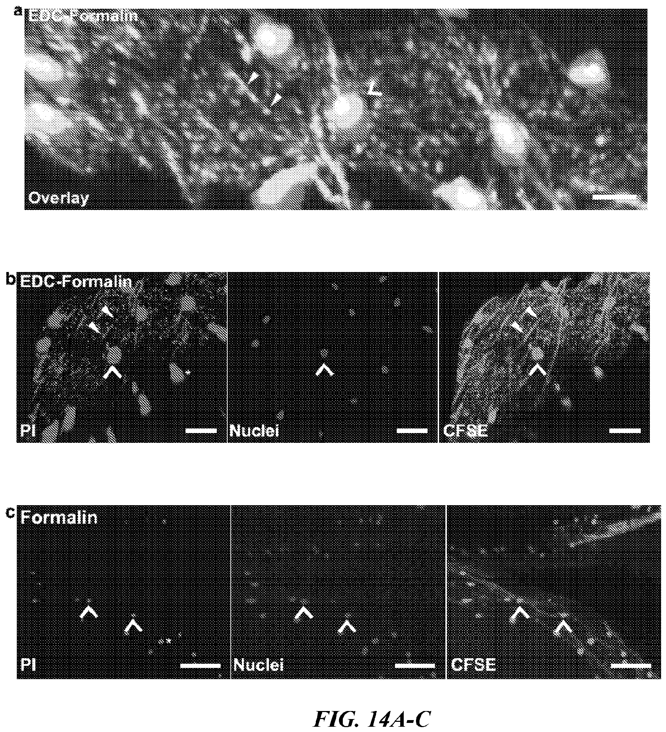

Figures 14A-C illustrates the fixation of bovine vitreous with 1-ethyl-3-(3-dimethylaminopropyl) carbodiimide -formalin retaining vitreous extracellular vesicles and extracellular RNA *in situ.* Figures 14A-B are representative confocal fluorescent photomicrographs of EDC-formalin fixed whole mount bovine vitreous specimens stained with propidium iodide (PI) to mark DNA and RNA, Hoechst to visualize DNA and nuclei, and carboxyfluorescein succinimidyl ester (CFSE) to stain for protein. Figure 14A is an overlay of images from EDC-formalin-fixed bovine vitreous show positive signal consistent with cell bodies (Figure 14A, open arrow) and foci of extracellular RNA (closed arrowhead) and extracellular protein (closed arrowhead) consistent in size and shape with EVs. Figure 14B is a representative confocal fluorescent photomicrographs of EDC-formalin-fixed vitreous show multiple round cellular foci **(both panels,** open arrowhead) and numerous focal signals of extracellular RNA **(left panel**, closed arrowhead, PI stain) and extracellular protein **(right panel**, closed arrowhead, CFSE stain) between the cells. Figure 14C are representative photomicrographs of whole mount bovine vitreous fixed with formalin alone show signal for RNA **(left panel,** open arrowhead, PI) in the nucleus, similar to nuclei staining **(middle panel,** open arrowhead, Hoechst). Formalin-only fixed vitreous show no foci of extracellular RNA signal **(left panel).** CFSE stain shows cellular protein signal **(right panel,** open arrow), but no EV-shaped extracellular protein signal **(right panel**, no punctate staining observed between open arrows). The cell size appears smaller in the formalin only fixation, presumably due to improved retention of cytoplasmic RNAs and protein with EDC-formalin fixation as compared to formalin fixation alone. Scale bars are (A) 25 µm, (B,C) 50 µm.

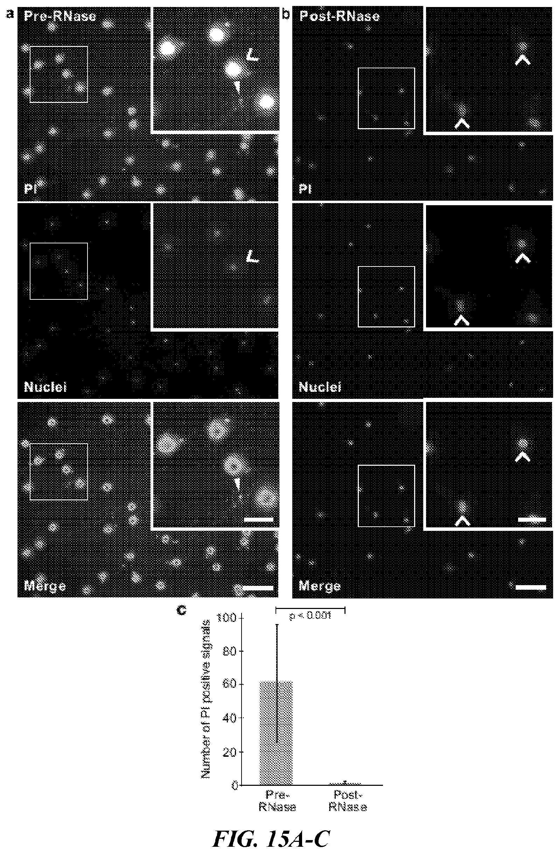

Figures 15A-C display RNase treatment of 1-ethyl-3-(3-dimethylaminopropyl) carbodiimide-formalin fixed bovine vitreous stained with propidium iodide (PI) showing a reduced extracellular nucleic acid signal. Figure 15A are a low-power wide-field fluorescent photomicrographs of whole mount bovine vitreous specimens crosslinked with EDC-formalin and stained with PI (Figure 15A, **top panel)** showing signal in the extracellular environment of vitreous tissue (denoted with closed arrowhead, **inset);** nuclei labeled (Figure 15A, **middle panel,** Hoechst, and merged images are shown **(bottom panel).** Vitreous cell nuclei stain positive with PI and Hoechst; co-localized signals are shown **(bottom panel, inset).** Cells are denoted with an open arrow (Figure 15A, **middle** and **bottom panels,** inset) and foci of extracellular PI signal are marked with a closed arrowhead **(top** and **bottom panels, inset).** Nuclei were stained, and no extracellular DNA signal is observed **(bottom panel).** Figure 15B are photomicrographs of whole mount bovine vitreous fixed with EDC-formalin and treated with RNase A. Samples were stained with PI **(top panel),** Hoechst **(middle panel),** and merged images are shown **(bottom panel).** RNase A treated samples show no evidence of extracellular RNAs as demonstrated by the lack of signal between the cell bodies **(top** and **bottom panel)** and show no signal between two cell nuclei **(middle** and **bottom panels,** open arrows Hoechst). The PI signal for cytoplasmic RNA in RNase A treated samples (Figure 15B, **top** and **bottom panels)** appear smaller than pre-RNase treated samples (Figure 15A, **top** and **bottom panels),** presumably due to EDC-formalin retaining more cytoplasmic RNA. Figure 15C is a graphical representation of the mean ± standard deviation of the foci of extracellular signal for EDC-formalin fixed tissues stained with PI pre-RNase treatment and after RNase treatment show significantly fewer EVs after RNase treatment, p < 0.001. Scale bars are (A, B) 50 µm and (A inset, B inset) 20 µm.

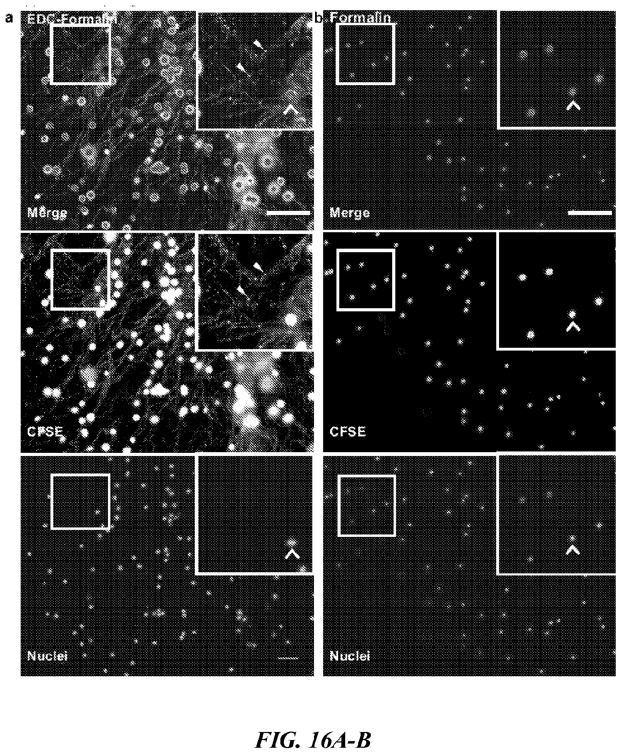

Figures 16A-B show wide-field epi-fluorescent microscopy imaging of 1-ethyl- 3-(3- dimethylaminopropyl) carbodiimide -formalin fixation of bovine vitreous extracellular vesicles. Figures 16A-B are low-power wide field fluorescent photomicrographs of whole mount bovine vitreous specimens crosslinked with EDC-formalin (Figure 16A) or formalin alone (Figure 16B). Figure 16A are representative photomicrographs of bovine vitreous fixed with EDC-formalin and stained with CFSE to label protein **(top** and **middle panel,** white) and Hoechst to label nuclei (bottom panel) show multiple round cellular foci **(all panels,** open arrowhead) with numerous extracellular protein signals **(top** and **middle panels**, closed arrowhead, CSFE, white) consistent with EVs. Figure 16B are photomicrographs of whole mount bovine vitreous fixed with formalin only show nuclear stain **(middle** and **bottom panels,** Hoechst, blue) co-localizing with CFSE **(top** and **middle panels,** white), consistent with cellular DNA and nucleic acid **(all panels,** open arrowhead), respectively. There is no evidence of extracellular protein signal **(top** and **middle panel,** CSFE, white). The CFSE stained cell size appears smaller in the formalin only fixation (Figure 16B, **middle panel,** white) as comparing to EDC-formalin fixation (Figure 16A, **middle panel,** white), presumably due to EDC-formalin retaining more small cellular protein as compared to formalin fixation alone. Scale bars are (A, B) 100 µm.

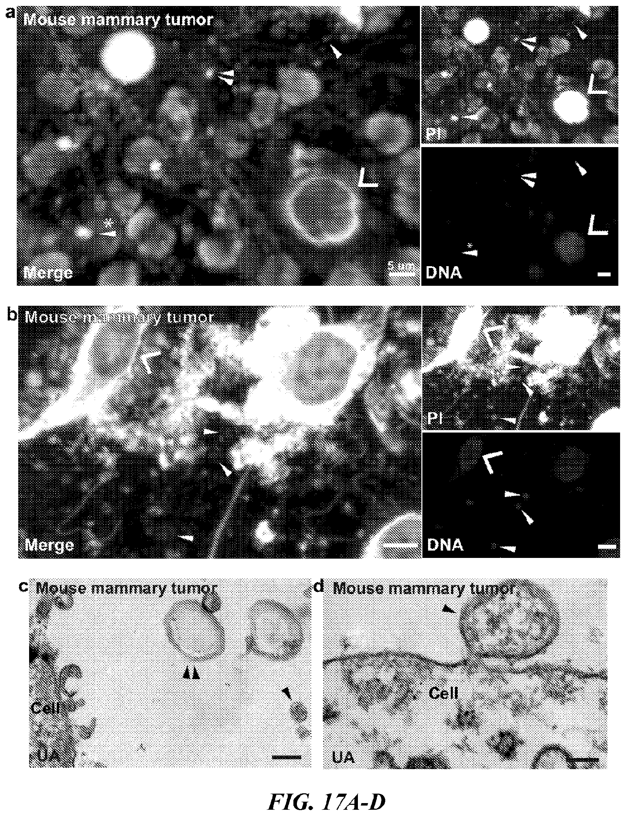

Figures 17A-D illustrate EDC-formalin fixation of metastatic breast cancer allowing for imaging of tumor extracellular RNA and extracellular DNA. Figures 17A-B are representative MPM photomicrographs of an EDC-formalin-fixed 4T1 mouse mammary carcinoma tumor cell line that was transplanted into the mammary fat pad of a mouse (syngeneic graft) showing EV-shaped extracellular RNA signal in the extracellular space (closed arrowheads). Tumors were dissected, fixed with EDC-formalin, and nucleic acids were labeled with PI (white signal only), DNA stained (Hoechst), and images were captured near the tumor surface within the extracellular matrix (Figure 17A, right). An overlay image shows signal from a cell (open arrowhead, Hoechst) and numerous foci of extracellular RNA (closed arrowhead, PI) between the cells consistent in size and shape with EVs. The photomicrographs show a heterogeneous population of EVs and highlighted are a small microvesicle (single arrowhead, ~270 nm), medium microvesicle (double arrowhead, ~850 nm) and an apoptotic body (arrowhead with asterisk, ~1.7 µm). Figure 17B are representative MPM photomicrographs of an EDC-formalin-fixed 4T1 mouse mammary carcinoma tumor showing signal from cell (open arrowhead, Hoechst) as well as co-localization of PI (RNA and DNA) with the DNA stain (Hoechst) in the extracellular space (closed arrowhead). Figure 17C is a representative transmission electron microscopy (TEM) photograph of an EDC-glutaraldehyde-fixed 4T1 mouse mammary carcinoma tumor shows a heterogeneous population of EVs (arrowhead) adjacent to a cell (labelled, Cell). Larger EVs are shown (double arrowhead, ~510 nm) and an exosome is marked (single arrowhead, ~146 nm). Figure 17D is a representative TEM photograph showing an EV (arrowhead, ~373 nm) connected to a cell membrane. Scale bars are (A, B) 5 µm, (C) 250 nm, and (D) 125 nm.

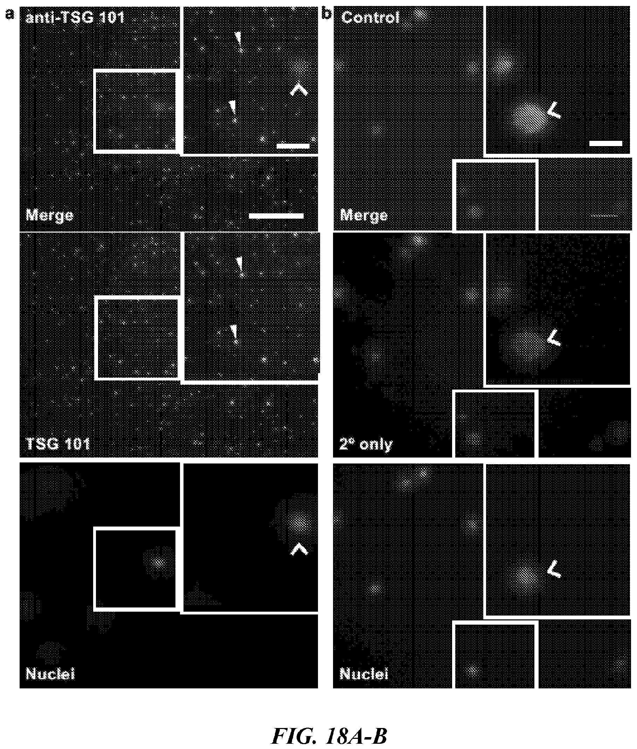

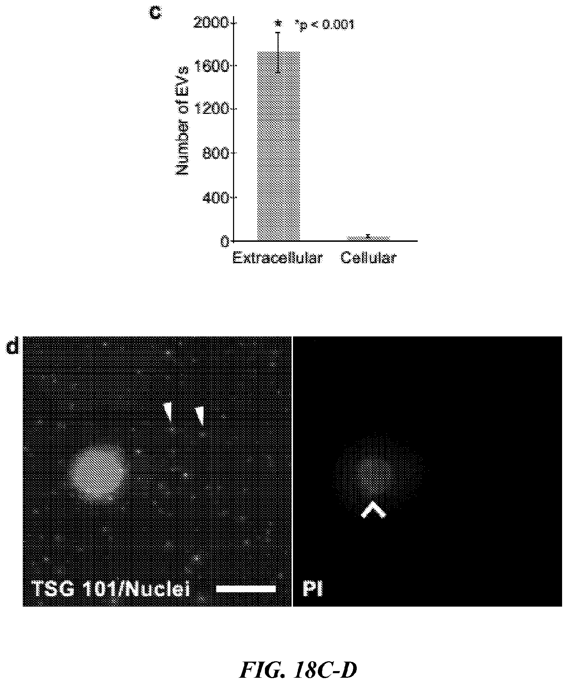

Figures 18A-D display the immunohistochemistry results of staining of extracellular vesicle (EV)-specific protein TSG-101 in a normal bovine vitreous. Figure 18A shows representative wide-field fluorescent photomicrographs of whole mount bovine vitreous specimens fixed with formalin and processed at cold temperatures demonstrate immunohistochemical stain for the EV-associated protein, TSG-101, in the extracellular space **(top** and **middle,** arrowhead, Alexa 488). The inset **(all panels,** top right) is a higher magnification image of the box in the middle **(all panels).** Nuclei are marked with Hoechst counterstain **(top** and **bottom,** open arrow). Hundreds of punctate extracellular protein signals were observed **(top** and **middle,** Alexa 488). No evidence of extracellular DNA was observed **(bottom,** Hoechst). Figure 18B are representative photomicrographs from specificity controls for TSG-101 immunohistochemistry: whole mount normal bovine vitreous labeled with non-specific IgG antibody. The inset **(all panels,** top right) is a higher magnification image of the box in the middle **(all panels).** Signal was observed surrounding the nuclei **(top** and **middle,** Alexa 488). Images show no evidence of extracellular TSG-101 signal **(top** and **middle). Nuclei are marked (top** and **bottom,** Hoechst). Figure 18C is a graphical representation of mean ± standard error for TSG-101 signal in extracellular and intracellular spaces, *p<0.001. Figure 18D displays positive signal for TSG-101 in the extracellular space of the formalin-fixed vitreous **(left,** Alexa 488). Nuclei are labeled with Hoechst **(left)** and PI **(right).** There is no evidence of extracellular RNA in formalin-fixed samples **(right,** PI). Scale bars are (Figures 18A,B) 40 µm and (Figure 18A inset, Figure 18B inset and Figure 18D) 10 µm.

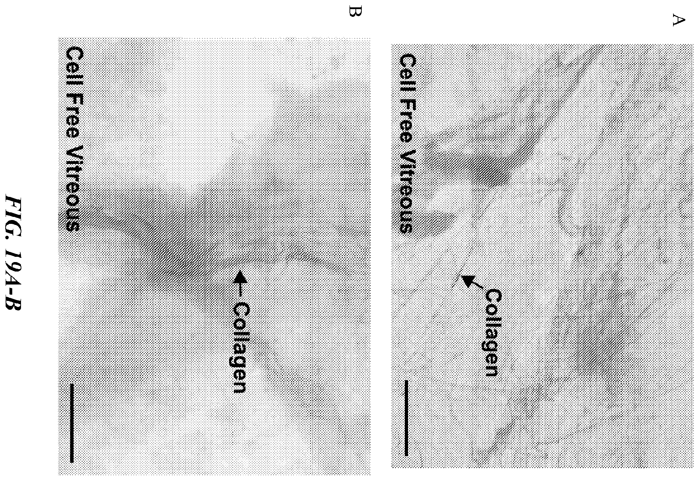

Figures 19A-B are images of bovine vitreous free of cells after low-speed centrifugation. Representative low power light photomicrographs of whole mount bovine vitreous after low-speed centrifugation followed by hematoxylin and eosin staining shows eosinophilic signal consistent with vitreous collagen (arrow) without evidence of hematoxylin stained cellular nuclei. Scale bars are 50 µm.

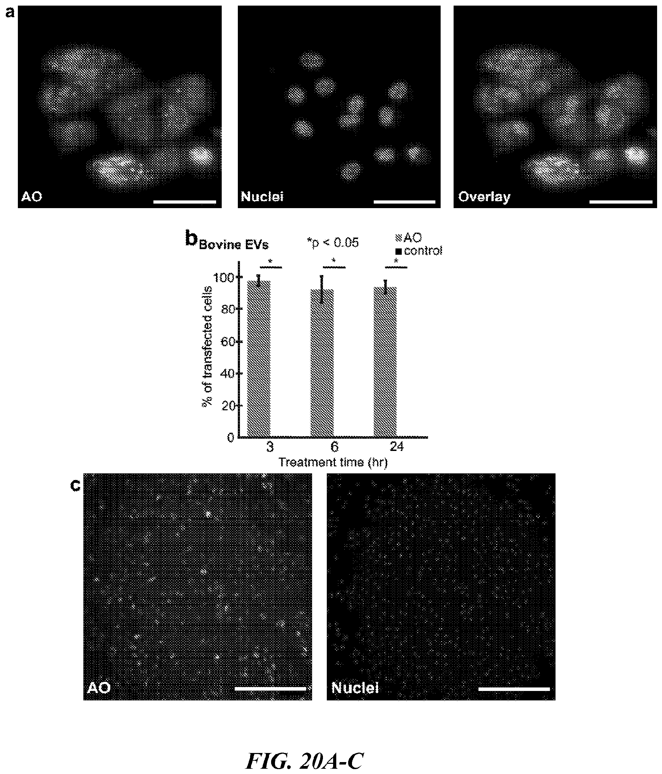

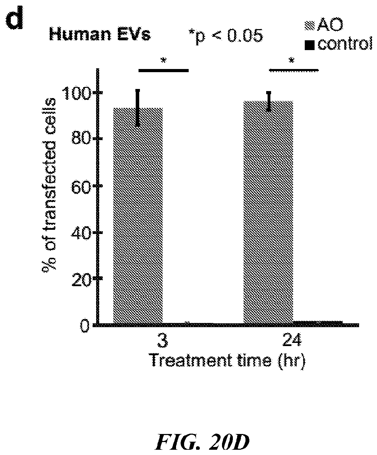

Figures 20A-D displays that human and bovine vitreous extracellular vesicles can transfer endogenous RNA into cultured cells. Figure 20A are representative confocal photomicrograph images of a human retinal pigment epithelial cells (ARPE-19) after 24h treatment with a bolus of bovine vitreous EVs that were pre-labeled with the nucleic acid stain acridine orange (AO). Images show uptake of labelled EV-RNA in ARPE-19 cells **(left** and **right panels**, AO). Nuclei are labeled **(middle** and **right panels**, Hoechst), and a merged image **(right panel)** show transfection of ARPE-19 cells, with AO signal in the cytoplasm. Figure 20B is a graphical representation of mean ± standard deviation of the transfection efficiency (% of cells transfected) for ARPE-19 cells treated with bovine vitreous EVs (n = 3, *p<0.05 versus controls). Figure 20C are representative wide-field epi-fluorescent low-power photomicrographs of ARPE-19 cells treated with a 3 h bolus of EVs that were isolated from post-mortem human vitreous and pre-labeled with AO. Images show transfection of cells **(left panel,** AO). Nuclei were marked **(right panel,** Hoechst). Figure 20D is a graphical representation of the mean ± standard deviation of the transfection efficiency (% of cells transfected) for ARPE-19 cells treated with human vitreous EVs (n = 3, *p<0.05 versus controls). Scale bars are (Figure 20A) 50 µm, (Figure 20C) 15 µm, and (Figure 20D) 100 µm.

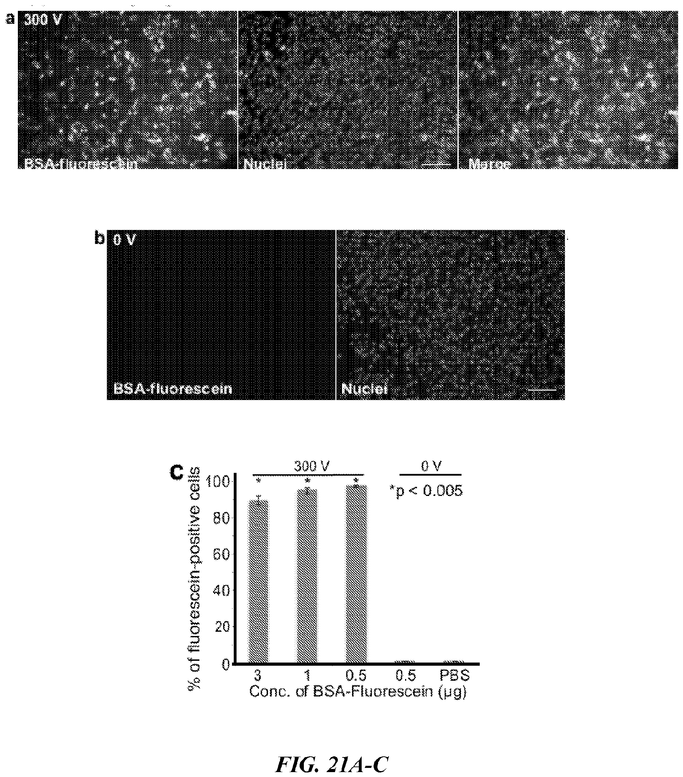

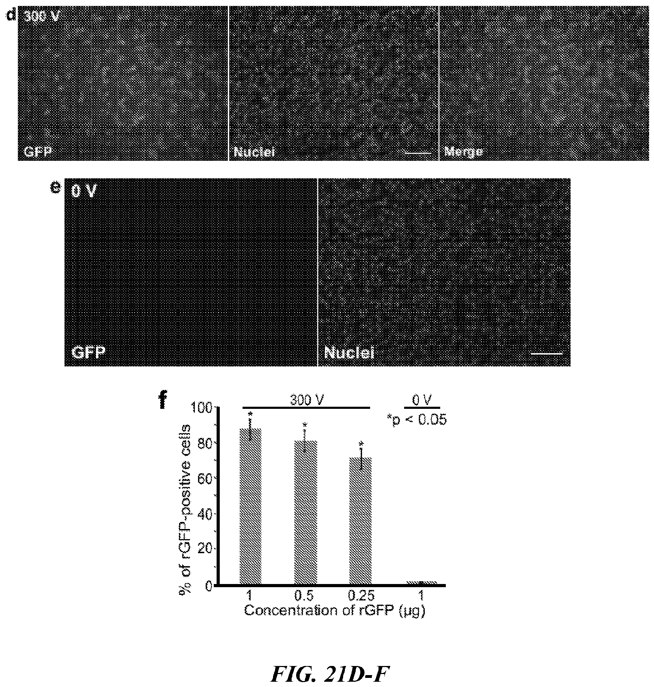

Figures 21A-F are images and graphical representation of the delivery of recombinant bovine serum albumin (BSA) protein and recombinant green fluorescent protein (GFP) by bovine vitreous extracellular vesicles to cultured human retinal pigment epithelial (ARPE-19) cells. Figure 21A are representative photomicrograph of ARPE-19 cells treated with a bolus of bovine vitreous EVs that had been pre-loaded with 1 µg BSA conjugated to fluorescein by electroporation at 300 V show fluorescein staining **(left)** in the cytoplasm. Nuclei are labelled **(middle,** Hoechst stain), and a merged image **(right)** shows substantial number of cells transfected. Figure 21B are representative photomicrographs of ARPE-19 cells treated with a bolus of bovine vitreous EVs that had been mixed with BSA-fluorescein without electroporation (0 V, control) show no fluorescein staining **(left).** Nuclei are labelled **(right,** Hoechst stain). Figure 21C is a graphical representation of the mean ± standard deviation transfection efficiency (% of cells transfected) of ARPE-19 cells treated with vitreous EVs loaded with 3 µg, 1 µg, or 0.5 µg BSA-fluorescein by electroporation at 300 V, with EVs loaded with 0.5 µg BSA-fluorescein without electroporation (0 V, control), or with PBS alone without electroporation (0 V, control). *p < 0.005 for each BSA-fluorescein dosages loaded at 300 V vs. each control at 0 V. Figure 21D are representative photomicrographs of ARPE-19 cells after application of a bolus of bovine vitreous EVs that had been pre-loaded with 1 µg of recombinant GFP by electroporation at 300 V showing positive GFP staining **(left)** in the cytoplasm. Nuclei are labeled **(middle,** Hoechst stain), and a merged image **(right)** shows substantial number of cells transfected. Figure 21E are representative photomicrographs of ARPE-19 cells after application of a bolus of bovine vitreous EVs that had been mixed with GFP without electroporation (0 V, control) showing no fluorescein staining **(left).** Nuclei are labelled **(right,** Hoechst stain). Figure 21F is a graphical representation of the mean ± standard deviation of the transfection efficiency (% cells transfected) of ARPE-19 cells after application of EVs loaded with 1 µg, 0.5 µg, or 0.25 µg GFP by electroporation at 300 V or 1 µg GFP without electroporation (0 V, control). *p < 0.05 for each GFP dosages loaded at 300 V vs. each controls at 0 V. Scale bars (Figures 21A,B,D,E) 50 µm. n = 3 for all experiments

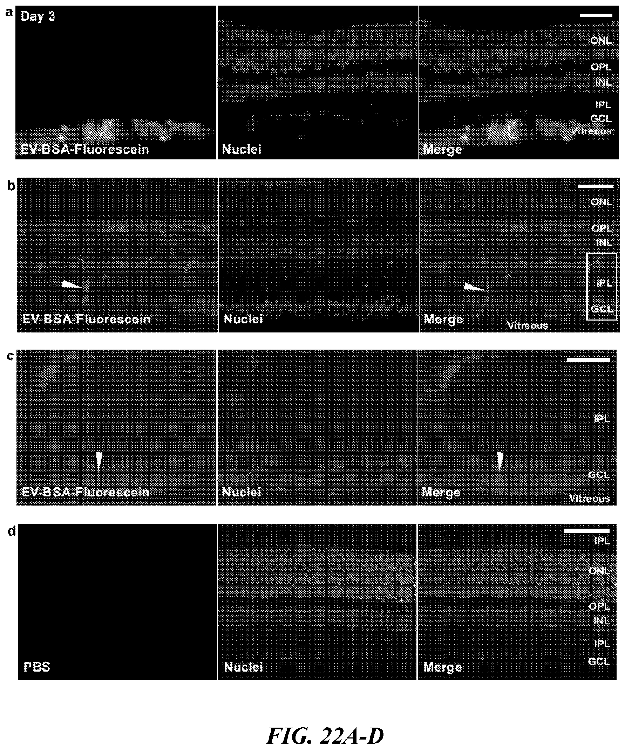

Figures 22A-D are the images from the *in vivo* study of bovine vitreous EVs targeting the retina and delivering recombinant protein to mouse retina. Figure 22A shows representative confocal photomicrographs of mouse retina tissue sections after injection of a dilute amount of bovine EVs (0.25 µg) loaded with recombinant bovine serum albumin (BSA) conjugated to fluorescein on day 3 post injection showing signal in vitreous that does not penetrate the inner limiting membrane. Figure 22B are representative confocal photomicrographs of mouse retina tissues section 3 weeks after injection of BSA-fluorescein-loaded EVs showing signal in cells traversing the ganglion cell layer (GCL), the IPL (inner plexiform layer) and the OPL (outer plexiform layer, arrowhead). The inset box from (Figure 22B) is shown in higher power in Figure 22C, demonstrating positive stain in a cluster of cells in the GCL and retinal nerve fiber layer. Figure 22D are representative confocal photomicrographs of mouse retina tissues 3 weeks after injection of PBS control show no fluorescein signal. (Figures 22A-D) Nuclei were stained with Hoechst **(middle panels)** and images merged in **right panels.** Abbreviations: outer nuclear layer (ONL), and inner nuclear layer (INL). Scale bars are (Figure 22A) 30 µm, (Figure 22B) 50 µm, (Figure 22C) 25 µm and (Figure 22D) 40 µm.

### DETAILED DESCRIPTION OF THE INVENTION

The present invention relates to a method of fixing extracellular vesicles. The fixation method entails providing a sample containing extracellular vesicles, and contacting the sample with a non-reversible cross-linking agent under conditions effective to fix the extracellular vesicles.

The method of the present invention can optionally further include contacting the sample with an aldehyde-containing fixative before, after, or at the same time as the contacting of the sample with a non-reversible cross-linking agent to fix the extracellular vesicles. Furthermore, the method can include imaging the fixed extracellular vesicles.

The term "extracellular vesicle" refers to a nanosized membranous particle secreted by a cell. Extracellular vesicles, which are also referred to as EVs, multivesicular bodies, and ectosomes, are natural transport nano-vesicles that have been implicated in intercellular communication via transfer of biomolecules such as proteins, lipids, and RNA from one cell to another. As used herein, extracellular vesicles can include exomeres, exosomes, multivesicular bodies, intraluminal vesicles (ILVs), multivesicular endosomes (MVEs), oncosomes, micro-vesicles ranging in size from 20-10,000 nm, apoptotic bodies, or vesicles originating from the endosome or plasma membrane.

In one aspect of the present invention, the extracellular vesicles have a size of 20 nanometers to 10,000 nm.

Extracellular vesicles are membrane enclosed vesicles released by all cells. Based on the biogenesis pathway, different types of vesicles can be identified: (1) exosomes are formed by inward budding of late endosomes forming multivesicular bodies (MVB) which then fuse with the limiting membrane of the cell concomitantly releasing the exosomes; (2) shedding vesicles are formed by outward budding of the limiting cell membrane followed by fission; and (3) when a cell is dying via apoptosis, the cell is desintegrating and divides its cellular content in different membrane enclosed vesicles termed apoptotic bodies. These mechanisms allow the cell to discard waste material and were more recently also found to be associated with intercellular communication. Their primary constituents are lipids, proteins, and nucleic acids. They are composed of a protein-lipid bilayer encapsulating an aqueous core comprising nucleic acids and soluble proteins. Identifying the origin of extracellular vesicles is typically done using biomolecular characterization techniques to determine the protein, nucleic acid and lipid content.

The terms "fixing" and "fixed" are used according to their art accepted meaning and refer to the chemical treatment (typically cross-linking) of biological materials such as proteins and nucleic acids that can be accomplished by the wide variety of fixation protocols known in the art (see, e.g., Current Protocols In Molecular Biology, Volume 2, Unit 14, Frederick M. Ausubul et al. eds., 1995).

The aldehyde fixation of tissue is believed to produce cross-linked proteins. This cross-linking is mediated by the reaction of aldehyde groups in the fixative with amino groups on amino acid residues of tissue proteins, such as lysine and the N-terminal a-amino acid group. The initial product of this interaction is an amino-aldehyde conjugate, either an imino Schiff base (CHR₁ NR₂ R₃) or an amino-methylol (CHR₁ OHNR₂ R₃) intermediate. The intermediate may then undergo nucleophilic attack by susceptible neighboring amino acid groups, such as α-carbonyl methylene carbons having an acidic proton, nucleophilic heteroatoms, or electron rich aromatic rings. Prime nucleophiles include aromatic rings such as the ortho-position of the phenol ring of tyrosine, the C-2 position of the indole ring of tryptophan, and the imidazole ring of histidine; the α-carbons adjacent to the side chain carboxylic acid groups of glutamate and aspartate; basic heteroatoms such as lysyl ε-amino groups; and neutral nitrogen atoms such as asparaginyl and glutaminyl amide groups and the indole ring nitrogen of tryptophan. Formally, all such reactions are types of or at least similar to Mannich reactions, at least inasmuch as the reactive electrophile is the intermediate amino-aldehyde conjugate species. These reactions result in a covalent bond between the electrophilic aldehyde carbon and a nucleophilic carbon or heteroatom.

The resulting cross-linking fixes proteins in a particular conformation and fixes the entire tissue by forming covalent bonds among adjacent proteins. The cross-linked proteins resist penetration by macromolecules such as antibodies. In addition, chemical modification of epitopes (which contain amine, amide, or aromatic amino acid residues) produces an altered structure unrecognizable to an antibody against that epitope.

The most common aldehyde fixative is formaldehyde, which is uni-functional and produces cross-linking by direct contact between methylol-amino groups of lysine and adjacent susceptible amino acid target residues. However, other di-functional or poly-functional cross-linking aldehydes are known. Of these, the most common is glutaraldehyde, a five carbon chain with aldehydes at both termini. This di-functional reagent provides additional opportunities for cross-linking, since the alkyl chain of the reagent functions as a spacer. The mechanism of reaction is believed similar, regardless of the particular aldehyde reagent used for fixation.

Cross-links preserve tissue morphology and integrity, harden the tissue for slicing, and inhibiting microbial attack. The chemistry of the cross-linking of amino acids and proteins by formaldehyde is well known in the art and described in Harlan and Feairheller, "Chemistry of the Cross-Linking of Collagen During Tanning," and Kelly, et al. "Cross-Linking of Amino Acids By Formaldehyde," (1976).

The role of Mannich-type reactions in cross-linking of protein amino groups and aromatic amino acids with formaldehyde is discussed in Fraenkel-Conrat, et al., J. Biol. Chem. 168:99-118 (1947), and Fraenkel-Conrat et al., Biol. Chem. 174:827-843 (1948). Further discussions of aldehyde cross-linking reactions are found in Fox, J. Histochem. Cytochem. 33:845-855(1985); Jones, "Reactions of Aldehyde with Unsaturated Fatty Acids During Histological Fixation," in P. J. Stoward, ed. Fixation in Histochemistry, (1973); and Kunkel et al., Mol. Cell. Biochem. 34:3 (1981). Mannich type reactions are described in general in March, "Advanced Organic Chemistry," particularly at 333,424, 670-672 (1968). See U.S. Patent No. 5,578,452.

Samples fixed with the method of the present invention can further be stained to enhance the imaging of the sample, as is common and well known in the art. Exemplary stains and their common uses are described, by way of example: monazo compound Janus Green B used to stain phosphoinositides; disazo compound ponceau S for staining proteins; diazonium salt Fast red TR for detecting esterase activity; diazonium salt Fast blue RR for detecting alkaline phosphatase, esterase, and β-glucouronidase activity; arylamethane compound Fast green FCF for staining and quantitating collagen and other proteins; arylmethane compound Coomasie brilliant blue R250 for staining proteins; arylmethane compound aldehyde fuchsine for staining cystein rich proteins and sulfated glycoproteins; hydroxytriphenylmethane Aurin tricarboxylic acid for the detection of aluminum; xanthene compound eosin Y for the staining of proteins; xanthene compound rhodamine B for the staining of keratin and lipids; xanthene compound pyronine Y for detecting the presence of RNA and DNA and staining of phospholipids; xanthene compound fluorescein isothiocyanate for reaction with nucleophilic groups, for example, amino, hydroxyl and thiol groups, particularly reactive groups on proteins and nucleic acids; acridine dye acriflavin for detecting sulfated glycosamininoglycans; acridine compound acridine orange for staining DNA and RNA and also starch granules; acridine compound phosphine for the staining of lipids and acid mucopolysaccharides; acridine compound quinacrine for the staining of nucleic acids; phenanthridine compound ethidium bromide for the detection of nucleic acids, particularly double stranded nucleic acids; azine compound nigrosine WS for the detection of proteins; azine compound neutral red for the detection of nucleic acids and lipid structures; azine compound safranine-O for the detection of proteoglycans and glycosaminoglycans; oxazine compound nile red for the staining of lipids; oxazine compound gallocyanine chrome alum for the detection of DNA and RNA; oxazine compound nile blue for staining lipids and hydrophobic compounds, including DNA; oxazine compound nile blue for staining lipids and hydrophobic compounds, including DNA; thiazine compound azure B for detecting DNA, RNA, and mucin (i.e., highly glycosylated glycoproteins); thiazine compound toluidine blue for staining of sulfated mucins and amyloid proteins; polyene compound calcofluor white M2R for the staining of chitin and cellulose; polyene compound fluoro-gold for the detection of DNA and mucopolysaccharides; polymethine compound YO-PRO-1 for staining of DNA; polymethine compounds DiO, DiI, DiD for the staining of lipid membranes; benzimidazole compounds DAPI and Hoechst 33342 for the staining of nucleic acids; thiazole compound thiazole orange for staining nucleic acids; thiazole compound thioflavin T for staining amyloid proteins; flavinoid compounds hematoxylin and hematein, and derivatives thereof staining nucleic acids, phospholipids, starch, cellulose, and muscle proteins; carbonyl compound indoxyl ester and its derivatives for detecting esterase and glyosidase activities; anthraquinone compound alizarin red S for detecting calcium, particularly in calcified tissues; phthalocyanine compound luxol fast blue MBS for detecting myelin; phthalocyanine compounds cuprolinic blue to stain RNA and glycosaminoglycans, and alcian blue 8G for glycoseaminoglycans; osmium tetraoxide for the staining of lipids, including fats and cholesterols; iodine for the differential staining of starch, glycogen, and proteins; dithiooxamide and p-dimethylaminobenzylidenerhodamine for the assay of copper, for instance in detecting physiological abnormalities of copper metabolism; tetracycline and its derivatives for detecting the presence of calcium; and diaminobenzidine for detecting oxidases, such as peroxidase and catalase.

As will be appreciated by those skilled in the art, the compounds and stains have applications for revealing structures in cells and tissues in addition to reactions with identified compounds. Binding of reagents to these cellular and tissue structures may occur through various components within the specimen (e.g., heterochromatic staining) rather than through a single cellular constituent. These stains and their uses are well known in the art and are disclosed in U.S. Patent Application Publication No. US2008/0070324.

In one embodiment of the present invention, the sample being treated with the present invention for the fixation of extracellular vesicles, is a biological fluid or tissue.

In one aspect of the present invention, the sample is a biological fluid or tissue. Preferred biological fluid samples are selected from blood products, sols, suspensions, gels, colloids, fluids, liquids, plasmas, plastic solids, suspension, gels, breast milk, nipple aspirate fluid, urine, semen, amniotic fluid, cerebrospinal fluid, vitreous, aqueous humor, synovial fluid, lymph, bile, saliva, bile, cerumen (earwax), chyle, chyme, endolymph, perilymph, exudates, feces, ejaculate, gastric acid, gastric juice, mucus, pericardial fluid, peritoneal fluid, pleural fluid, pus, rheum, sebum (skin oil), serous fluid, smegma, sputum, sweat, tears, vaginal secretion, surgical waste, and vomit. The most preferable biological fluid samples are vitreous or aqueous humor, urine, cerebrospinal fluid, nipple aspirate fluid and blood products. In another preferable embodiment of the invention the biological fluid sample is a blood product selected from the group consisting of whole blood, blood plasma, blood platelets, and blood serum.

In a further aspect of the present invention, the biological tissue sample is a tissue selected from skin, bone, cartilage, tendon, ligament, vertebral disc, cornea, lens, meniscus, hair, striated muscle, smooth muscle, cardiac muscle, adipose tissue, fibrous tissue, neural tissue, connective tissue, cochlea, testis, ovary, stomach, lung, heart, liver, pancreas, kidney, intestine, and eye.

In one embodiment of the present invention, the non-reversible cross-linking agent crosslinking agent used to fix the extracellular vesicles is selected from the group consisting of a water-soluble carbodiimide, cyanogen halide, and mixtures thereof. Preferably the non-reversible cross-linking agent is a cyanogen halide selected from cyanogen bromide, cyanogen fluoride, cyanogen chloride, and cyanogen iodide. Most preferably the non-reversible cross-linking agent is 1-ethyl-3-(3-dimethylaminopropyl)-carbodiimide.

Additionally, the invention can optionally include fixing extracellular vesicles with a further cross-linking agent, independently of, and before, after, or at the same time as contacting the sample with the non-reversible cross-linking agent and aldehyde-containing fixative. Exemplarily cross-linking agents include ethylene glycol di(meth)acrylate, ethylene glycol diacrylate, di(ethylene glycol) diacrylate, tetra(ethylene glycol) diacrylate, ethylene glycol dimethacrylate, di(ethylene glycol) dimethacrylate, tri(ethylene glycol) dimethacrylate, derivatives of methylenebisacrylamide, N,N- methylenebisacrylamide, N,N-methylenebisacrylamide, N,N- (1,2-dihydroxyethylene)bisacrylamide, formaldehyde-free cross-linking agents, N- (1-hydroxy-2,2-dimethoxyethyl)acrylamide, divinylbenzene, formalin fixatives, formal calcium, formal saline, zinc formalin (unbuffered), Zenker's fixative, Helly's fixative, B-5 fixative, Bouin's solution, Hollande's solution, Gendre's solution, Clarke's solution, Carnoy's solution, methacarn, alcoholic formalin, and formol acetic alcohol.

In a further aspect of the present invention, the imaging of the fixed extracellular vesicles may be carried out by transmission electron microscopy, scanning electron microscopy, cryoelectron microscopy, binocular stereoscopic microscopy, wide-field microscopy, polarizing microscopy, phase contrast microscopy, multi-photon microscopy, differential interference contrast microscopy, fluorescence microscopy, laser scanning confocal microscopy, multiphoton excitation microscopy, ray microscopy, ultrasonic microscopy, color metric assay, chemiluminescence, spectrophotometry, positron emission tomography, computerized tomography, or magnetic resonance imaging.

The three commonly used types of electron microscope each generate images via different contrast mechanisms (Ruska, E., "The Development of the Electron Microscope and of Electron Microscopy," Nobel Lectures, Physics 1981-1990 (1986); Reimer et al., Transmission Electron Microscopy: Physics of Image Formation, Springer (2008); Bozzola et al., Electron Microscopy, Jones and Bartlett (1992)).

In TEM, a stationary, spread electron beam with energy between 60 and 300 keV irradiates a sample that is thinner (often much thinner) than 0.5 µm. The sample modifies the phase and amplitude of the transmitted electrons, so that the resulting image contains information about the sample. In scanning TEM (STEM), the image is recorded by scanning a focused beam over the sample and detecting transmitted electrons pixel by pixel. Scanning electronmicroscopy (SEM) scans a focused beam, typically with energy between 500 eV and 30 keV, over the surface of a (bulk) sample, collecting backscattered or secondary electrons pixel by pixel. All electronmicroscopes require a vacuum, both to allow operation of the electronsource and to minimize scattering other than from the sample. Samples must therefore be stable under vacuum, and so are traditionally prepared in the solid state. However, recent advances have been made in the imaging of fluid samples, which is disclosed in U.S Patent Application Publication No. 2012/0120226. Furthermore, the use of imaging via microscopy, photometry, and tomography are well known in the art and are disclosed in U.S. Patent Nos.: 6,831,781 and 5,205,291; as well as U.S. Patent Application Publication Nos.: 2009/0091566 and 2012/0208184; and WO Application Nos. WO2006022342, and WO2012135961.

In one embodiment of the present invention, the detection of the extracellular vesicles fixed with a non-reversible cross-linking agent and optionally, an aldehyde-containing fixative in the biological sample is based on imaging. Furthermore, the biological sample can be a clinical sample. The clinical sample can be from a patient treated with a clinical drug. Additionally, the method of the invention includes diagnosing whether the subject providing the clinical sample has a disease or disorder based on imaging of the fixed extracellular vesicles. The patient can include, but is not limited to, mammals such as humans, animals, cats, dogs, cows, sheep, goats, and horses.

In another aspect of the present invention, the disease or disorder is selected from the group consisting of cancer, inflammatory diseases, infections, degenerative diseases, diseases caused by pathogens, neurological diseases and disorders, and internal dysfunctions. In a preferred embodiment the disease or disorder is an internal dysfunction selected from the group consisting of glaucoma and other ocular diseases.

Glaucoma disorders and ocular diseases that can be detected with the present invention as described herein include, but are not limited to, preglaucoma open angle with borderline findings, open angle, low risk, anatomical narrow angle primary angle closure suspect, steroid responder, ocular hypertension, primary angle closure without glaucoma damage (pas or high iop with no optic nerve or visual field loss), unspecified open-angle glaucoma, primary open-angle glaucoma, chronic simple glaucoma, low-tension glaucoma, pigmentary glaucoma, capsular glaucoma with pseudo-exfoliation of lens, residual stage of open-angle glaucoma, unspecified primary angle-closure glaucoma, acute angle-closure glaucoma attack, chronic angle-closure glaucoma, intermittent angle-closure glaucoma, residual stage of angle-closure glaucoma, glaucoma secondary to eye trauma, glaucoma secondary to eye inflammation, glaucoma secondary to other eye disorders including, retinal vascular occlusions, diabetes type 1 complicated, diabetes type 2 complicated, disorders of lens, disorders of intraocular lens, disorders after other ocular symptoms, neoplasms, benign neoplasms, or malignant. Also included is glaucoma secondary to drugs, glaucoma with increased episcleral venous pressure, hypersecretion glaucoma, aqueous misdirection malignant glaucoma, glaucoma in diseases classified elsewhere, congenital glaucoma, axenfeld's anomaly, buphthalmos, glaucoma of childhood, glaucoma of newborn, hydrophthalmos, keratoglobus, congenital glaucoma macrocornea with glaucoma, macrophthalmos in congenital glaucoma, megalocornea with glaucoma, and absolute glaucoma. Also included are adverse effects of ophthalmological drugs and preparations, acute follicular conjunctivitis, adverse effect of carbonic anhydrase inhibitors, and adverse effect of under dosing of ophthalmological drugs and preparations.

In the determination of diseases and disorders the extracellular vesicles remain undisrupted and whole. By observing the fixed extracellular vesicles by any of the above mentioned methods, the size, morphology, density and any possible coating on the vesicles can be used to determine the if a sample provided by a subject has a diseases or disorder.

In a further embodiment of the present invention, the disease or disorder is an internal dysfunction characterized by an immunodeficiency or hypersensitivity. Preferred immunodeficiency or hypersensitivities include rheumatoid arthritis, osteoarthritis, psoriatic arthritis, psoriasis, dermatitis, polymyositis/dermatomyositis, toxic epidermal necrolysis, systemic scleroderma, Crohn's disease, ulcerative colitis, allergic conditions, eczema, asthma, lupus erythematosus (SLE), multiple sclerosis, allergic encephalomyelitis, sarcoidosis, granulomatosis (including Wegener's granulomatosis), agranulocytosis, vasculitis (including ANCA), aplastic anemia, Diamond Blackfan anemia, immune hemolytic anemia, pernicious anemia, pure red cell aplasia (PRCA), Factor VIII deficiency, hemophilia A, autoimmune neutropenia, pancytopenia, leukopenia, diseases involving leukocyte diapedesis, multiple organ injury syndrome, mysathenia gravis, antigen-antibody complex mediated diseases, anti-glomerular basement membrane disease, anti-phospholipid antibody syndrome, allergic neuritis, Bechet disease, Castleman's Syndrome, Goodpasture's Syndrome, Lambert-Eaton Myasthenic Syndrome, Reynaud's Syndrome, Sjorgen's Syndrome, Stevens-Johnson Syndrome, solid organ transplant rejection, graft versus host disease (GVHD), pemphigoid bullous, pemphigus, autoimmune polyendocrinopathies, Reiter's disease, or Guillain-Barre' Syndrome.

In another embodiment of the present invention, the disease or disorder diagnosed based on the imaging of the extracellular vesicles fixed with a non-reversible cross-linking agent and an aldehyde-containing fixative is a cancer selected from the group consisting of acute granulocytic leukemia, acute lymphocytic leukemia (ALL), acute myelogenous leukemia (AML), adenocarcinoma, adenosarcoma, adrenal cancer, adrenocortical carcinoma, anal cancer, anaplastic astrocytoma, angiosarcoma, appendix cancer, astrocytoma, basal cell carcinoma, B-Cell lymphoma, bile duct cancer, bladder cancer, bone cancer, bone marrow cancer, bowel cancer, brain cancer, brain stem glioma, brain tumor, breast cancer, carcinoid tumors, cervical cancer, cholangiocarcinoma, chondrosarcoma, chronic lymphocytic leukemia (CLL), chronic myelogenous leukemia, colon cancer, colorectal cancer, craniopharyngioma, cutaneous lymphoma, cutaneous melanoma, diffuse astrocytoma, ductal carcinoma in situ (DCIS), endometrial cancer, ependymoma, epithelioid sarcoma, esophageal cancer, Ewing sarcoma, extrahepatic bile duct cancer, eye cancer, fallopian tube cancer, fibrosarcoma, gallbladder cancer, gastric cancer, gastrointestinal cancer, gastrointestinal carcinoid cancer, gastrointestinal stromal tumors (GIST), germ cell tumor, gestational trophoblastic disease, glioblastoma multiforme (GBM), glioma, hairy cell leukemia, head and neck cancer, hemangioendothelioma, Hodgkin lymphoma, Hodgkin's disease, hypopharyngeal cancer, infiltrating ductal carcinoma (IDC), infiltrating lobular carcinoma (ILC), inflammatory breast cancer (IBC), intestinal cancer, intrahepatic bile duct cancer, invasive/infiltrating breast cancer, islet cell cancer, jaw cancer, Kaposi sarcoma, kidney cancer, laryngeal cancer, leiomyosarcoma, leptomeningeal metastases, leukemia, lip cancer, liposarcoma, liver cancer, lobular carcinoma in situ, low-grade astrocytoma, lung cancer, lymph node cancer, lymphoma, male breast cancer, medullary carcinoma, medulloblastoma, melanoma, meningioma, Merkel cell carcinoma, mesenchymal chondrosarcoma, mesenchymous, mesothelioma, metastatic breast cancer, metastatic melanoma, metastatic squamous neck cancer, mixed gliomas, mouth cancer, mucinous carcinoma, mucosal melanoma, multiple myeloma, mycosis fungoides, myelodysplastic syndrome, nasal cavity cancer, nasopharyngeal cancer, neck cancer, neuroblastoma, neuroendocrine tumors (NETs), Non-Hodgkin lymphoma (NHL), non-small cell lung cancer, oat cell cancer, ocular cancer, ocular melanoma, oligodendroglioma, oral cancer, oral cavity cancer, oropharyngeal cancer, osteogenic sarcoma, osteosarcoma, ovarian cancer, ovarian epithelial cancer, ovarian germ cell tumor, ovarian primary peritoneal carcinoma, ovarian sex cord stromal tumor, Paget's disease, pancreatic cancer, papillary carcinoma, paranasal sinus cancer, parathyroid cancer, pelvic cancer, penile cancer, peripheral nerve cancer, peritoneal cancer, pharyngeal cancer, pheochromocytoma, pilocytic astrocytoma, pineal region tumor, pineoblastoma, pituitary tumors, primary central nervous system (CNS) lymphoma, prostate cancer, rectal cancer, renal cell carcinoma, renal pelvis cancer, rhabdomyosarcoma, salivary gland cancer, sarcoma, Sarcoma (bone), Sarcoma (soft tissue), Sarcoma (uterine), sinus cancer, skin cancer, small cell lung cancer (SCLC), small intestine cancer, soft tissue sarcoma, spinal cancer, spinal column cancer, spinal cord cancer, spinal tumor, squamous cell carcinoma, stomach cancer, synovial sarcoma, T-cell lymphoma, testicular cancer, throat cancer, thymoma/thymic carcinoma, thyroid cancer, tongue cancer, tonsil cancer, transitional cell cancer (bladder), Transitional cell cancer (kidney), Transitional cell cancer (ovarian), triple-negative breast cancer, tubal cancer, tubular carcinoma, undiagnosed cancer, ureteral cancer, uterine adenocarcinoma, uterine cancer, uterine sarcoma, vaginal cancer, and vulvar cancer. In a more preferable embodiment, the cancer is ocular cancer.

In a further embodiment, the disease or disorder diagnosed based on the imaging of the extracellular vesicles fixed with a non-reversible cross-linking agent and optionally, an aldehyde-containing fixative is a neurological disease selected from the group consisting of Demyleinating Diseases, Multiple Sclerosis, Parkinson's disease, Huntington's disease, Creutzfeld-Jakob disease, Alzheimer's disease, Wilson's Disease, Spinal muscular atrophy, Lewy body disease, Friedreich's Ataxia, Autism, Autism spectrum disorders, synaptic density associated with disease, and Amyotrophic lateral sclerosis (ALS).

In yet another embodiment, the disease or disorder being diagnosed includes neurological disorders such as substance abuse-related disorders, alcohol use disorders, amphetamine-use disorders, cannabis-use disorders, caffeine-induced disorders, cocaine-use disorders, inhalant-use disorders, opioid-use disorders, hallucinogen disorders, sedative-use, hypnotic-use, or anxiolytic-use disorders, polysubstance-use disorders, sexual dysfunctions, sexual arousal disorder, male erectile disorder, male hypoactive disorder, female hypoactive disorder, eating disorders, overeating disorder, bulimia nervosa, anorexia nervosa, anxiety, obsessive compulsive disorder syndromes, panic attacks, post-traumatic stress disorder, agoraphobia, obsessive and compulsive behavior, impulse control disorders, pathological gambling, intermittent explosive disorder, kleptomania, pyromania, personality disorders, schizoid personality disorder, paranoid personality disorder, schizotypal personality disorder, borderline personality disorder, narcissistic personality disorder, histrionic personality disorder, obsessive compulsive personality disorder, avoidant personality disorder, dependent personality disorder, and anti-social personality disorder, schizophrenia subtypes, schizoaffective disorder, schizophrenia undifferentiated, delusional disorder, cyclothymic disorder, somatoform disorder, hypochondriasis, dissociative disorder, and depersonalization disorder.

In another embodiment of the present invention, the disease or disorder diagnosed based on the imaging of the extracellular vesicles fixed with a non-reversible cross-linking agent and, optionally, an aldehyde-containing fixative is a cardiovascular disease.

Another aspect of the invention includes diagnosing of a disease or disorder by performing two or more assays for disease markers.

Exemplary infections that may be diagnosed include Influenza A Matrix protein, Influenza H3N2, Influenza H1N1 (seasonal), Influenza H1N1 (novel), Influenza B, Streptococcus pyogenes (A), Mycobacterium Tuberculosis, Staphylococcus aureus (MR), Staphylococcus aureus (RS), Bordetella pertussis (whooping cough), Streptococcus agalactiae (B), Influenza H5N1, Influenza H7N9, Adenovirus B, Adenovirus C, Adenovirus E, Hepatitis b, Hepatitis c, Hepatitis delta, Treponema pallidum, HSV-1, HSV-2, HIV-1, HIV-2, Dengue 1, Dengue 2, Dengue 3, Dengue 4, Malaria, West Nile Virus, Ebola virus, Marburg virus, Cueva virus, Trypanosoma cruzi (Chagas), Klebsiella pneumoniae (Enterobacteriaceae spp), Klebsiella pneumoniae carbapenemase (KPC), Epstein Barr Virus (mono), Rhinovirus, Parainfluenza virus (1), Parainfluenza virus (2), Parainfluenza virus (3), Parainfluenza virus (4a), Parainfluenza virus (4b), Respiratory syncytial virus (RSV) A, Respiratory syncytial virus (RSV) B, Coronavirus 229E, Coronavirus HKU1, Coronavirus OC43, Coronavirus NL63, Novel Coronavirus, Bocavirus, human metapneumovirus (HMPV), Streptococcus pneumoniae (penic R), Streptococcus pneumoniae (S), Mycoplasma pneumoniae, Chlamydia pneumoniae, Bordetella parpertussis, Haemophilus influenzae (ampic R), Haemophilus influenzae (ampic S), Moraxella catarrhalis, Pseudomonas spp (aeruginosa), Haemophilus parainfluenzae, Enterobacter cloacae (Enterobacteriaceae spp), Enterobacter aerogenes (Enterobacteriaceae spp), Serratia marcescens (Enterobacteriaceae spp), Acinetobacter baumanii, Legionella spp, Escherichia coli, Candida, Chlamydia trachomatis, Human Papilloma Virus, Neisseria gonorrhoeae, plasmodium, and Trichomonas (vagin).

In one embodiment, the diagnosing includes providing a standard image of a clinical sample containing extracellular vesicates fixed with a non-reversible cross-linking agent. This image is from a subject having a particular disease or disorder. The image is then used in comparison to the image of the clinical sample of the subject. The imaged fixed extracellular vesicles are compared in regard to size, density, morphology, and/or spacial distribution. This comparison is then used to determine if the subject has the particular disease or disorder. This method can optionally further include contacting all of the samples with an aldehyde-containing fixative before, after, or at the same time as contacting the samples with a non-reversible cross-linking agent to fix the extracellular vesicles. Additionally, the method may further include administering a therapeutic agent to the subject based on the step of determining if the subject has the particular disease or disorder.

EVs, such as exomeres, exosomes, ectosomes (referred here as micro-vesicles, MVs), and apoptotic bodies, exist in various sizes, and their characteristics such as size, morphology, concentration, and spatial localization can be utilized for EVs characterization. Variations in EV morphology may represent either normal or pathological conditions, and methods that allow for reliable characterization of EVs properties, may help determine the origin of the EV. The first step is to determine if imaging EVs can be used as a reliable biomarker. It is important to differentiate EVs derived from healthy patients and EVs present in disease. Here, it is shown that the plasma of multiple patients with glioma contains numerous EVs that are grouped in clusters with surrounding electron dense materials as shown in Figure 3A-C. This EV morphology and spatial localization in relation to each other is substantially different than the morphology and size of EVs isolated from healthy control patient plasma in Figure 4A-B. In the healthy control patients, EVs were observed with less frequency and without clustering. These data suggest that EVs derived from disease differ from healthy control, suggesting that this method can be used as a diagnostic to identify disease from normal.

Another important quality of a potential liquid biopsy test is to differentiate one disease from another. It is hypothesized that the morphology, size and spatial localization of EVs could facilitate disease diagnosis. To test this, various malignancies were examined, the EVs isolated from the plasma were visualized, and their morphology compared. In patients with systemic melanoma, an electron dense signal resembling EVs was observed (Figure 5A-B); albeit with differing morphology when compared to EVs from patients with glioma (Figure 3A-C) or healthy controls (Figure 4A-B). Moreover, other cancers and other fluids like cerebrospinal fluid were tested, to determine if EV morphology differed in various diseases. Therefore, samples were collected from the cerebrospinal fluid from patients with the diagnosis of neuroblastoma and sarcoma; the EVs were imaged using EDC-glutaraldehyde fixation and it was possible to identify EVs in both disorders (Figure 6A-D). The data show that EVs imaged from patients with neuroblastoma contain large EVs that are clustered. However, when EVs from cerebrospinal fluid from sarcoma patients were visualized, the EVs were smaller than those observed in neuroblastoma CSF. These data imply that EV morphology differs in each liquid biopsy tested for various cancers. It is expected that the EDC-glutaraldehyde fixation method will be broadly applicable for imaging EVs associated with other biological fluid specimens (plasma, cerebrospinal fluid, and ductal fluid) from patients with a variety of other highly prevalent cancers. Moreover, this basic technology will allow for the study of the structure of EVs in ocular fluids, plasma, CSF, and ductal fluid. The morphology of EVs in various diseases and in healthy controls can then be compared. This information may be useful for cancer diagnosis, exclusion, prognosis, or as an indicator of metastatic potential.

The diagnosing can also involve monitoring the progression or regression of a disease or disorder in a subject. This is accomplished by providing a prior image of a clinical sample of a subject containing extracellular vesicles fixed with a non-reversible cross-linking agent, and comparing it to an image of a clinical sample of a subject containing extracellular vesicles fixed with a non-reversible cross-linking agent. The extracellular vesicles are compared in regard to size, density, morphology, and/or spacial distribution and it is determined if the disease or disorder is progressing or regressing based on the comparison. This method can optionally further include contacting the samples with an aldehyde-containing fixative before, after, or at the same time as contacting the samples with a non-reversible cross-linking agent to fix the extracellular vesicles. Additionally, this method may further include administering a therapeutic agent to the subject based on the step of determining if the disease or disorder is progressing or regressing.

A kit used in the method of the present invention includes a support substrate for holding the sample, an aldehyde-containing fixative and a non-reversible cross-linking agent. The non-reversible cross-linking agent used in the method of the present invention is selected from a water-soluble carbodiimide, cyanogen halide, and mixtures thereof. Most preferably the non-reversible cross-linking agent is 1-ethyl-3-(3-dimethylaminopropyl) carbodiimide.

The support substrate of the kit can include a solid support, such as a slide, chip, column matrix, dipstick, membrane, particle (e.g., bead or nanoparticle) or well of a microtitre plate.

### EXAMPLES

The examples below are intended to exemplify the practice of embodiments of the disclosure but are by no means intended to limit the scope thereof.

### Example 1 - Fluid Sample Preparation and Processing

Aqueous humor or vitreous humor specimens collected for EV isolation were processed immediately without fixation. EVs were isolated from human or bovine vitreous humor, aqueous humor, plasma nipple aspirate fluid or cerebrospinal fluid (CSF) using ultracentrifugation protocols described below. Patients with a diagnosis of various melanoma or glioma donated plasma and EVs were isolated using methods described. Patients with a diagnosis of neuroblastoma or sarcoma donated cerebral spinal fluids and the EVs were isolated. Patients with a diagnosis of breast cancer donated nipple aspirate fluid and the EVs were isolated.

### Example 2 - Extracellular Vesicle Isolation and Purification of Fluid Samples

Methods for isolating extracellular vesicles from fluids were adapted (Wald et al., "The Light Reaction in the Bleaching of Rhodopsin," Science 111:179-181 (1950).

For this study, the goal was to have vitreous humor, aqueous humor, nipple aspirate fluid, plasma and cerebral spinal fluid (CSF) specimens free of cells. The vitreous was cleared with a series of low-speed centrifugations. For bovine vitreous or aqueous humor, approximately 8 ml of vitreous (or 100 µl of aqueous humor) was placed in 15 ml tubes and centrifuged in Sorvall legend RT Swinging bucket (Sorvall) at 2,000 g (2500 rpm) at 4°C for 30 minutes. The supernatant was then transferred to a new 15 ml tube. Then the centrifugation step was repeated. The supernatant was then transferred to new tube and centrifuged at 10,000 g in a Sorvall RC-58 centrifuge (Sorvall) using an SS-34 rotor (DuPont) for 30 min at 4°C. The supernatant was then transferred and the step was repeated. The sample was transferred to an ultracentrifuge tube (Beckman) and in a swinging bucket rotor (SW-41, Beckman) and centrifuged at 100,000 g in an L7-55 ultracentrifuge (Beckman) at 4°C for 1 hour. The supernatant was transferred to a new tube. The step was repeated. Samples were resuspended in 50 µl of sterile tris buffered saline (TBS, pH 8) and placed in a siliconized tube. Samples for imaging were immediately processed, and the remaining sample was frozen at -80°C.

### Example 3 - Nanoparticle Tracking Analysis for Liquid Samples

The NanoSight NS300 system (Malvern) was used to perform nanoparticle tracking analysis to characterize particles from 30 - 800 nm in solution. Extracellular vesicles isolated from vitreous humor, aqueous humor, plasma or CSF were re-suspended in 100 µl of tris buffered saline (TBS, pH 7.0) at a concentration of approximately 2.5 µg of protein per ml, and then the sample was diluted to a final volume of 2 ml in TBS for analysis. Particles were loaded, the camera was focused, and 5 videos were captured for 60 sec each. Videos were recorded and then analyzed using NanoSight software (Version 3.0) to determine the size distribution and particle concentration of EVs. Graphs were created. The Brownian motion of each particle is tracked between frames, ultimately allowing calculation of the size through application of the Stokes-Einstein equation.

### Example 4 - Conventional Glutaraldehyde only Fixation of Liquid Samples for Electron Microscopy

EV solutions that were processed with conventional TEM fixation methods are referred to as "glutaraldehyde only" or "Glut only". EVs were obtained and resuspended in buffered solution as described above. Formvar/carbon-coated EM grids (Electron Microscopy Sciences) were coated on the surface with Poly-L-lysine solution (%, Sigma Aldrich). Approximately 15 µl of poly-L-lysine was applied to the formvar/carbon-coated surface of the EM grid and incubated the sample in a humidified chamber for 15 min at room temperature. The poly-L-lysine solution was removed with a pipette, and the grid allowed to dry for 10 minutes at room temperature.

Next, 5 µL of EV-containing solution was pipetted onto a poly-L-lysine-formvar/carbon-coated EM grid and incubated in a humidified chamber for 30 minutes at room temperature. Next, the EV solution was removed with a pipette. The samples were fixed in a "glutaraldehyde fixation solution"; consisting of 2.5% glutaraldehyde, 4% paraformaldehyde, 0.02% picric acid in 0.1M sodium cacodylate buffer. 15µl of glutaraldehyde solution was pipetted on the EM grid and incubated the sample for 15 min at room temperature (Faivre et al., "In Frame Fibrillin-1 Gene Deletion in Autosomal Dominant Weill-Marchesani Syndrome," J. Med. Genet. 40:34-36 (2003)).

After, the glutaraldehyde fixation solution was removed with a pipette. Grids were washed with 15 µl of double distilled water for 5 minutes at room temperature. Samples were washed 2 times for 5 min each at room temperature. The samples were dried at room temperature and viewed on a JEM 1400 electron microscope (JEOL, USA, Inc) as described below. EDC-formalin fixed specimens were processed further as described below.

### Example 5 - EDC-ETT Solution Preparation

Methods for EDC solution fixation were adapted from previous reports (Reardon et al., "Identification in Vitreous and Molecular Cloning of Opticin, a Novel Member of the Family of Leucine-Rich Repeat Proteins of the Extracellular Matrix," J. Biol. Chem. 275:2123-2129 (2000); Wheatley et al., "Immunohistochemical Localization of Fibrillin in Human Ocular Tissues. Relevance to the Marfan Syndrome," Arch. Ophthalmol. 113 :103-109 (1996).

A 0.1 M 1-Methylimidazole buffer solution (0.1 M 1-methylimidazole, 300 mM NaCl, with an adjusted pH to 8.0 with 12 N NaOH) was prepared and the solution stored for up to 3 months at room temperature. The EDC solution was freshly prepared for each experiment. 0.96 ml of 0.1 M 1-Methylimidazole buffer solution was measured and 13 mg of 5-(Ethylthio)-1H-tetrazole added (ETT, Sigma Aldrich, final concentration was 0.1 M). The pH was adjusted to 8.0 with 12 N NaOH. Next, 19.2 mg of 1-ethyl-3-(3-dimethylaminopropyl) carbodiimide (EDC) was added (Sigma Aldrich, final concentration 0.10 M) and then the pH readjusted to 8.0 using 12 M HCl. The EDC-ETT solution was placed on ice until use.

### Example 6 - EDC-glutaraldehyde Fixation of Liquid Samples on Electron Microscopy

All isolated EVs were resuspended in 20 µl of TBS (pH 8.0) and kept at 4°C. Formvar/carbon-coated EM grids (Electron Microscopy Sciences) were coated on the surface with Poly-L-lysine solution (%, Sigma Aldrich). Approximately 15 µl of poly-L-lysine was applied to the formvar/carbon-coated surface of the EM grid and incubated the sample in a humidified chamber for 15 min at room temperature. The poly-L-lysine solution was removed with a pipette and the grid set aside in a humidified chamber until it is ready for use. Next, equal parts of freshly made EDC/ETT solution and the EVs solution were combined by adding 5 µl of ice cold EDC/ETT solution with 5 µl of ice-cold EVs suspended in TBS (pH 8.0) into a 1.5 ml pre-chilled siliconized tube. The sample was incubated for 30 min on ice. 10 µl of the EDC/ETT-EV solution was applied to the surface of the formvar/carbon-coated EM grids and incubated the sample for 30 min at 4°C in a humidified chamber. In order to activate the EDC regent's crosslinking capability, the samples were placed in a humidified chamber in an incubator for 3 h at 50°C. The samples were removed from the incubator and the EDC-solution was removed using a pipette. The samples were fixed with a secondary fixation using a glutaraldehyde-based crosslinking solution containing; 2.5% glutaraldehyde, 4% paraformaldehyde, 0.02% picric acid in 0.1M sodium cacodylate buffer and incubated for 15 min at room temperature. The glutaraldehyde solution was removed by pipetting the bubble from the EM grid. The grid was washed by placing 15 µl of double distilled water onto the grid and incubating for 5 minutes at room temperature. The water was removed and washed a second time. Finally, the samples were negatively stained or stained for DNA, RNA and protein as described below. For negative staining, the samples were contrasted successively in 2% uranyl acetate, pH 7, and 2% methylcellulose/0.4% uranyl acetate, pH 4. For positive staining, the samples were stained with acridine orange or CFSE as described below. After staining with the respective stain(s), the EM grids were then mounted for imaging on the electron microscope as described below.

### Example 7 - Transmission Electron Microscope (TEM) Imaging of Fluid Samples

All EM grids were viewed on a TEM 1400 electron microscope (JEOL, USA, Inc) operated at 100kV. Digital images were captured on a Veleta 2K × 2K CCD camera (Olympus-SIS). Electron microscopy images were recorded and analyzed for size and frequency of EVs using ImageJ software.

### Example 8 - Staining Nucleic Acids of Fluid Samples for TEM

For TEM staining of nucleic acids, Acridine Orange stain solution (Exo-Red Exosome RNA Fluorescent Label, System Biosciences) was incubated with 5 µl of ultracentrifuge purified EVs for 30 min at 25°C. For ethidium bromide (EtBr) stained EVs, we mixed 5 µg/ml of EtBr solution with 5 µl of ultracentrifuge purified EVs for 30 min at 25°C. For protein staining on TEM, 500 µM CFSE diluted in TBS (pH 7.4) was mixed with 5 µl of ultracentrifuge purified EVs for 30 min at 25°C. All samples above were then fixed, mounted, and imaged with TEM as above.

### Example 9 - Transmission Electron Microscopy of Vitreous Humor and Ocular Tissues

Human or bovine vitreous tissue was obtained as described above. Samples were cleared of cells with low speed centrifugation and whole mount specimens tested with H and E staining and imaging as described below. For vitreous, 2 µL was pipetted onto a block and fixed in a solution of 2.5% glutaraldehyde, 4% paraformaldehyde, 0.02% picric acid in 0.1M sodium cacodylate buffer and incubated for 60 min at room temperature (Faivre et al., "In Frame Fibrillin-1 Gene Deletion in Autosomal Dominant Weill-Marchesani Syndrome," J. Med. Genet. 40:34-36 (2003)).

Specimens were washed with excess volume of buffer (pH 7.3) for 5 minutes each at room temperature. Samples were post-fixed with 1% OsO₄-1.5% K-ferricyanide (aqueous) for 60 min at room temperature (Hubmacher et al., "Human Eye Development is Characterized by Coordinated Expression of Fibrillin Isoforms," Invest. Ophthalmol. Vis. Sci. 55:7934-7944 (2014). Samples were washed with buffer 3 times for 5 min each at room temperature. Samples were set *en bloc* and stained with 1.5% uranyl acetate for 60 min at room temperature. Samples were dehydrated through graded ethanol series and transitioned through acetonitrile. Samples were infiltrated and embedded in Embed 812 resin (Electron Microscopy Sciences). Tissue sections were cut at 60-65 nm using a Diatome diamond knife (Diatome) on Leica Ultracut T ultramicrotome (Leica Microsystems). Sections were contrasted with lead citrate (Sakuma et al., "Isolation and Characterization of the Human X-Arrestin Gene," Gene 224:87-95 (1998) and viewed on a JEM 1400 electron microscope (JEOL, USA, Inc) operated at 100kV. Digital images were captured on a Veleta 2K × 2K CCD camera (Olympus-SIS). Electron microscopy images were recorded and analyzed for size and frequency of EVs using ImageJ software. For TEM staining of nucleic acids, Acridine Orange stain solution (Exo-Red Exosome RNA Fluorescent Label, System Biosciences) was incubated with 5 µl of ultracentrifuge purified EVs for 30 min at 25°C. For ethidium bromide (EtBr) stained EVs, 5 µg/ml of EtBr solution was mixed with 5 µl of ultracentrifuge purified EVs for 30 min at 25°C. For protein staining on TEM, 500 µM CFSE diluted in TBS (pH 7.4) was mixed with 5 µl of ultracentrifuge purified EVs for 30 min at 25°C. All samples above were then fixed, mounted, and imaged with TEM as above.

For TEM visualization of vitreous vesicles, vesicles were isolated from human or bovine vitreous through ultracentrifugation as described above, re-suspended in formaldehyde, loaded on Formvar/carbon-coated EM grids, postfixed in 1% glutaraldehyde, and contrasted successively in 2% uranyl acetate, pH 7, and 2% methylcellulose/0.4% uranyl acetate, pH 4, or acridine orange or CFSE.

### Example 10 - Tissue Preparation and Processing From Post Mortem Ocular Samples

Post-mortem human eyes without disease were obtained (The Eye-Bank for Sight Restoration, New York, NY). The Weill Cornell Medicine Institutional Review Board granted exemption from IRB approval for use of post-mortem eye bank eyes for this research study. Post-mortem bovine eyes were acquired from a local butcher shop (Green Village Packing, Green Village, New Jersey). For dissection procedures, eyes were placed in a 100 mm plastic petri dish on ice to prevent RNA and protein degradation. Using a SZX-16 stereo dissecting microscope (Olympus), the orbital fat and extraocular muscles attached to the globe were removed. The globe was rinsed with 5 ml of ice-cold Tris Buffered Saline (TBS) containing 50 mM Tris-HCl, 150 mM NaCl (pH 8.0) for 1 minute at 4°C. Vitreous was dissected by making an sclerotomy incision 4 mm or 8 mm posterior to the limbus (human and bovine eye, respectively) using a 16g needle and then making a circumferential sagittal incision with scissors to separate the globe into an anterior and posterior cup. Scissors were used to cut and remove the formed vitreous and to sever adhesions between vitreous and ocular structures. Care was taken to avoid vitreous contamination by uveal tissue or neural retina. Tissue samples were rinsed with TBS (pH 8.0) for 1 min at 4°C. Vitreous specimens collected for electron microscopy and EV isolation were processed immediately without fixation as described below. Samples used for immunohistochemistry, western blot, or EDC-formalin fixation were placed in 15 ml centrifuge tubes and immersed in 10 ml of 4% formalin (also known as formaldehyde, paraformaldehyde (PFA)) diluted in TBS (pH 8.0) for at least 24 h at 4°C. Tissues that were "formalin only," were washed three times in TBS (pH 8.0) for 5 min at 4°C and not further processed or fixed with EDC. Formalin only tissues were used for immunohistochemistry, western blot or nucleic acid, and protein imaging. EDC-formalin fixed specimens were processed further as described below.

### Example 11 - 4T1 Mouse Mammary Carcinoma Tumor Model and Tissue Processing

The 4T1 mouse breast cancer cell line was obtained (ATCC) and maintained according to the supplier's instructions. Exponentially growing 4T1 cells were collected and centrifuged for 5 min at 900 rpm at room temperature. The pellet was resuspended in PBS. A 50 ul suspension containing 5× 10⁴ 4T1 cells was injected orthotopically into the mammary fat pad of BALB/c female mice age 8 weeks. At 2 weeks animals were sedated and euthanized in accordance with NIH Animal Welfare guidelines. The tumor and surrounding tissue was dissected, rinsed with TBS (pH 8.0) for 1 min at 4°C, and fixed in 10 ml of 4% formalin diluted in TBS (pH 8.0) for at least 24 h at 4°C. Tissues were sectioned (1 mm thickness). EDC-formalin fixed specimens were processed further as described below and subsequently stained and imaged using MPM as described below.

### Example 12 - EDC-formalin Tissue Fixation

Methods for EDC-formalin fixation were adapted from previous reports (Pena et al., "miRNA in situ Hybridization in Formaldehyde and EDC-Fixed Tissues," Nat Methods 6:139-141 (2009); Renwick et al., "Multiplexed miRNA Fluorescence in situ Hybridization for Formalin-Fixed Paraffin-Embedded Tissues," Methods Mol Biol 1211:171-187 (2014)).

Vitreous tissue was isolated as described as above and examined under the microscope to ensure the sample was free of contaminating tissues like retina or choroid. Breast cancer tumors from mouse were isolated as described above. The tissue was placed into a 100 mm plastic petri dish and washed two times in 5 ml of TBS (pH 8.0) for 5 min at 4°C, and then immersed in 5 ml of 4% formalin diluted in TBS (pH 8.0) for 24 h and stored in a humidified chamber at 4°C. The samples were washed three times in ice-cold TBS (pH 8.0) for 5 min at 4°C. To remove residual phosphate from the tissue, the sample was incubated in 10 ml of a freshly prepared 0.1 M 1-Methylimidazole buffer solution (0.1 M 1-methylimidazole, 300 mM NaCl, with an adjusted pH to 8.0 with 12 N NaOH) for 30 min at 4°C. Next, the EDC fixation solution was prepared. First, 9.6 ml of 0.1 M 1-Methylimidazole buffer solution was made and 130 mg of 5-(Ethylthio)-1H-tetrazole (ETT, Sigma Aldrich, final concentration was 0.1 M) was added. The pH was adjusted to 8.0 with 12 N NaOH. Next, 192 mg of 1-ethyl-3-(3-dimethylaminopropyl) carbodiimide (EDC) (Sigma Aldrich, final concentration 0.10 M) was added and then the pH readjusted to 8.0 using 12 M HCl. The tissue (1 cm × 1 cm) was transferred to a 35 mm plastic petri dish and 2 ml of EDC fixation solution was added. The samples were placed in a humidified chamber and the specimens were incubated for 3 h at 37°C. After incubation, the EDC-ETT solution was removed and specimens were washed in 5 ml of 0.2% (w/v) glycine diluted in TBS (pH 7.4). The samples were washed twice in TBS (pH 7.4). Finally, the samples were stained for DNA, RNA and protein as described below.

### Example 13 - Staining for DNA, RNA and Protein

The tissues fixed with 4% formalin only, or EDC-formalin, as described above, were stained. Tissues were then immersed with various dyes to label DNA, RNA or proteins. To mark DNA, the tissue (1 cm × 1 cm) was placed in a 35 mm petri dish and immersed with 1 ml of 0.5 µg/ml of Hoechst 33342 Stain Solution (Sigma Aldrich). Samples were incubated at for 15 min at room temperature and then tissues were washed with 5 ml of 1 × TBS (pH 7.4) for 3 min at room temperature. Wash steps were repeated twice. Samples were stained with secondary marker or mounted for imaging. To label both DNA and RNA with a single dye, propidium iodide (PI, Sigma Aldrich) was used, which intercalates between DNA bases and also binds to RNA with less affinity (Suzuki et al., "DNA Staining for Fluorescence and Laser Confocal Microscopy," J Histochem Cytochem 45:49-53 (1997)).

It was found that a solution of 50 µg/ml of PI diluted in TBS (pH 7.4) was the optimal concentration of PI for co-staining DNA and RNA in whole mounted vitreous samples. Therefore, tissues were placed in a 35 mm petri dish and then immersed in 1 ml solution 50 µg/ml of PI (diluted in TBS) for 24 h at 37°C in a humidified chamber. Samples were washed with TBS (pH 7.4) three times. Samples were stained with another marker or mounted for imaging. To differentiate between DNA and RNA, all PI-stained tissues were co-stained with Hoechst 33342 Stain Solution as described above. Hoechst has a strong affinity for DNA and does not label RNA. For Hoechst and PI stained samples, the RNA signal was determined by excluding the Hoechst signal. To label cellular and extracellular proteins in whole mount vitreous, a cell permeable and electron dense (Griffith et al., "Epithelial-Mesenchymal Transformation During Palatal Fusion: Carboxyfluorescein Traces Cells at Light and Electron Microscopic Levels," Development 116:1087-1099 (1992)) stain carboxyfluorescein succinimidyl ester (CFSE, Sigma Aldrich) (Bronner-Fraser M., "Alterations in Neural Crest Migration by a Monoclonal Antibody That Affects Cell Adhesion," J Cell Biol 101:610-617 (1985)), was used, which covalently links to intracellular amines. The tissue was placed in a 35 mm plastic petri dish and then immersed in 1 ml of 500 µM CFSE diluted in TBS (pH 7.4) and incubated the sample for 24 h at 37°C in a humidified chamber. After incubation, the CFSE solution was removed and the tissues were placed in a 100 mm plastic petri dish. The tissues were washed in 5 ml of 0.2% (w/v) glycine diluted in TBS (pH 7.4) for 30 min at room temperature. Next, tissues were washed in 10 ml of TBS (pH 7.4) for 5 min at room temperature, and wash steps were repeated twice. Finally, samples were counterstained with Hoescht and or PI as described above. After staining with the respective dye(s), the samples were then mounted in custom chambers for imaging on the multiphoton, confocal, or wide-field fluorescent microscope as described below.

### Example 14 - RNase Digestion of Extracellular RNA in situ

Vitreous tissues were fixed with EDC-formalin and immersed with 2 ml of RNase buffer (50 mM Tris-Cl, pH 8.0, 10 mM EDTA) containing 100 µg / mL RNase A (Sigma Aldrich), and then incubated for 16 h at 42°C. Next, the RNase solution was removed, and samples were washed, stained with PI as described above, and imaged with wide-field fluorescent microscopy.

### Example 15 - Light Microscopy, Confocal Microscopy, and Image Processing

Color bright field images were captured on a Nikon eclipse upright e600 microscope (Nikon) equipped with an axiocam 105 color camera (Zeiss), and images were processed with Zen software (Zeiss, version 4.3). Tissues were mounted on a 60 mm glass bottom dish (20 mm viewing area, MatTek) for fluorescent imaging studies. An Axio Observer Z1 inverted microscope (Zeiss) was used with the following filter sets: Ziess filter set 49 (Ziess) for Hoechst; Ziess filter set 38 (Ziess) for Alexa 488, green fluorescent protein (GFP), and fluorescein; and Ziess filter set 45 (Ziess) for PI. Confocal imaging was conducted on a Zeiss LSM 880 with a 25x/0.8 NA oil immersion objective, (Weill-Cornell Medicine Imaging Core Facility).

### Example 16 - Multiphoton Imaging of Tissue Sample

Whole mount tissue fixed with EDC-formalin or formalin alone and labelled with DNA, RNA and/or protein stained described above was mounted on a specialized chamber made of silicone and a glass coverslip, and was placed on top of the chamber. The coverslip was immersed in 1 ml of 1 × TBS and then imaged using multiphoton microscopy (Olympus FV1000MPE, using a specialized 25x/1.05 NA water immersion objective, Weill-Cornell Medicine Imaging Core Facility). The tissue was then imaged in sectors. The images were captured, z-stacks were assembled, and a 2-dimensional reconstruction was constructed (Fiji software (Schneider et al., "NIH Image to ImageJ: 25 Years of Image Analysis," Nat Methods 9:671-675 (2012); Schindelin et al., "Fiji: An Open-Source Platform for Biological-ImageAnalysis," Nat. Methods 9:676-682 (2012)) and Imaris software (Bitplane), 6-regions imaged per specimen, n = 3). The data was analyzed for staining of extracellular protein EVs and cells were measured and counted as described below.

### Example 17 - Differentiating Vitreous Cells from Extracellular Vesicles in Tissue Sample

The goal was to identify EVs and extracellular RNA in the vitreous tissue. To do this, vitreous cells (presumed hyalocytes) and EVs were differentiated by the following method. Multiphoton or confocal images were obtained of EDC-formalin fixed bovine vitreous co-stained with Hoechst and CFSE as described above. Using these images, vitreous cells were identified by identifying the nuclei using the Hoechst signal and then identifying the cell bodies by using the CFSE signal. The diameter of the cell bodies was measured from over 100 cells (n = 3 biological samples, 6 image frames per sample) using ImageJ software (Schneider et al., "NIH Image to ImageJ: 25 Years of Image Analysis," Nat. Methods 9:671-675 (2012)). The average vitreous cell body diameter and standard deviation (SD) was calculated and the data graphically presented. It was found that the average vitreous cell size was 10.5 µm ± 1.77 µm and normally distributed. Thus, an upper limit diameter of 2 SD above the mean (14 µm) would encompass approximately 97.5% of cells. Therefore, in ImageJ software, a 14 µm circle centered on the nuclei was drawn, and positive signal was considered within this circle as intracellular protein. Signal outside this 14 µm circle was considered as extracellular. Two independent and blinded research assistants were used to count EVs. The criteria for counting EVs included round shape, location outside of the cell radius, and size larger than 100 nm and smaller than cells. The data was normalized by dividing the number of EVs counted per frame by the number of cells in the frame. The data are represented graphically. The size of bovine vitreous EVs were also measured using similar techniques (n = 4, and 3 biological replicates).

### Example 18 - Electron Microscopy of Vitreous Humor and Ocular Tissues

Human or bovine vitreous tissue was obtained as described above. Samples were cleared of cells with low speed centrifugation and whole mount specimens tested with H and E staining and imaging as described below. For vitreous, 2 µL was pipetted onto a block and fixed in a solution of 2.5% glutaraldehyde, 4% paraformaldehyde, 0.02% picric acid in 0.1M sodium cacodylate buffer and incubated for 60 min at room temperature (Ito et al., "Formaldehyde-Glutaraldehyde Fixatives Containing Trinitro Compounds," J Cell Biol 39:A168 (1968), which is hereby incorporated by reference in its entirety). Specimens were washed with excess volume of buffer (pH 7.3) for 5 minutes each at room temperature. Samples were post-fixed with 1% OsO₄-1.5% K-ferricyanide (aqueous) for 60 min at room temperature (de Bruijn W.C., "Glycogen, Its Chemistry and Morphologic Appearance in the Electron Microscope. I. A Modified OsO4 Fixative Which Selectively Contrasts Glycogen," J UltrastructRes 42:29-50 (1973)). Samples were washed with buffer 3 times for 5 min each at room temperature. Samples were set *en bloc* and stained with 1.5% uranyl acetate for 60 min at room temperature. Samples were dehydrated through graded ethanol series and transitioned through acetonitrile. Samples were infiltrated and embedded in Embed 812 resin (Electron Microscopy Sciences). Tissue sections were cut at 60-65 nm using a Diatome diamond knife (Diatome) on Leica Ultracut T ultramicrotome (Leica Microsystems). Sections were contrasted with lead citrate (Venable et al., "A Simplified Lead Citrate Stain for Use in Electron Microscopy," J Cell Biol 25:407-408 (1965)) and viewed on a JEM 1400 electron microscope (JEOL, USA, Inc) operated at 100kV. Digital images were captured on a Veleta 2K × 2K CCD camera (Olympus-SIS). Electron microscopy images were recorded and analyzed for size and frequency of EVs using ImageJ software. For protein staining on TEM, 500 µM CFSE diluted in TBS (pH 7.4) was mixed with 5 µl of ultracentrifuge purified EVs for 30 min at 25°C. All samples above were then fixed, mounted, and imaged with TEM as above. For TEM visualization of vitreous EVs, vesicles were isolated from human or bovine vitreous through ultracentrifugation as described below, re-suspended in formaldehyde, loaded on Formvar/carbon-coated EM grids, postfixed in 1% glutaraldehyde, and contrasted successively in 2% uranyl acetate, pH 7, and 2% methylcellulose/0.4% uranyl acetate, pH 4, or CFSE.

### Example 19 - Extracellular Vesicle Isolation and Purification of Tissue Samples

Methods for isolating extracellular vesicles from fluids were adapted (Thery et al., Isolation and Characterization of Exosomes from Cell Culture Supernatants and Biological Fluids," Curr Protoc Cell Biol Chapter 3, Unit 3:22 (2006)).

In this study, the goal was to have vitreous specimens free of cells. Therefore, the vitreous was cleared with a series of low-speed centrifugations. Approximately 8 ml of vitreous was placed in 15 ml tubes and centrifuged in Sorvall legend RT Swinging bucket (Sorvall) at 2,000 g (2500 rpm) at 4°C for 30 minutes. The supernatant was then transferred to a new 15 ml tube. Then, the centrifugation step was repeated. The supernatant was then transferred to new tube and centrifuged at 10,000 g in a Sorvall RC-58 centrifuge (Sorvall) using an SS-34 rotor (DuPont) for 30 min at 4°C. For each aliquot of vitreous or aqueous humor, whole mount hematoxylin and eosin (H and E) staining was conducted to survey for cells as described below (Figure 18). Whole mount slides were then imaged and all cell free samples were further processed. The supernatant was then transferred and the step was repeated. The sample was transferred to an ultracentrifuge tube (Beckman) and in a swinging bucket rotor (SW-41, Beckman) and centrifuged at 100,000 g in an L7-55 ultracentrifuge (Beckman) at 4°C for 1 hour. The supernatant was transferred to a new tube. The step was repeated. Samples were resuspended in 50 µl of sterile phosphate buffered saline (PBS, pH 7.5) and placed in a siliconized tube. Samples for imaging were immediately processed, and remaining sample was frozen at -80°C.

### Example 20 - Vitreous Histochemical Staining to Confirm Acellularity of Samples

To optimize vitreous EV isolation techniques, histochemical stains were applied after low-speed centrifugation to exclude vitreous samples contaminated by cells. Vitreous samples were dissected and collected as above. Acellularity was confirmed by whole mounting centrifuged vitreous onto glass slides and then subjecting the specimen to histochemical staining with H and E. Approximately 1 ml of vitreous supernatant was placed on SuperFrost Plus glass slides (Thermo Fisher Scientific) and then dried in a chamber for 16 hours at 4 °C. The dried slides were rinsed with 5 mls of 1 × TBS for 3 min at room temperature, and then washed again. The slides were then stained with H and E using standard procedures. Slides were preserved by mounting glass coverslips and then sealed. Samples were analyzed with light microscopy as described below. Specimens with hematoxylin-stained cells were subjected to repeat centrifugation or discarded. Therefore, all vitreous fractions used for further experiments were free from contaminating vitreous cells.

### Example 21 - Nanoparticle Tracking Analysis

The NanoSight NS300 system (Malvern) was used to perform nanoparticle tracking analysis to characterize particles from 30 - 800 nm in solution. Extracellular vesicles isolated from bovine vitreous were re-suspended in 100 µl of phosphate buffered saline (PBS, pH 7.0) at a concentration of approximately 2.5 µg of protein per ml, and then the sample was diluted to a final volume of 2 ml in PBS for analysis. Particles were loaded, the camera was focused, and 5 videos were captured for 60 see each. Videos were recorded and then analyzed using NanoSight software (Version 3.0) to determine the size distribution and particle concentration of EVs. Graphs were created. The Brownian motion of each particle was tracked between frames, ultimately allowing calculation of the size through application of the Stokes-Einstein equation.

### Example 22 - Evaluation of Extracellular Vesicle Loss from Formalin-Fixed Tissue

Whole bovine vitreous micro-dissected as described above was placed in a 50 ml conical tube and then submerged in 10 ml of 4 % formalin diluted in TBS (pH 7.4) and incubated for 24 h at 4°C. After fixation, tissues were dissected on ice into approximately 1 cm × 1 cm sections and the weight of vitreous section was recorded. The tissues were then placed in 15 ml centrifuge tubes. The tissues were immersed in 250 µl of TBS and the samples and overlying wash buffer (or supernatant) were incubated at 37°C for 1 hr (n = 3). The supernatants were collected and imaged with TEM and UA negative staining for further studies.

### Example 23 - Immunohistochemistry of Exosome Marker Proteins in Vitreous

Immunohistochemistry (IHC) was performed on whole mounted 4% formalin-fixed bovine vitreous. To prevent formalin crosslinks from reverting, and thus reduce the rate of EV loss, all experiments were conducted at 4°C for the duration of the experiment, except for wide-field epi-fluorescent microscopic imaging. The bovine vitreous humor was cut into approximately 1 cm × 1 cm pieces and then rinsed the specimen in 5 ml of ice-cold TBS (pH 7.4) for 3 minutes at 4°C. The wash steps were repeated twice. Specimens were then examined with a dissecting microscope (SZX-16 Olympus) to remove potentially contaminating tissues. Samples were then immersed in 500 µl of blocking buffer (10% goat serum diluted in TBS) for 1 h at 4°C. The samples were briefly washed in 5 ml of TBS for 3 min at 4°C. Rabbit monoclonal anti-TSG-101 antibody (Abeam PLC, diluted 1:500) was used to immunostain the bovine vitreous overnight at 4°C. The samples were washed in 5 ml of TBS for 3 min at 4°C. The wash steps were repeated twice. IHC staining was visualized using a secondary antibody, goat anti-rabbit IgG conjugated to Alexa Fluor 488 (Abcam PLC). The samples were washed three times. Bovine vitreous was counterstained with Hoechst stain (as described above) to mark nuclei and then washed twice in 5 ml of TBS for 5 min at 4°C. The vitreous was then immediately imaged and photomicrographs were recorded. For negative controls, normal goat serum (1:1000 dilution) was substituted for the primary antibody (secondary antibody only).

### Example 24 - Vitreous Proteome Analysis

Bovine vitreous samples were cleared of cells using the above protocol and whole mount samples were determined to be cell free by whole mount Hand E staining and subsequent imaging as described above. Samples free of cells were then selected for proteomic analysis. Extracellular vesicles were isolated as described above. Protein from the extracellular vesicle fraction or cell free vitreous fraction was denatured in 8M urea, and cysteines were reduced with dithiothreitol (Sigma Aldrich) prior to alkylation with iodoacetamide (Sigma Aldrich). The proteins were digested with LysC (Wako Chemicals) followed by trypsin (Promega) and desalted with Empore C18 STaGETips (3M) (Ishihamaet al., "Modular Stop and go Extraction Tips with Stacked Disks for Parallel and Multidimensional Peptide Fractionation in Proteomics," J Proteome Res 5:988-994 (2006)).

One µg of total protein was injected for nano-LC-MS/MS analysis (Q-Exactive Plus, Thermo Scientific). The peptides were separated using a 12 cm × 75 µm C18 column (Nikkyo Technos Co., Ltd. Japan) at a flow rate of 200 nL / min, with a 5-40% gradient over 160 minutes (buffer A 0.1% formic acid, buffer B 0.1% formic acid in acetonitrile). The Q-Exactive Plus was operated in data-dependent mode, with a top 20 method. Nano-LC-MS/MS data were analyzed using MaxQuant (version 1.6) (Cox et al., "MaxQuant Enables High Peptide Identification Rates, Individualized p.p.b.-Range Mass Accuracies and Proteome-Wide Protein Quantification," Nat Biotechnol 26:1367-1372 (2008)) and Perseus software (version 1.4) (Tyanova et al., "The Perseus Computational Platform for Comprehensive Analysis of (Prote)omics Data," Nat Methods (2016)), searching against a Uniprot *Bos taurus* database (downloaded July 2014) (UniProt C, "UniProt: A Hub for Protein Information," Nucleic Acids Res 43:D204-212 (2015)), allowing oxidation of methionine and protein N-terminal acetylation, and filtering at a 2% false discovery rate at the peptide and 1% at protein level. The proteins were quantified using iBAQ values. Protein enrichment was compared between vitreous extracellular vesicle fraction and cell free whole vitreous fraction.

### Example 25 - ARPE-19 Cell Culture

Human retinal pigmented epithelial cells, ARPE-19 (ATCC) were cultured in DMEM:F12 medium (ThermoFisher Scientific) supplemented with 10% fetal bovine serum and penicillin and streptomycin. All cells were incubated at 37 °C in 95% air and 5% CO₂ and maintained using standard sterile techniques.

### Example 26 - Loading Recombinant Proteins into Extracellular Vesicles

The isolated bovine vitreous EVs, as described above, were measured for the total protein concentration (Pierce^{™} BCA Protein Assay Kit, Thermo Fisher Scientific). 4 µg of vitreous EVs were used for *in vitro* treatments and 0.025 µg of bovine vitreous EVs for *in vivo* injections along with the following concentrations of BSA-fluorescein (3 µg, 1 µg, and 0.5 µg) or GFP (0.25 µg, 0.5 µg, and 1 µg). Recombinant protein and EVs were mixed in 300 µl of electroporation buffer (BioRad) and electroporated in a 4 mm cuvette. The electroporation of the EVs was preformed using a square wave program under the following conditions; voltage at 300 V, pulse length time of 35 ms, with the number of pulses at 2, and pulse interval of 0.1 sec. For controls, the same concentrations of EVs were mixed with the optimal concentration of recombinant protein without electroporation (0 V). For *in vivo* studies, samples were desalted after resuspension in balanced salt solution 5 volumes and then concentrated with centrifugal size exclusion filters (Amicon, Millipore Sigma). The re-suspension volume in balanced salt solution (BSS) was 75 µl and 0.5 µl used per injection.

### Example 27 - In Vitro Application of Extracellular Vesicles to Cultured Cells

Bovine or post-mortem human vitreous EVs were isolated and loaded with recombinant protein via electroporation as described above. ARPE-19 cells were cultured on a 12-well plate and approximately 70% confluent at the time of EV treatment. Then, 100 µl of the electroporated EV solution was added to 1 ml of complete media. The cells were incubated for 16 h under standard culture conditions and then the media was removed and replaced with complete media. At 48 h post-treatment, cell media was removed and cultures immersed with 1 ml of Hoechst stain and incubated for 15 min at 37°C. The stain was removed and cells were washed with 2 ml of phosphate buffered saline and fixed with 2 mls of 4% formalin diluted in PBS for 10 min at room temperature. Cells were washed with 2 ml of PBS for 5 min. The wash was repeated twice. Cells were evaluated for transfection efficiency with using wide-field fluorescent microscopy.

### Example 28 - In Vivo Injection of Vitreous Extracellular Vesicles

All procedures were performed in accordance with NIH guidelines and approved by Weill Cornell Medicine's Institutional Animal Care and Use Committee (IACUC). Male, 6-week-old C57BL/6J mice (Jackson Labs) were maintained on a 12-h light/dark cycle at Weill Cornell Medical College's Research Animal Resource Center (RARC). Intravitreal injections of mouse eyes occurred at 8 weeks of age in all experimental variables (n ≥ 3). The animals were sedated with a ketamine and xylazine cocktail in accordance with NIH Animal Welfare guidelines. The animals' pupils were dilated with 1 drop of 2.5% phenylephrine, 1 drop of 1% tropicamide, and then a lubricating ophthalmic ointment was applied. After 15 min, the animals were prepared for injection. The ophthalmic ointment was removed using a cotton swab and eyes rinsed with 10 drops of 1X TBS. Under a dissecting stereo microscope (Olympus SZX50), a guide track was made in the eye by positioning a 32-gauge needle at the limbus and then traversed from the sclera and into the posterior chamber. Care was taken to avoid disrupting the crystalline lens. Next, the guide needle was withdrawn and the micro-injector (Pneumatic picopump, PV830, World Precision Instruments) was positioned into the guide needle track and the glass pipette tip was inserted into the posterior segment avoiding the retina. 500 nl of EV solution or control solutions was injected. After completion of the injection, a 10 sec interval was maintained before removing the glass pipette. The glass pipette was removed and ophthalmic antibiotic ointment applied to the injected eye immediately after the intravitreal injection procedure. The animals were then monitored for recovery from anesthesia and then returned to the Weill Cornell Medicine's RARC Facility.

### Example 29 - Evaluation of Bio-distribution of Intravitreally Injected Extracellular Vesicles or Controls in Rodent Eyes

The bio-distribution of EV intravitreal injection was analyzed at post injection day 3, week 1, and week 3 (n ≥ 3). Animals were sedated and euthanized in accordance with NIH Animal Welfare guidelines. The eyes were enucleated and placed in 5 ml of 4% formalin in 1X TBS for 16-hr at 4°C and then immersed in 5 ml of 0.5 M sucrose diluted in TBS for 12 h at 4°C. The tissues were mounted in OCT Compound (Tissue-Tek), frozen in a dry-ice/ethanol bath in a Cryomold (Tissue-Tek), immediately serial sectioned from 5 to 40 µm with a cryostat (Leica 3050 S, Leica) and mounted on SuperFrost Plus glass slides (Thermo Fisher Scientific). The specimens were counterstained with 1 ml of Hoechst stain for 15 min at room temperature. The slides were rinsed in 5 ml of TBS (pH 7.4) for 5 min at room temperature. Wash steps were repeated twice. Then 300 µl of mounting media was added and a cover-slip (VWR International LLC) placed on top. Slides were imaged with wide field fluorescent microscopy for BSA-fluorescein. Unprocessed specimen and mounted slides were stored at -80°C.

### Example 30 - Statistical Analyses

Graph visualization and calculations were performed using Excel (version 2011, Microsoft). All experiments, unless otherwise stated, were performed with n ≥ 3 of distinct experimental samples. For nanoparticle tracking analysis the particle size, concentration, and distribution was calculated using Stokes- Einstein equation. Statistical analyses were carried out using unpaired Student's t-test using SPSS software, and p values < 0.05 were taken to be significant.

To optimize established TEM and negative staining procedures for fluid samples, EVs isolated from the vitreous humor (gel like matrix, located in the eye) and aqueous humor were used as a model system. The vitreous body (vitreous) of the eye is located between the lens and the retina, and is mostly acellular tissue. The vitreous is largely composed of water and an extracellular gel matrix of predominantly Type II collagen fibrils in association with hyaluronic acid. First, the vitreous humor was dissected from the posterior chamber of the eye, the sample homogenized, EVs isolated using ultracentrifugation, and the sample resuspended in buffered saline. Next, the number and size of EVs was quantified using nanoparticle tracking analysis (NTA) and 3.98 × 10⁸ EVs per ml were found. To visualize the ultrastructure of vitreous EVs suspended in a fluid, conventional glutaraldehyde -based TEM imaging protocols were followed (Figure 1A) (Stradleigh et al., "Fixation Strategies for Retinal Immunohistochemistry," Progress in Retinal and Eye Research 48:181-202 (2015)). Approximately 4 × 10⁶ EVs were applied to an electron microscopy grid, after incubation the sample was removed, glutaraldehyde fixation applied, the sample washed, and then negative staining with a uranyl acetate solution was conducted (Figure 1A). Subsequent imaging of the specimens using TEM detected very few EVs (Figure 1B). Using glutaraldehyde fixation, an average of 0.033 (± 0.182) EVs were observed per 25,000x high-powered micrographic field (n = 3 biological replicates, and 10 photos captured of equal size), which seems incongruent with loading over 4 million EVs. These data suggested that EVs were either destroyed during specimen processing or lost to the aspirated fluid. Therefore, to permanently adhere EVs on the grid, an additional fixation step was added using EDC, a carbodiimide that creates a non-reversible crosslink between positively charged amino group side chains and carboxyl groups of proteins.

To test the hypothesis that EDC would irreversibly fix EVs, inactive EDC (cold, (4°C)) was mixed with 4 million bovine vitreous EVs that were re-suspended in buffered saline, then applied the ice-cold solution to the surface of a poly-l-lysine coated formvar electron microscopy grid (Figure 1C). Next, the EDC solution was activated by applying heat and later added glutaraldehyde, the sample washed, followed by negative staining. The images showed a robust amount of bovine vitreous EVs that were imaged with negative staining (Figure 1D). Aqueous humor samples fixed with EDC showed 16.5 EVs (± 16.9) per 25,000x high-powered field under matching conditions. Significantly, more EVs (357-fold) were identified in EDC fixed fluid samples, when compared to glutaraldehyde-fixed samples (p<0.05, n=3), (Figure 1E), suggesting that EDC fixation of EVs suspended in biological fluids is superior to conventional glutaraldehyde fixation. These data show that for imaging EVs in a fluid, EDC-glutaraldehyde fixation is significantly superior when compared to glutaraldehyde fixation alone. In summary, standard TEM and negative staining protocols result in substantial failure of EVs to adhere to the surface of the electron microscopy grids. However, fixation of proteins with EDC-glutaraldehyde acts to retain EVs and allows for robust imaging of EVs in biological fluids. Moreover, this technology represents a substantial improvement in EV imaging methods.

To explain the discrepancy in the amount of EVs imaged using the EDC-glutaraldehyde fixed fluid technique and conventional glutaraldehyde alone fixed specimen, each procedural step of the glutaraldehyde-based protocol was examined for the potential cause for EV loss. It was hypothesized that EV were located on surface of the grid or in the aspirated wash buffer. Therefore, the EV content in the aspirated fluid was examined by imaging the aspirate using a separate grid and EDC-glutaraldehyde fixation. To test for EV loss, a solution of isolated bovine aqueous humor EVs were applied to the grid and then incubated. Next, the aspirated content was kept and imaged on a separate grid using EDC-glutaraldehyde fixation. It was noted that there was a considerable amount of EVs present in the aspirated solution (Figure 2A), suggesting that the EVs failed to adhere to the surface of the grid. Then, it was also found that EVs were lost during glutaraldehyde fixation (Figure 2B), and wash steps (Figure 2C); with only a few EVs remaining on the grid during TEM imaging (Figure 2D). The amount of loss at each step was quantified, and the data showed that most EVs do not adhere to the grid at the initial step, with very few remaining at the final imaging step (Figure 2E). Overall, these data show that imaging EV suspended in a fluid using conventional TEM and negative staining protocols results in massive failure of EVs to adhere to the grid and subsequent loss of EVs during glutaraldehyde fixation and wash steps. Moreover, conventional TEM and negative staining results in non-representative population of EVs, suggesting that the conventional method is an inefficient and inconsistent for imaging EVs in liquids.

To broaden the scope of this technique, other clinically relevant biological fluids were examined. EVs are known to be present in higher concentration in the blood of patients with cancer and tumor-derived EVs are thought to play an important role in tumor growth and metastasis. Therefore, imaging EVs in plasma (blood product) from patients with central nervous system tumors was pursued. The plasma from patients with a diagnosis of glioma was obtained, the EVs isolated with ultracentrifugation, and then EDC-glutaraldehyde-fixation conducted, followed by negative staining and TEM imaging. The data showed that multiple patients with glioma contain numerous EVs (Figures 3A-C), which have significant different morphology, abundance, and size when compared to healthy control patient plasma (Figures 4A -B). To examine other malignancies, the EVs isolated from the plasma of patients with systemic melanoma were visualized. Electron dense signal resembling EVs in the plasma of patients with systemic melanoma was observed (Figures 5A-B), albeit with differing morphology when compared to EVs from patients with glioma (Figures 3A-C) or healthy controls (Figures 4A-B). These data suggest that modified EDC-glutaraldehyde fixation enables identification of tumor-derived EVs in the blood product of patients with cancer. Moreover, these data suggest that this method may be a useful tool for cancer diagnosis, prognosis or an indicator of metastatic potential.

Next, biomarkers secreted in biological fluid of central nervous system tumors were examined by inspecting EV contents in patients' cerebrospinal fluid (CSF). Therefore, CSF was obtained from patients with neuroblastoma, a tumor arising from progenitor cells of the sympathetic nervous system, and the most common solid pediatric solid tumor (Brodeur, "Neuroblastoma: Biological Insights Into A Clinical Enigma," Nat Rev Cancer 3:203-216 (2003)).

The EVs isolated, the samples fixed with EDC-glutaraldehyde fixation, conducted negative staining, and then the samples were imaged with TEM. The images showed that neuroblastoma derived EVs are larger in size and contain an electron dense substance surrounding the EVs (Figures 6A-B). To broaden the scope of this technique to other tumors, EVs isolated from the CSF donated by a patient with the diagnosis of sarcoma were examined (Figures 6C-D), a tumor derived from mesenchymal cells, such as muscle, bone or vascular tissue. Again, the EDC-glutaraldehyde fixation enabled the identification of EVs in the CSF that were smaller and morphologically distinct when compared to EVs isolated from patients with neuroblastoma (Figures 6A-B). These data suggest that the CSF is another source of biological fluids that may be imaged using this technique. Moreover, the results suggest that EDC-glutaraldehyde fixation is useful for more than one biological fluid and imaging biomarkers from various cancers. Finally, these findings may have important implications for the application of EV imaging for central nervous system involving cancers.

Finally, biomarkers using liquid biopsy from patients with the most highly prevalent cancer type, a carcinoma patients were examined (Siegel, et al., "Cancer Statistics, 2017," CA Cancer J Clin 67:7-30 (2017)).

To do this, nipple aspirate fluid (NAF) (Harris et al. "American Society of Clinical Oncology 2007 Update of Recommendations for the Use of Tumor Markers in Breast Cancer," J Clin Oncol 25:5287-5312 (2007)) was collected from patients with a diagnosis of breast cancer or healthy controls, and EDC-glutaraldehyde fixation conduced, followed by negative staining and TEM imaging. The data showed a signal resembling EVs the nipple aspirate fluid (Figure 7). These data suggest that the EDC-glutaraldehyde fixation method is capable of detecting EVs in carcinoma, and that nipple aspirate fluid is another source of biological fluid that may be used for liquid biopsy.

To further optimize the TEM liquid imaging technique, methods for staining EVs were improved. Conventional TEM imaging of EVs requires negative staining, which allows for buildup of electron dense uranyl acetate stain around the edges of the EV, producing a signal on the edge of the vesicle and a less electron dense central core (Figure 8A and Figure 9A). Here, it is proposed to use a "positive stain" that shows an electron dense signal that highlights the EV itself. Exosomes are known to contain RNAs (Valadi et al., "Exosome-Mediated Transfer of mRNAs and MicroRNAs is a Novel Mechanism of Genetic Exchange Between Cells," Nat Cell Biol 9:654-659 (2007)); therefore, bovine vitreous humor with an was stained electron dense and nucleic acid selective dye, acridine orange that a showed positive staining within the EVs (Figure 8B). It is possible to negatively stain EVs from the plasma from a patient with glioma (Figure 8C, left panel) and then positively stain the sample with acridine orange as shown in Figure 8C, right panel. Then, proteins of whole mount bovine vitreous were labeled with a cell permeable and electron dense (Griffith et al., "Epithelial-Mesenchymal Transformation During Palatal Fusion: Carboxyfluorescein Traces Cells at Light and Electron Microscopic Levels," Development 116:1087-1099 (1992)), stain carboxyfluorescein succinimidyl ester (CFSE) (Bronner-Fraser, M., "Alterations in Neural Crest Migration by a Monoclonal Antibody That Affects Cell Adhesion," J. Cell Biol. 101:610-617 (1985)), which covalently links to intracellular amines. Then, proteins in the specimens were labeled with CSFE and imaged with TEM (Figure 9B) and the electron microscopy images show an abundant number of vesicles with intra-vesicular staining. Isolated bovine vitreous EVs were labeled with AO, showing positive staining (Figure 9C). Next, whole mounted bovine vitreous were stained with ethidium bromide (EtBr), an electron dense nucleic acid stain, and multiple EVs (arrowheads) were seen in a network of collagen (Figure 9D). EVs were also a isolated from post-mortem human vitreous and stained with AO (Figure 9E). The data suggest that EVs can be marked with various electron dense dyes and labeled with a "positive stain."

The final objective was to image the spatial localization of EVs as they normally exist within a small volume of biological fluid, or to visualize EVs *in situ.* Therefore, attempts to detect EVs without using purification protocols were made to try and directly detect EVs in their native environment of the biological fluid. A minute sample of human aqueous humor (2.5 µl) was obtained and the undiluted fluid applied to the surface of the grid, EDC-glutaraldehyde fixation was conducted followed by negative staining and then imaging with TEM. For the undiluted aqueous fluid, the photographs showed a high amount of background (Figure 10A, left and right, black signal) with no easily identifiable EVs. To improve the signal to noise ratio, the sample was diluted in buffered saline and the procedure repeated. It was noted that substantially less background was observed after diluting the sample, which allows for the identification of more EVs in the diluted specimen (Figure 10B-D). These data show that it is possible to image EVs directly from a small amount of fluid from a human liquid biopsy; that may serve as biomarker for diagnostic, prognostic or to influence therapy for EV-related disease, or to exclude disease in the aqueous humor, or in other biological fluids.

In summary, it is shown that conventional protocols for imaging EVs with negative staining and TEM result in massive loss of EVs suspended in solution, resulting in inconsistent imaging, underestimation of EV abundance or negative results. In contrast, crosslinking EVs using EDC-glutaraldehyde fixation significantly improves retention of EVs, enables robust imaging of EV ultrastructure in biological fluids, and allows for improved representation of the heterogeneous population of EVs. Additionally, this fixation method may be broadly applied towards EV-based diagnostic techniques, including liquid biopsy. Finally, this technique allows for imaging the structural mediators of metastasis in many types of cancers. It is expected that the EDC-glutaraldehyde fixation method will be broadly applicable for imaging EVs associated with other biological fluid specimens (plasma, cerebrospinal fluid, and ductal fluid) from patients with a variety of other highly prevalent cancers. Moreover, this basic technology will allow for the study of the structure of EVs in ocular fluids, plasma, CSF, and ductal fluid and may aid the elucidation of basic mechanisms underlying cancer progression and metastasis. In summary, EDC fixation combined with TEM may serve as a new technology for liquid biopsy that may help distinguish, assess, and monitor cancer stages and progression.

This method was then applied to the study EVs in tissues, and the vitreous body of the eye was used as a model system. The vitreous, located between the lens and the retina, is an optically clear, paucicellular tissue with abundant extracellular matrix (ECM) and little-known biological function (Le Goff et al., "Adult Vitreous Structure and Postnatal Changes," Eye (Lond) 22:1214-1222 (2008)). Vitreous EV-associated microRNAs have been described (Ragusa et al., "miRNA Profiling in Vitreous Humor, Vitreal Exosomes and Serum From Uveal Melanoma Patients: Pathological and Diagnostic Implications," Cancer Biol. Ther. 16:1387-1396 (2015)); however, normal vitreous EVs have not yet been imaged nor characterized. It is hypothesized that normal vitreous possesses EVs, yet repeated attempts to visualize the nanoparticles using multiphoton, confocal or wide-field microscopy failed Here, it is shown that standard formalin fixation results in loss of EVs from tissue, whereas fixation of proteins with 1-ethyl-3-(3-dimethylaminopropyl) carbodiimide (EDC) retains EVs and allows for EV imaging *in situ.*

The study then shifted focus onto optimizing tissue fixation. Conventional fixation methods use 10% formalin to create protein-protein crosslinks. Tissue processing steps generally occur at or above room temperature; however, elevated temperatures are known to revert formalin protein-protein and RNA-protein crosslinks (Shi et al., "Antigen Retrieval in Formalin-Fixed, Paraffin-Embedded Tissues: an Enhancement Method for Immunohistochemical Staining Based on Microwave Oven Heating of Tissue Sections," J. Histochem. Cytochem. 39:741-748 (1991); Ikeda et al., "Extraction and Analysis of Diagnostically Useful Proteins from Formalin-Fixed, Paraffin-Embedded Tissue Sections," J. Histochem. Cytochem. 46:397-403 (1998); Pena et al., "miRNA In Situ Hybridization in Formaldehyde and EDC-Fixed Tissues," Nat. Methods 6:139-141 (2009). It is hypothesized that EVs are lost from formalin-fixed tissues during processing and imaging steps (Figure 11A). To examine EV loss from formalin-fixed tissues, formalin-fixed bovine vitreous is immersed in wash buffer at 37°C and then the supernatant collected. Transmission electron microscopy (TEM) of this supernatant revealed a substantial number of EVs lost from the tissue (Figure 11B-C). EV loss was noted at all temperatures tested, with fewer EVs lost at 4°C (Figure 11D) and considerable loss at elevated temperature (Figure 11E-F). To permanently retain EVs within the tissue, fixation with EDC was added to create a non-reversible crosslink between positively-charged amino group side chains and carboxyl groups of proteins. Under similar conditions, EDC-formalin fixation showed no EV loss to wash buffer (Figure 11G-H). Particulate matter was observed in the EDC-formalin supernatant, as well as the wash buffer control (Figure 11I). These data suggest that EV loss from formalin-fixed specimens, and that EDC-formalin fixation retains EVs in tissues.

To visualize EVs in the extracellular space of vitreous tissue (Figure 12A), compared conventional fixation (formalin alone) was compared to versus EDC-formalin fixation, and then EVs visualization *in situ* was attempted. EVs are known to contain proteins; thus, protein were labeled in whole mounted specimens with carboxyfluorescein succinimidyl ester (CFSE) fluorescent dye (Bronner-Fraser, M., "Alterations in Neural Crest Migration by a Monoclonal Antibody That Affects Cell Adhesion," J. Cell Biol. 101:610-617 (1985)) in whole mounted specimens and then imaged with multiphoton microscopy. Formalin-fixed vitreous showed protein signal within cells but showed no extracellular signal (Figure 12B , n = 4), suggesting that EVs were either absent or lost during processing. In contrast, EDC-formalin-fixed vitreous show a robust EV-shaped protein signal in the ECM (Figure 12C-D). Significantly more EVs were identified in EDC-formalin-fixed tissues (143.2 with SD ± 23.8 EVs counted per image, n = 4) versus formalin-fixed tissues (1.2 with SD ± 0.9 EVs counted per image, n = 4), as shown in Figure 12E. Vitreous EVs show a heterogeneous population EV size based on MPM imaging (Figure 12F). To correlate the in situ optical microscopy findings with other methods used to visualize EVs, vitreous EV ultrastructure using TEM was studied (Raposo et al., "B Lymphocytes Secrete Antigen-Presenting Vesicles," J. Exp. Med. 183:1161-1172 (1996); Consortium et al., "EV-TRACK: Transparent Reporting and Centralizing Knowledge in Extracellular Vesicle Research," Nat. Methods 14:228-232 (2017)). Bovine vitreous tissue sections showed a substantial number of EVs in the ECM of the vitreous (Figure 12G). Next, bovine vitreous EVs were isolated and stained with CFSE, an electron dense dye (Griffith et al., "Epithelial-Mesenchymal Transformation During Palatal Fusion: Carboxyfluorescein Traces Cells at Light and Electron Microscopic Levels," Development 116:1087-1099 (1992)), and observed an abundance of EVs with intra-vesicular protein signal (Figure 12H). Nanoparticle-tracking analysis (NTA) (Dragovic et al., "Sizing and Phenotyping of Cellular Vesicles Using Nanoparticle Tracking Analysis," Nanomedicine 7:780-788 (2011)) revealed an EV concentration of at least 2.98 x 10⁷ particles per ml (s.e.m ± 8.98 x 10⁶, Figure 13A). EV size measured by NTA differed from EV size observed in situ by MPM (Figure 12F), which is likely the result of ultracentrifugation-based isolation methods removing larger EVs from the fluid being analyzed (van der Pol et al., "Recent Developments in the Nomenclature, Presence, Isolation, Detection and Clinical Impact of Extracellular Vesicles," J. Thromb. Haemost. 14:48-56 (2016)). TEM was performed on post-mortem human eyes and numerous EVs were identified in the ECM near the vitreous base and ciliary body (Figure 12I-J). The size distribution of isolated human vitreous EVs is shown in Figure 13B. These data suggest that EVs are present in the ECM of the vitreous and that ultrastructural imaging correlates with the optical imaging of EDC-formalin fixed tissue. Moreover, it is shown that EDC-formalin fixation is superior to formalin fixation for imaging EVs *in situ.*

EVs are known to contain extracellular RNA (Valadi et al., "Exosome-Mediated Transfer of mRNAs and MicroRNAs is a Novel Mechanism of Genetic Exchange Between Cells," Nat. Cell Biol. 9:654-659 (2007)); therefore, bovine vitreous nucleic acids were stained with propidium iodide (PI), which marks DNA and RNA (Suzuki et al., "DNA Staining for Fluorescence and Laser Confocal Microscopy," J. Histochem. Cytochem. 45:49-53 (1997)). Confocal microscopy imaging of EDC-formalin-fixed vitreous show signals positive for extracellular RNA and extracellular protein (Figure 14A-B). In contrast, fixation with formalin alone resulted in substantially less extracellular RNA and protein signal (Figure 14C). The loss of RNAs from tissues fixed with formalin is consistent with prior studies (Pena et al., "miRNA In Situ Hybridization in Formaldehyde and EDC-Fixed Tissues," Nat. Methods 6:139-141 (2009)). To verify that the extracellular PI signal was RNA, the EDC-formalin-fixed vitreous was treated with RNase and a significant reduction in extracellular signal (p<0.001; Figure 15A-C) was noted. Similar findings were observed using standard wide-field fluorescent microscopy (Figure 16A-B). Interestingly, normal vitreous EVs express RNA, but show no DNA signal. These data suggest that EDC-formalin fixation enables evaluation of the differential expression RNA or DNA within EVs *in situ.*

To broaden the usefulness of this technique for other tissues, a focused on imaging EVs secreted by cancer tissues was utilized (Becker et al., "Extracellular Vesicles in Cancer: Cell-to-Cell Mediators of Metastasis," Cancer Cell 30:836-848 (2016); D'Souza-Schorey et al., "Tumor-Derived Microvesicles: Shedding Light on Novel Microenvironment Modulators and Prospective Cancer Biomarkers," Genes Dev. 26: 1287-1299 (2012); Peinado et al., "The Secreted Factors Responsible for Pre-Metastatic Niche Formation: Old Sayings and New Thoughts," Semin. Cancer Biol. 21:139-146 (2011). Therefore, a murine metastatic breast cancer model was studied. To do this, 4T1 mouse mammary carcinoma tumor cells were transplanted into the mammary pad of a mouse (Pulaski et al., "Mouse 4T1 Breast Tumor Model," Curr. Protoc. Immunol. Chapter 20, Unit 20:22 (2001), the tumor dissected, the sample fixed with EDC-formalin, and the nucleic acids labeled. Next, the tumor surface was studied using MPM and the data showed extracellular RNA signal in the ECM, revealing a heterogeneous population of EVs (Figure 17A). Moreover, extracellular DNA was detected within a subpopulation of larger EVs (Figure 17B), consistent with other laboratories' findings that extracellular DNA is present within tumor-derived EVs (D'Souza-Schorey et al., "Tumor-Derived Microvesicles: Shedding Light on Novel Microenvironment Modulators and Prospective Cancer Biomarkers," Genes Dev. 26:1287-1299 (2012); Thakur et al., "Double-Stranded DNA in Exosomes: A Novel Biomarker in Cancer Detection," Cell Res. 24:766-769 (2014)). These data suggest that EDC-formalin fixation technique enables the spatial localization of nucleic acid expression in a subpopulation of EVs within a tissue. The ultrastructural analysis of mammary tumor tissues fixed with EDC and glutaraldehyde was also preformed using TEM. The data show a heterogeneous population of EVs in the ECM near the tumor cell (Figure 17C-D). The images support that EDC-formalin fixation retentions EVs and allows for imaging of EVs in the ECM of cancer specimens.

To determine if vitreous EVs expressed exosome-associated proteins, proteomic analysis was conducted using liquid chromatography mass spectrometry (LC-MS) comparing whole bovine vitreous with the EV isolated fraction. The data in Table 1 show EV-associated proteins like TSG-101 were enriched in the EV fraction. The table shows exosome markers that are enriched in the EV fraction identified by liquid chromatography-mass spectrometry analysis. The cell-free vitreous fraction was obtained by serial low-speed centrifugation, and the EV-enriched fraction (extracellular vesicle fraction) was obtained by serial ultracentrifugation of cell-free vitreous. Proteome analysis shows known exosome protein markers were enriched in the EV fraction (left column). The log₂ difference of EV fraction compared to cell-free vitreous fraction is listed, based on the amount of proteins quantified by label free quantification (LFQ) intensity in the EV-enriched fraction (third column) and the cell-free vitreous fraction. The proteins total intensity is represented by the iBAQ value (Schwanhausser et al., "Global Quantification of Mammalian Gene Expression Control," Nature 473:337-342 (2011); Voloboueva et al., "(R)-Alpha-Lipoic Acid Protects Retinal Pigment Epithelial Cells from Oxidative Damage," Invest Ophthalmol Vis Sci 46:4302-4310 (2005); Vlassov et al., "Exosomes: Current Knowledge of Their Composition, Biological Functions, and Diagnostic and Therapeutic Potentials," Biochim Biophys Acta 1820:940-948 (2012)). The right column references prior studies that identified the exosome, ectosome or EV markers (Vlassov et al., "Exosomes: Current Knowledge of Their Composition, Biological Functions, and Diagnostic and Therapeutic Potentials," Biochim Biophys Acta 1820:940-948 (2012); Conde-Vancells et al., "Characterization and Comprehensive Proteome Profiling of Exosomes Secreted by Hepatocytes," J Proteome Res 7:5157-5166 (2008); Higashiyama et al., "The Membrane Protein CD9/DRAP 27 Potentiates the Juxtacrine Growth Factor Activity of the Membrane-Anchored Heparin-Binding EGF-Like Growth Factor," J Cell Biol 128:929-938 (1995); Keerthikumar et al., "ExoCarta: A Web-Based Compendium of Exosomal Cargo," J Mol Biol 428:688-692 (2016); Thery et al., "Molecular Characterization of Dendritic Cell-Derived Exosomes. Selective Accumulation of the Heat Shock Protein hsc73," J Cell Biol 147:599-610 (1999)).

Additionally, the table displays the protein name, accession number and gene symbol, in addition to, the number of peptides matched (all and unique), and sequence coverage. Each experiment has listed the associated log₂ transformed iBAQ (intensity-based absolute quantification) value grouped according percentile (%ile) groups. For the cell-free vitreous fraction the 0.90, 0.75, median, 0.25 and 0.10 iBAQ percentiles were: 24.4, 22.3, 21.2, 19.1 and 17.5, respectively. For the extracellular vesicle enriched fraction the corresponding numbers were: 29.1, 26.8, 25.2, 22.5 and 21.1.

**Table 1. Selected Extracellular Vesicle Marker Proteins Enriched in Vitreous Extracellular Vesicles**

| | | | | | | | **Extracellular vesicle enriched fraction** | | **Cell-free vitreous fraction** | |
|---|---|---|---|---|---|---|---|---|---|---|
| **Ref.^{a}** | **Description** | **Gene** | **ID** | **Unique peptides** | **Peptides** | **Sequence coverage** | **iBAQ (log₂)** | ***%ile*** | **iBAQ (log2)** | ***%ile*** |
| 1 | Ras-related protein Rab-6B | RAB6B | A6QR46 | 4 | 8 | 48.6% | 23.8 | *0.75-0.90* | 25.0 | *0.25-0.5* |
| 1 | Ras-related protein Rab-11B | RB11B | Q3MHP2 | 10 | 10 | 56% | 22.8 | *0.75-0.90* | 25.2 | *0.5-0.75* |
| 2 | Ras-related C3 botulinum toxin substrate 1 | RAC1 | P62998 | 7 | 8 | 41.1% | 22.7 | *0.75-0.90* | 25.5 | *0.5-0.75* |
| 1, 2 | Ras-related protein Rab-3A | RAB3A | P11023 | 5 | 7 | 45% | 22.5 | *0.75-0.90* | 26.5 | *0.5-0.75* |
| 3, 4 | Ras-related | RAB5A | Q0IIG7 | 3 | 5 | 36.7% | 21.5 | *0.5-0.75* | - | - |
| | protein Rab-5A | | | | | | | | | |
| 5-8 | Tetraspanin | CD9 | G8JKX6 | 2 | 2 | 16.7% | 21.4 | *0.5-0.75* | - | - |
| 3, 4 | Ras-related protein Rab-7a | RAB7A | Q3T0F5 | 6 | 6 | 33.3% | 21.3 | *0.5-0.75* | 24.6 | *0.25-0.5* |
| 4 | RAB1A, member RAS oncogene family | RAB1A | A1L528 | 4 | 9 | 40.5% | 21.2 | *0.25-0.5* | 25.4 | *0.5-0.75* |
| 9 | Annexin A6 | ANXA6 | P79134 | 10 | 10 | 19% | 20.8 | *0.25-0.5* | - | |
| 10 | Ras-related protein Rab-3C | RAB3C | E1BF18 | 3 | 4 | 22.5% | 20.7 | *0.25-0.5* | - | - |
| 3, 4 | Ras-related protein Rab-5C | RAB5C | Q58DS9 | 4 | 6 | 421% | 206 | *0.25-0.5* | 22.7 | *0.25-0.5* |
| 11 | Annexin A1 | ANXA1 | P46193 | 7 | 7 | 26.3% | 20.5 | *0.25-0.5* | - | - |
| 10 | Annexin A5 | ANXA5 | P81287 | 7 | 7 | 25.5% | 20.5 | *0.25-0.5* | - | - |
| 11 | Ras-related protein Rap-1A | RAP1A | P62833 | 3 | 3 | 20.7% | 20.1 | *0.25-0.5* | 22.1 *0.10-0.25* | |
| 1, 10 | Annexin A2 | ANXA2 | P04272 | 4 | 5 | 162% | 19.4 | *0.25-0.5* | - | - |
| 10 | Ras-related protein Rab-4A | RAB4A | Q2TBH7 | 3 | 3 | 16.1% | 19.2 | *0.25-0.5* | - | - |
| 4 | Integrin beta-2 | ITB2 | P32592 | 4 | 4 | 4.6% | 18.7 | *0.10-0.25* | 21.7 | *0.10-0.25* |
| 12, 13 | Lysosome-associated membrane glycoprotein 1 | LAMP1 | Q05204 | 2 | 2 | 4.9% | 18.5 | *0.10-0.25* | - | - |
| 1, 10 | TSG101 protein | TSG10 1. | A3KN51 | 2 | 2 | 5.1% | 17.8 | *0.10-0.25* | - | - |

Table 1 References: (1) Ji et al., "Proteome Profiling of Exosomes Derived From Human Primary and Metastatic Colorectal Cancer Cells Reveal Differential Expression of Key Metastatic Factors and Signal Transduction Components," Proteomics 13:1672-1686 (2013); (2) Koppen et al., "Proteomics Analyses of Microvesicles Released by Drosophila Kc167 and S2 Cells," Proteomics 11:4397-4410 (2011); (3) Baietti et al., "Syndecan-Syntenin-ALIX Regulates the Biogenesis of Exosomes," Nat Cell Biol 14:677-685 (2012); (4) Kim et al., "Proteomic Analysis of Microvesicles Derived From Human Mesenchymal Stem Cells," J Proteome Res 11:839-849 (2012); (5) Schwanhausser et al., "Global Quantification of Mammalian Gene Expression Control," Nature 473:337-342 (2011); (6) Vlassov et al., "Exosomes: Current Knowledge of Their Composition, Biological Functions, and Diagnostic and Therapeutic Potentials," Biochim Biophys Acta 1820:940-948 (2012); (7) Higashiyama et al., "The Membrane Protein CD9/DRAP 27 Potentiates the Juxtacrine Growth Factor Activity of the Membrane-Anchored Heparin-Binding EGF-Like Growth Factor," J Cell Biol 128:929-938 (1995); (8) Keerthikumar et al., "ExoCarta: A Web-Based Compendium of Exosomal Cargo," J Mol Biol 428:688-692 (2016); (9) Keerthikumar et al., "Proteogenomic Analysis Reveals Exosomes are More Oncogenic Than Ectosomes," Oncotarget 6:15375-15396 (2015), (10) Inui et al., "Annexin VI Binds to a Synaptic Vesicle Protein, Synapsin I," J Neurochem 63:1917-1923 (1994); (11) Mallawaaratchy et al., "Comprehensive Proteome Profiling of Glioblastoma-Derived Extracellular Vesicles Identifies Markers for More Aggressive Disease," J Neurooncol 131:233-244 (2017); (12) Wolfers et al., "Tumor-Derived Exosomes are a Source of Shared Tumor Rejection Antigens for CTL Cross-Priming," Nat Med 7:297-303 (2001); (13) Raposo et al., "B Lymphocytes Secrete Antigen-Presenting Vesicles," J Exp Med 183:1161-1172 (1996).

To confirm that extracellular protein signals observed in the EDC-formalin-fixed vitreous were indeed EVs, immunohistochemistry (IHC) for TSG-101 was conducted. EDC-formalin fixation was incompatible with IHC; and TSG-101 signal was not reliably detected in formalin-fixed tissues processed at room temperature, presumably due to EV loss into wash buffer. Since formalin crosslink reversal is temperature dependent (Tkach etal. "Communication by Extracellular Vesicles: Where We Are and Where We Need to Go," Cell 164:1226-1232 (2016), IHC was performed at 4°C and then the samples immediately imaged with the microscope at room temperature. Punctate TSG-101-positive signals were visualized in the extracellular space (Figure 18A), consistent with the spatial distribution of CFSE-stained EVs in EDC-formalin-fixed tissues. Specificity controls showed no extracellular signal (Figure 18B). TSG-101 was 136-fold more prevalent in the extracellular space than within cell bodies (p<0.001; Figure 18C). Of note, the signal for TSG-101 was lost within minutes during imaging at room temperature, likely due to temperature-dependent reversion of formalin cross-links. Unlike vitreous fixed with EDC-formalin (Figure 14A-B), formalin-fixed samples processed at 4°C showed no extracellular nucleic acid signal (Figure 18D), also presumably from reversion of formalin nucleic acid cross-links. These data show that vitreous EVs contain markers consistent with well-established EV studies (Consortium et al., "EV-TRACK: Transparent Reporting and Centralizing Knowledge in Extracellular Vesicle Research," Nat. Methods 14:228-232 (2017)).

Table 2 shows proteins implicated in ocular physiology and pathophysiology that are enriched in the EV fraction, as identified by liquid chromatography-mass spectrometry analysis (nano-LC-MS/MS, Q-Exactive Plus, Thermo Scientific). The cell-free vitreous fraction was obtained by serial low-speed centrifugation, and the EV-enriched fraction was obtained by serial ultracentrifugation of cell-free vitreous. Proteome analysis shows known eye-specific proteins that are enriched in the EV fraction (left column). Proteome analysis shows known exosome protein markers were enriched in the EV fraction (left column). The log₂ difference of EV-enriched fraction compared to cell-free vitreous fraction is listed, based on the amount of proteins quantified by label free quantification (LFQ) intensity in the EV-enriched fraction (third column) and the cell-free vitreous fraction (data not shown). The right column references prior studies that identified these proteins in ocular physiology and pathophysiology. Protein name, accession number and gene symbol are shown in addition to number of peptides matched (all and unique), and sequence coverage. For each experiment is listed the associated log₂ transformed iBAQ (intensity-based absolute quantification) value grouped according percentile (%ile) groups. For the cell-free vitreous fraction the 0.90, 0.75, median, 0.25 and 0.10 iBAQ percentiles were: 24.4, 22.3, 21.2, 19.1 and 17.5, respectively. For the extracellular vesicle enriched fraction the corresponding numbers were: 29.1, 26.8, 25.2, 22.5 and 21.1.

**Table 2: Known eye-specific proteins enriched in vitreous extracellular vesicle fraction**

| | | | | | | | **Extracellular vesicle enriched fraction** | | **Cell-free vitreous fraction** | |
|---|---|---|---|---|---|---|---|---|---|---|
| **Ref.^{a}** | **Description** | **Gene** | **ID** | **Unique peptides** | **Peptides** | **Sequence coverage** | **iBAQ (log₂)** | ***%ile*** | **iBAQ (log2)** | ***%ile*** |
| 1-3 | Retinol-binding protein 3 | RET3 | P12661 | 87 | 2 | 72.2% | 30.7 | *>0.90* | 32.1 | *>0.90* |
| 4, 5 | Retinaldehyde-binding protein 1 | RLBP 1 | P10123 | 23 | 23 | 84.9% | 28.3 | *>0.90* | 30.1 | *>0.90* |
| 6, 7 | Opticin | OPT | P58874 | 15 | 15 | 43.9% | 28.1 | *>0.90* | 30.1 | *>0.90* |
| 8-11 | Fibrillin-1 | FBN1 | P98133 | 94 | 88 | 38.6% | 25.2 | *>0.90* | 26.4 | *0.50-0.75* |
| 12 | Arrestin-C | ARRC | Q9N0H5 | 8 | 8 | 31.6% | 21.9 | *0.50-0.75* | 23.1 | *0.25-0.50* |
| 8 | Fibulin-5 | FBLN5 | Q5EA62 | 6 | 6 | 15.8% | 21.4 | *0.50-0.75* | 21.8 | *0.10-0.25* |
| 13-15 | Rhodopsin | OPSD | P02699 | 2 | 2 | 8% | 21.1 | *0.25-0.50* | - | - |
| 16, 17 | 11-cis retinol dehydrogenase | RDH1 | Q27979 | 5 | 5 | 18.6% | 20.8 | *0.25-0.50* | - | - |
| 18, 19 | Retinoid isomerohydrolase (RPE65) | RPE6 5 | Q28175 | 5 | 5 | 16.9% | 19.8 | *0.25-0.50* | - | - |
| 20, 21 | Phakinin | BFSP 2 | Q28177 | 2 | 2 | 5.5% | 17.3 | *<0.10* | - | - |

Table 2 References: (1) Saari et al., "Photochemistry and Stereoselectivity of Cellular Retinaldehyde-Binding Protein from Bovine Retina," J Biol Chem 262:7618-7622 (1987); (2) Maw et al., "Mutation of the Gene Encoding Cellular Retinaldehyde-Binding Protein in Autosomal Recessive Retinitis Pigmentosa," Nat Genet 17:198-200 (1997); (3) Crabb et al., "Structural and Functional Characterization of Recombinant Human Cellular Retinaldehyde-Binding Protein," Protein Sci 7:746-757 (1998); (4) den Hollander et al., "A Homozygous Missense Mutation in the IRBP Gene (RBP3) Associated with Autosomal Recessive Retinitis Pigmentosa," Invest Ophthalmol Vis Sci 50:1864-1872 (2009), (5) Li et al., "Secretory Defect and Cytotoxicity: The Potential Disease Mechanisms for the Retinitis Pigmentosa (RP)-Associated Interphotoreceptor Retinoid-Binding Protein (IRBP)," J Biol Chem 288:11395-11406 (2013); (6) Friedman et al.," Protein Localization in the Human Eye and Genetic Screen of Opticin," Hum Mol Genet 11:1333-1342 (2002); (7) Reardon et al., "Identification in Vitreous and Molecular Cloning of Opticin, A Novel Member of the Family of Leucine-Rich Repeat Proteins of the Extracellular Matrix," J Biol Chem 275:2123-2129 (2000); (8) Stone et al., "Missense Variations in the Fibulin 5 Gene and Age-Related Macular Degeneration," N Engl J Med 351:346-353 (2004); (9) Faivre et al., "In Frame Fibrillin-1 Gene Deletion in Autosomal Dominant Weill-Marchesani Syndrome," J Med Genet 40:34-36 (2003); (10) Hubmacher et al., "Human Eye Development Is Characterized by Coordinated Expression of Fibrillin Isoforms," Invest Ophthalmol Vis Sci 55:7934-7944 (2014); (11) Wheatley et al., Immunohistochemical Localization of Fibrillin in Human Ocular Tissues. Relevance to the Marfan Syndrome," Arch Ophthalmol 113:103-109 (1995); (12) Sakuma et al., "Isolation and Characterization of the Human X-Arrestin Gene," Gene 224:87-95 (1998); (13) Dryja et al., "A Point Mutation of the Rhodopsin Gene in one Form of Retinitis Pigmentosa," Nature 343:364-366 (1990); (14) Wald et al., "The Light Reaction in the Bleaching of Rhodopsin," Science 111:179-181 (1950); (15) Dryja et al., "Mutations Within the Rhodopsin Gene in Patients with Autosomal Dominant Retinitis Pigmentosa," N Engl J Med 323:1302-1307 (1990); (16) Liden et al., "Biochemical Defects in 11-cis-Retinol Dehydrogenase Mutants Associated With Fundus Albipunctatus," J Biol Chem 276:49251-49257 (2001); (17) Yamamoto et al., "Mutations in the Gene Encoding 11-cis Retinol Dehydrogenase Cause Delayed Dark Adaptation and Fundus Albipunctatus," Nat Genet 22: 188-191 (1999); (18) Moiseyev et al., "RPE65 Is an Iron(II)-Dependent Isomerohydrolase in the Retinoid Visual Cycle," J Biol Chem 281:2835-2840 (2006); (19) Nicoletti et al., "Molecular Characterization of the Human Gene Encoding an Abundant 61 kDa Protein Specific to the Retinal Pigment Epithelium," Hum Mol Genet 4:641-649 (1995); (20) Carter et al., "Mapping of the Human CP49 Gene and Identification of an Intragenic Polymorphic Marker to Allow Genetic Linkage Analysis in Autosomal Dominant Congenital Cataract," Biochem Biophys Res Commun 270:432-436 (2000); (21) Merdes et al., "The 47-kD Lens-Specific Protein Phakinin is a Tailless Intermediate Filament Protein and an Assembly Partner of Filensin," J Cell Biol 123, 1507-1516 (1993).

The inventors sought to characterize vitreous EVs and determine if these EVs can transfer their RNA and protein cargo into target cells (Valadi et al., "Exosome-Mediated Transfer of mRNAs and MicroRNAs is a Novel Mechanism of Genetic Exchange Between Cells," Nat. Cell Biol. 9:654-659 (2007); Skog et al., "Glioblastoma Microvesicles Transport RNA and Proteins That Promote Tumour Growth and Provide Diagnostic Biomarkers," Nat. Cell Biol. 10:1470-1476 (2008)). To accomplish this bovine or human vitreous EV RNA was labeled with acridine orange , the EV fraction was purified (Figures 19A-B), and then retinal pigment epithelium cells (ARPE-19) were exposed to a bolus of the labeled EVs. A transfection rate of up to 96.2% at 48 hours with bovine vitreous EVs was observed (Figures 20A-B). Human vitreous EVs isolated from post-mortem ocular samples show a transfect rate of 96% at 24 hours (Figure 20C-D), both of which were significantly more than controls (p<0.05). EVs are also known to function as a vector to deliver recombinant proteins. Thus, bovine serum albumin (BSA, 66 kD protein) conjugated to fluorescein was loaded into bovine vitreous EVs via electropermeabilization. Then cultured retinal pigment epithelial (ARPE-19) cells were treated and observed that cells were transfected up to 97.6%. The controls, PBS alone or EVs mixed with BSA-fluorescein without electroporation, did not result in transfection of ARPE-19 cells (Figures 21A-C, p<0.005, n = 3). The controls demonstrated that uptake of BSA-fluorescein is EV-dependent. To evaluate whether vitreous EVs can transfect a functional protein, which must retain its conformational state to fluoresce, recombinant green fluorescent protein (GFP, 26.9 kD) was loaded into bovine vitreous EVs. The data showed that ARPE-19 cells were transfected up to 88.3% (Figures 21D-F), significantly more than controls (p<0.05, n = 3). These data show that vitreous EVs are capable of transferring RNA and recombinant protein *in vitro.*

Finally, vitreous EV transfection *in vivo* was studied. A dilute concentration of EVs loaded with BSA-fluorescein was administered to rodent eyes through intravitreal injection. On day 3, EVs showed no evidence of retinal penetration (Figure 22A). At 3 weeks, transfection of multiple retinal cell layers *in vivo* was observed (Figures 22B-C). Specificity controls, PBS alone (Figure 22D) or EV samples mixed with BSA-fluorescein without electropermeabilization were negative. These data show that the vitreous EVs function as a vector for recombinant protein delivery *in vivo.*

## Claims

1. A method of fixing extracellular vesicles, said method comprising:
providing a sample containing extracellular vesicles and
contacting the sample with a non-reversible cross-linking agent under conditions effective to fix the extracellular vesicles.

2. The method of claim 1 further comprising:
contacting said sample with an additional fixative before, after, or at the same time as said contacting the sample with the non-reversible cross-linking agent,
wherein the additional fixative is an aldehyde-containing fixative, preferably wherein the aldehyde-containing fixative is glutaraldehyde or paraformaldehyde; or
wherein the additional fixative is one of ethylene glycol di(meth)acrylate, ethylene glycol diacrylate, di(ethylene glycol) diacrylate, tetra(ethylene glycol) diacrylate, ethylene glycol dimethacrylate, di(ethylene glycol) dimethacrylate, tri(ethylene glycol) dimethacrylate, N,N- methylenebisacrylamide, N,N- (1,2-dihydroxyethylene)bisacrylamide, formaldehyde-free cross- linking agents, N- (1-hydroxy-2,2-dimethoxyethyl)acrylamide, divinylbenzene, formalin fixatives, formal calcium, formal saline, zinc formalin (unbuffered), Zenker's fixative, Helly' s fixative, B-5 fixative, Bouin's solution, Hollande's solution, Gendre's solution, Clarke's solution, Carnoy's solution, methacarn, alcoholic formalin, and formol acetic alcohol.

3. The method of claim 1 or 2, wherein said extracellular vesicles are selected from the group consisting of exomeres, exosomes, multivesicular bodies, intraluminal vesicles (ILVs), multivesicular endosomes (MVEs), oncosomes, micro-vesicles, apoptotic bodies, and vesicles originating from endosome or plasma membranes, wherein the extracellular vesicles range in size from 20 nanometers to 10,000 nanometers.

4. The method of any one of the preceding claims, wherein the sample is a biological fluid or tissue, preferably wherein the biological fluid is selected from the group consisting of blood products, vitreous or aqueous humor, cerebrospinal fluid or nipple aspirate fluid, and preferably wherein the biological tissue is a tissue selected from the group consisting of skin, bone, cartilage, tendon, ligament, vertebral disc, cornea, lens, meniscus, hair, striated muscle, smooth muscle, cardiac muscle, adipose tissue, fibrous tissue, neural tissue, connective tissue, cochlea, testis, ovary, stomach, lung, heart, liver, pancreas, kidney, intestine, and eye.

5. The method of any one of the preceding claims, wherein the non-reversible cross-linking agent is selected from the group consisting of a water-soluble carbodiimide, cyanogen halide, and mixtures thereof, preferably wherein the non-reversible cross-linking agent is 1-ethyl-3-(3-dimethylaminopropyl)-carbodiimide, cyanogen bromide, cyanogen fluoride, cyanogen chloride, or cyanogen iodide.

6. Fixed extracellular vesicles obtained by the method of any one of claims 1-5.

7. The use of a non-reversible cross-linking agent in the method of any one of claims 1-5, for detecting extracellular vesicles in the biological sample by imaging the fixed extracellular vesicles.

8. The use of claim 7, wherein said imaging is carried out by transmission electron microscopy, scanning electron microscopy, cryoelectron microscopy, binocular stereoscopic microscopy, wide-field microscopy, polarizing microscopy, phase contrast microscopy, multiphoton microscopy, differential interference contrast microscopy, fluorescence microscopy, laser scanning confocal microscopy, multiphoton excitation microscopy, ray microscopy, ultrasonic microscopy, color metric assay, chemiluminescence assay, spectrophotometry, positron emission tomography, computerized tomography, or magnetic resonance imaging.

9. The use of a non-reversible cross-linking agent in the method of any one of claims 1-5, for detecting biomarkers in the sample.

10. The use of claim 9, wherein the biomarkers are extracellular vesicles, proteins, RNA or DNA.

11. A method of diagnosing whether a first subject has a particular disease, the method comprising:
fixing extracellular vesicles according to the method of any one claims 1-5 wherein the sample is taken from said first subject;
imaging the fixed extracellular vesicles; and
diagnosing whether said subject has the particular disease based on said imaging, wherein said diagnosing comprises
providing a standard image of a clinical sample containing extracellular vesicles fixed with the non-reversible cross-linking agent from a second subject who has been diagnosed with the particular disease;
comparing the image of the sample of the first subject to the standard image with regard to size, density, morphology, or spatial distribution of the fixed extracellular vesicles; and determining if the subject has a disease or disorder based on said comparing.

12. The method of claim 11, wherein the disease is selected from the group consisting of cancer, inflammatory diseases, infections, degenerative diseases, diseases caused by pathogens, neurological diseases, and internal dysfunctions.

13. The method of claim 1, wherein the sample is a biological sample and the method is carried out using a kit comprising:
a support substrate for holding the sample; and
a non-reversible cross-linking agent, wherein the non-reversible cross-linking agent is selected from the group consisting of a water-soluble carbodiimide, cyanogen halide, and mixtures thereof, preferably wherein the non-reversible cross-linking agent is 1-ethyl-3-(3-dimethylaminopropyl) carbodiimide.

14. The method of claim 13, wherein the kit further comprises an additional fixative, wherein the additional fixative is an aldehyde-containing fixative, glutaraldehyde, paraformaldehyde, ethylene glycol di(meth)acrylate, ethylene glycol diacrylate, di(ethylene glycol) diacrylate, tetra(ethylene glycol) diacrylate, ethylene glycol dimethacrylate, di(ethylene glycol) dimethacrylate, tri(ethylene glycol) dimethacrylate, N,N- methylenebisacrylamide, N,N-methylenebisacrylamide, N,N-(1,2-dihydroxyethylene)bisacrylamide, formaldehyde-free cross-linking agents, N-(1-hydroxy-2,2-dimethoxyethyl)acrylamide, divinylbenzene, formalin fixatives, formal calcium, formal saline, zinc formalin (unbuffered), Zenker's fixative, Helly's fixative, B-5 fixative, Bouin's solution, Hollande's solution, Gendre's solution, Clarke's solution, Carnoy's solution, methacarn, alcoholic formalin, and formol acetic alcohol.

## Patentansprüche

1. Verfahren zur Fixierung extrazellulärer Vesikel, wobei das Verfahren umfasst:
Bereitstellen einer Probe, die extrazelluläre Vesikel enthält und
Inkontaktbringen der Probe mit einem nicht-reversiblen Vernetzungsmittel unter Bedingungen, die eine Fixierung der extrazellulären Vesikel bewirken.

2. Verfahren nach Anspruch 1, das ferner umfasst:
Inkontaktbringen der Probe mit einem zusätzlichen Fixiermittel vor, nach oder gleichzeitig mit dem Inkontaktbringen der Probe mit dem nicht-reversiblen Vernetzungsmittel,
wobei das zusätzliche Fixiermittel ein aldehydhaltiges Fixiermittel ist, vorzugsweise wobei das aldehydhaltige Fixiermittel Glutaraldehyd oder Paraformaldehyd ist, oder
wobei das zusätzliche Fixiermittel eines der Folgenden ist: Ethylenglykoldi(meth)acrylat, Ethylenglykoldiacrylat, Di(ethylenglykol)diacrylat, Tetra(ethylenglykol)diacrylat, Ethylenglykoldimethacrylat, Di(ethylenglykol)dimethacrylat, Tri(ethylenglykol)dimethacrylat, N,N-Methylenbisacrylamid, N,N-(1,2-Dihydroxyethylen)bisacrylamid, formaldehydfreie Vernetzer, N-(1-Hydroxy-2,2-dimethoxyethyl)acrylamid, Divinylbenzol, Formalinfixiermittel, formales Kalzium, formale Kochsalzlösung, Zinkformalin (ungepuffert), Zenker's Fixativ, Helly's Fixativ, B-5 Fixativ, Bouin's Lösung, Hollande's Lösung, Gendre's Lösung, Clarke's Lösung, Camoy's Lösung, Methacarn, alkoholisches Formalin und Formol-Essigalkohol.

3. Verfahren nach Anspruch 1 oder 2, wobei die extrazellulären Vesikel ausgewählt sind aus der Gruppe bestehend aus Exomeren, Exosomen, multivesikulären Körpern, intraluminalen Vesikeln (ILVs), multivesikulären Endosomen (MVEs), Onkosomen, Mikrovesikeln, apoptotischen Körpern und Vesikeln, die von Endosomen oder Plasmamembranen stammen, wobei die extrazellulären Vesikel eine Größe von 20 Nanometern bis 10.000 Nanometern aufweisen.

4. Verfahren nach einem der vorhergehenden Ansprüche, wobei die Probe eine biologische Flüssigkeit oder ein Gewebe ist, vorzugsweise wobei die biologische Flüssigkeit ausgewählt ist aus der Gruppe bestehend aus Blutprodukten, Glaskörper oder Kammerwasser, Liquor cerebrospinalis oder Nippelaspirat, und wobei das biologische Gewebe vorzugsweise ein Gewebe ist, das ausgewählt ist aus der Gruppe bestehend aus Haut, Knochen, Knorpel, Sehne, Band, Wirbelscheibe, Hornhaut, Linse, Meniskus, Haar, quergestreifter Muskel, glatter Muskel, Herzmuskel, Fettgewebe, faseriges Gewebe, Nervengewebe, Bindegewebe, Cochlea, Hoden, Eierstock, Magen, Lunge, Herz, Leber, Bauchspeicheldrüse, Niere, Darm und Auge.

5. Verfahren nach einem der vorhergehenden Ansprüche, wobei das nicht-reversible Vernetzungsmittel ausgewählt ist aus der Gruppe bestehend aus einem wasserlöslichen Carbodiimid, Cyanogenhalogenid und Gemischen davon, wobei das nicht-reversible Vernetzungsmittel vorzugsweise 1-Ethyl-3-(3-Dimethylaminopropyl)-carbodiimid, Cyanogenbromid, Cyanogenfluorid, Cyanogenchlorid oder Cyanogenjodid ist.

6. Fixierte extrazelluläre Vesikel, das durch das Verfahren nach einem der Ansprüche 1 - 5 erhalten wird.

7. Verwendung eines nicht-reversiblen Vernetzungsmittels in dem Verfahren nach einem der Ansprüche 1 - 5, um extrazelluläre Vesikel in der biologischen Probe durch Abbildung der fixierten extrazellulären Vesikel zu erfassen.

8. Verwendung von Anspruch 7, wobei die Abbildung durchgeführt wird durch Transmissionselektronenmikroskopie, Rasterelektronenmikroskopie, Kryoelektronenmikroskopie, binokulare stereoskopische Mikroskopie, Weitfeldmikroskopie, Polarisationsmikroskopie, Phasenkontrastmikroskopie, Multiphotonenmikroskopie, Differentialinterferenzkontrastmikroskopie, Fluoreszenzmikroskopie, Laser-Scanning-Konfokalmikroskopie, Multiphotonenanregungsmikroskopie, Strahlenmikroskopie, Ultraschallmikroskopie, Farbmetrik-Assay, Chemilumineszenz-Assay, Spektrophotometrie, Positronen-Emissions-Tomographie, Computertomographie oder Magnetresonanztomographie.

9. Verwendung eines nicht-reversiblen Vernetzungsmittels in dem Verfahren nach einem der Ansprüche 1 - 5, um Biomarker in der Probe zu erfassen.

10. Verwendung nach Anspruch 9, wobei die Biomarker extrazelluläre Vesikel, Proteine, RNA oder DNA sind.

11. Verfahren zum Diagnostizieren, ob ein erstes Subjekt eine bestimmte Krankheit hat, wobei das Verfahren Folgendes umfasst:
Fixieren extrazellulärer Vesikel nach dem Verfahren eines der Ansprüche 1 - 5, wobei die Probe dem ersten Subjekt entnommen wird;
Bildgebung der fixierten extrazellulären Vesikel, und
Diagnostizieren, ob das Subjekt die bestimmte Krankheit hat, basierend auf der Bildgebung, wobei das Diagnostizieren umfasst:
Bereitstellen eines Standardbildes einer klinischen Probe, die extrazelluläre Vesikel enthält, die mit dem nicht-reversiblen Vernetzungsmittel fixiert wurden, von einem zweiten Patienten, bei dem die bestimmte Krankheit diagnostiziert wurde;
Vergleichen des Bildes der Probe des ersten Patienten mit dem Standardbild im Hinblick auf Größe, Dichte, Morphologie oder räumliche Verteilung der fixierten extrazellulären Vesikel, und Bestimmen, ob das Subjekt basierend auf dem Vergleich eine Krankheit oder Störung hat.

12. Verfahren nach Anspruch 11, wobei die Krankheit ausgewählt ist aus der Gruppe bestehend aus Krebs, Entzündungskrankheiten, Infektionen, degenerativen Krankheiten, durch Krankheitserreger verursachten Krankheiten, neurologischen Krankheiten und inneren Dysfunktionen.

13. Verfahren nach Anspruch 1, wobei die Probe eine biologische Probe ist und das Verfahren unter Verwendung eines Kits durchgeführt wird, umfassend:
ein Trägersubstrat zum Halten der Probe, und
ein nicht-reversibles Vernetzungsmittel, wobei das nicht-reversible Vernetzungsmittel ausgewählt ist aus der Gruppe bestehend aus einem wasserlöslichen Carbodiimid, Cyanogenhalogenid und Gemischen davon, wobei das nicht-reversible Vernetzungsmittel vorzugsweise 1-Ethyl-3-(3-dimethylaminopropyl)-carbodiimid ist.

14. Verfahren nach Anspruch 13, wobei der Kit ferner ein zusätzliches Fixiermittel umfasst, wobei das zusätzliche Fixiermittel Folgendes ist: ein aldehydhaltiges Fixativ, Glutaraldehyd, Paraformaldehyd, Ethylenglykoldi(meth)acrylat, Ethylenglykoldiacrylat, Di(ethylenglykol)diacrylat, Tetra(ethylenglykol)diacrylat, Ethylenglykoldimethacrylat, Di(ethylenglykol)dimethacrylat, Tri(ethylenglykol)dimethacrylat, N,N-Methylenbisacrylamid, N,N-Methylenbisacrylamid, N,N-(1,2-Dihydroxyethylen)bisacrylamid, formaldehydfreie Vernetzer, N-(1-Hydroxy-2,2-dimethoxyethyl)acrylamid, Divinylbenzol, FormalinFixiermittel, formales Kalzium, formale Kochsalzlösung, Zinkformalin (ungepuffert), Zenker's Fixiermittel, Helly's Fixiermittel, B-5-Fixiermittel, Bouin's Lösung, Hollande's Lösung, Gendre's Lösung, Clarke's Lösung, Camoy's Lösung, Methacarn, alkoholisches Formalin und Formolessigalkohol.

## Revendications

1. Procédé de fixation de vésicules extracellulaires, ledit procédé comprenant :
la fourniture d'un échantillon contenant des vésicules extracellulaires et
la mise en contact de l'échantillon avec un agent de réticulation non réversible dans des conditions efficaces pour fixer les vésicules extracellulaires.

2. Procédé selon la revendication 1 comprenant en outre :
la mise en contact dudit échantillon avec un fixateur supplémentaire avant, après, ou en même temps que ladite mise en contact de l'échantillon avec l'agent de réticulation non réversible,
dans lequel le fixateur supplémentaire est un fixateur contenant un aldéhyde, de préférence dans lequel le fixateur contenant un aldéhyde est le glutaraldéhyde ou le paraformaldéhyde ; ou
dans lequel le fixateur supplémentaire est l'un parmi le di(méth)acrylate d'éthylène glycol, le diacrylate d'éthylène glycol, le diacrylate de di(éthylène glycol), le diacrylate de tétra(éthylène glycol), le diméthacrylate d'éthylène glycol, le diméthacrylate de di(éthylène glycol), le diméthacrylate de tri(éthylène glycol), le N,N-méthylènebisacrylamide, le N,N-(1,2-dihydroxyéthylène)bisacrylamide, des agents de réticulation sans formaldéhyde, le N-(1-hydroxy-2,2-diméthoxyéthyl)acrylamide, le divinylbenzène, des fixateurs au formol, le calcium formel, une solution saline formelle, le zinc-formol (non tamponné), le fixateur de Zenker, le fixateur de Helly, le fixateur B-5, la solution de Bouin, la solution de Hollande, la solution de Gendre, la solution de Clarke, la solution de Carnoy, le méthacarn, le formol alcoolique, et l'alcool acétique formolique.

3. Procédé selon la revendication 1 ou 2, dans lequel lesdites vésicules extracellulaires sont sélectionnées dans le groupe constitué d'exomères, d'exosomes, de corps multivésiculaires, de vésicules intraluminales (ILV), d'endosomes multivésiculaires (MVE), d'oncosomes, de micro-vésicules, de corps apoptotiques, et de vésicules provenant d'endosomes ou de membranes plasmiques, dans lequel la taille des vésicules extracellulaires est dans la plage de 20 nanomètres à 10 000 nanomètres.

4. Procédé selon l'une quelconque des revendications précédentes, dans lequel l'échantillon est un fluide ou un tissu biologique, de préférence dans lequel le fluide biologique est sélectionné dans le groupe constitué de produits sanguins, d'humeur vitrée ou aqueuse, de liquide céphalo-rachidien ou de liquide d'aspiration mamelonnaire, et de préférence dans lequel le tissu biologique est un tissu sélectionné dans le groupe constitué de peau, d'os, de cartilage, de tendon, de ligament, de disque vertébral, de cornée, de cristallin, de ménisque, de cheveu, de muscle strié, de muscle lisse, de muscle cardiaque, de tissu adipeux, de tissu fibreux, de tissu neural, de tissu conjonctif, de cochlée, de testicule, d'ovaire, d'estomac, de poumon, de coeur, de foie, de pancréas, de rein, d'intestin, et d'oeil.

5. Procédé selon l'une quelconque des revendications précédentes, dans lequel l'agent de réticulation non réversible est sélectionné dans le groupe constitué d'un carbodiimide hydrosoluble, d'un halogénure de cyanogène, et de mélanges de ceux-ci, de préférence dans lequel l'agent de réticulation non réversible est le 1-éthyl-3-(3-diméthylaminopropyl)-carbodiimide, le bromure de cyanogène, le fluorure de cyanogène, le chlorure de cyanogène, ou l'iodure de cyanogène.

6. Vésicules extracellulaires fixées obtenues par le procédé de l'une quelconque des revendications 1 à 5.

7. Utilisation d'un agent de réticulation non réversible dans le procédé de l'une quelconque des revendications 1 à 5, pour détecter des vésicules extracellulaires dans l'échantillon biologique par imagerie des vésicules extracellulaires fixées.

8. Utilisation selon la revendication 7, dans laquelle ladite imagerie est réalisée par microscopie électronique à transmission, microscopie électronique à balayage, microscopie cryoélectronique, microscopie stéréoscopique binoculaire, microscopie à grand champ, microscopie polarisante, microscopie à contraste de phase, microscopie multiphotonique, microscopie à contraste d'interférence différentiel, microscopie à fluorescence, microscopie confocale à balayage laser, microscopie à excitation multiphotonique, microscopie à rayons, microscopie à ultrasons, essai colorimétrique, essai de chimiluminescence, spectrophotométrie, tomographie par émission de positrons, tomographie informatisée, ou imagerie par résonance magnétique.

9. Utilisation d'un agent de réticulation non réversible dans le procédé de l'une quelconque des revendications 1 à 5, pour détecter des biomarqueurs dans l'échantillon.

10. Utilisation selon la revendication 9, dans laquelle les biomarqueurs sont des vésicules extracellulaires, des protéines, de l'ARN ou de l'ADN.

11. Procédé pour diagnostiquer si un premier sujet est atteint d'une maladie particulière, le procédé comprenant :
la fixation de vésicules extracellulaires selon le procédé de l'une quelconque des revendications 1 à 5 dans lequel l'échantillon est prélevé sur ledit premier sujet ;
l'imagerie des vésicules extracellulaires fixées ; et
le diagnostic pour déterminer si ledit sujet est atteint de la maladie particulière sur la base de ladite imagerie, dans lequel ledit diagnostic comprend
la fourniture d'une image standard d'un échantillon clinique contenant des vésicules extracellulaires fixées avec l'agent de réticulation non réversible provenant d'un deuxième sujet chez qui la maladie particulière a été diagnostiquée ;
la comparaison de l'image de l'échantillon du premier sujet à l'image standard en termes de taille, de densité, de morphologie, ou de distribution spatiale des vésicules extracellulaires fixées ; et le fait de déterminer si le sujet est atteint d'une maladie ou d'un trouble sur la base de ladite comparaison.

12. Procédé selon la revendication 11, dans lequel la maladie est sélectionnée dans le groupe constitué du cancer, de maladies inflammatoires, d'infections, de maladies dégénératives, de maladies causées par des agents pathogènes, de maladies neurologiques, et de dysfonctionnements internes.

13. Procédé selon la revendication 1, dans lequel l'échantillon est un échantillon biologique et le procédé est réalisé à l'aide d'un kit comprenant :
un substrat de support pour contenir l'échantillon ; et
un agent de réticulation non réversible, dans lequel l'agent de réticulation non réversible est sélectionné dans le groupe constitué d'un carbodiimide hydrosoluble, d'un halogénure de cyanogène, et de mélanges de ceux-ci, de préférence dans lequel l'agent de réticulation non réversible est le 1-éthyl-3-(3-diméthylaminopropyl)-carbodiimide.

14. Procédé selon la revendication 13, dans lequel le kit comprend en outre un fixateur supplémentaire, dans lequel le fixateur supplémentaire est un fixateur contenant un aldéhyde, le glutaraldéhyde, le paraformaldéhyde, le di(méth)acrylate d'éthylène glycol, le diacrylate d'éthylène glycol, le diacrylate de di(éthylène glycol), le diacrylate de tétra(éthylène glycol), le diméthacrylate d'éthylène glycol, le diméthacrylate de di(éthylène glycol), le diméthacrylate de tri(éthylène glycol), le N,N-méthylènebisacrylamide, le N,N-méthylènebisacrylamide, le N,N-(1,2-dihydroxyéthylène)bisacrylamide, des agents de réticulation sans formaldéhyde, le N-(1-hydroxy-2,2-diméthoxyéthyl)acrylamide, le divinylbenzène, des fixateurs au formol, le calcium formel, une solution saline formelle, le zinc-formol (non tamponné), le fixateur de Zenker, le fixateur de Helly, le fixateur B-5, la solution de Bouin, la solution de Hollande, la solution de Gendre, la solution de Clarke, la solution de Carnoy, le méthacarn, le formol alcoolique, et l'alcool acétique formolique.
